# EUROPEAN PATENT APPLICATION

(11) **EP 4 534 647 A1**
(43) Date of publication of application: **09.04.2025**
(21) Application number: 23812220.4
(22) Date of filing: 26.05.2023
(51) Int. Cl.: C12N 1/20, C12N 15/88, A61K 47/46, A61K 39/00

(54) **METHOD FOR CONSTRUCTING GRAM-NEGATIVE BACTERIAL OUTER MEMBRANE-DERIVED NANOVESICLES AND USE THEREOF**

(30) Priority: 27.05.2022 KR 20220065067
(71) Applicant: POSTECH Research and Business Development Foundation, Gyeongsangbuk-do 37673 (KR)
(72) Inventor: GHO, Yong Song, Pohang-si Gyeongsangbuk-do 37673 (KR); LEE, Jie Oh, Pohang-si Gyeongsangbuk-do 37673 (KR); LEE, Chang Jin, Seongnam-si Gyeonggi-do 13494 (KR); ROH, Tae Young, Pohang-si Gyeongsangbuk-do 37673 (KR); LEE, Tae Ryong, Seongnam-si Gyeonggi-do 13494 (KR); BAE, Seo Yoon, Pohang-si Gyeongsangbuk-do 37673 (KR); SHIN, Ha Young, Pohang-si Gyeongsangbuk-do 37673 (KR); JEONG, Hye Jin, Pohang-si Gyeongsangbuk-do 37673 (KR); YOO, Mi Joung, Pohang-si Gyeongsangbuk-do 37673 (KR); SONG, Sung Hyun, Seongnam-si Gyeonggi-do 13494 (KR); LEE, Jae Wook, Pohang-si Gyeongsangbuk-do 37673 (KR)
(74) Representative: Richardt Patentanwälte PartG mbB
(86) International application number: PCT/KR2023/007333
(87) International publication number: WO 2023/229446

(57) **Abstract**

The present invention relates to a method for preparing Gram-negative bacterial outer membrane-derived nanovesicles (OMNVs) and uses thereof. Additionally, the present invention provides a method for treating or diagnosing cancer using OMNVs. Furthermore, the present invention provides a method for delivering therapeutic or diagnostic substances to target cells or tissues by loading them into OMNVs. Also, the present invention provides a method for preventing and treating various diseases, including cancer and bacterial/viral infections, by co-administering OMNVs with antigens or administering OMNVs expressing antigens.

## Description

### TECHNICAL FIELD

This application claims priority to Korean Patent Application No. 10-2022-0065067, filed on May 27, 2022, and the entire disclosure of the aforementioned application is incorporated herein by reference.

The present invention relates to a method for preparing Gram-negative bacterial outer membrane-derived nanovesicles (OMNVs) and uses thereof. More specifically, the present invention provides a method for preparing OMNVs and a method for reducing adverse effects, enhancing efficacy, and delivering to target cells or tissues *in vitro* and *in vivo* using the OMNVs, thereby providing pharmaceutical compositions and methods for enhancing the stability and efficacy of therapeutic agents, drug delivery systems, and/or vaccine delivery systems for treating or preventing various diseases, including cancer, and bacterial/viral infections.

### BACKGROUND OF THE INVENTION

It is known that all cells, including Gram-negative bacteria, naturally secrete extracellular vesicles. The extracellular vesicles secreted from Gram-negative bacteria are also known as outer membrane vesicles (OMVs). OMVs range in size from 20-200 nm and contain various biologically active substances such as proteins, lipids, genetic materials, lipopolysaccharides (LPS), and metabolites. OMVs perform various functions, including delivering proteins or genetic materials, eliminating competing bacteria, enhancing bacterial survival, delivering toxins to host cells, and modulating immune responses.

Recent research results indicate that OMVs naturally secreted by various Gram-negative bacteria contain various substances that can activate the immune system. Since these OMVs are non-living entities with the bacteria themselves removed, they are safer than the bacteria themselves and have direct therapeutic efficacy against various diseases, including cancer. They are also being clinically used or developed as drug delivery systems or vaccine delivery systems for preventing or treating various diseases such as cancer, COVID-19, and meningitis.

It is known that when OMVs naturally secreted by Gram-negative bacteria are intravenously administered into mice with cancer, they induce anti-cancer immune responses that effectively kills cancer tissues without significant adverse effects.

Additionally, due to their nano sizes, OMVs naturally secreted by Gram-negative bacteria can specifically accumulate in cancer tissues through enhanced permeability and retention (EPR) effects. Therefore, they can be used as drug delivery systems after loading drugs for cancer treatment or diagnosis.

On the other hand, it is known that when OMVs naturally secreted by Gram-negative bacteria are intraperitoneally administered into mice three times with one-week intervals, and the corresponding Gram-negative bacteria are intraperitoneally introduced into the mice one week after the last administration, the mice can be prevented from death due to the bacteria. OMVs are presumed to prevent death due to bacteria by inducing Th1 and Th17 immune responses. In fact, OMVs are being clinically used or developed as vaccine delivery systems for preventing or treating various diseases such as cancer, COVID-19, and meningitis.

Furthermore, to enhance therapeutic and preventive efficacy of OMVs, various attempts are being made, such as loading various therapeutic drugs or using bacterial-derived extracellular vesicles expressing various proteins as fusion proteins, including cytokines, growth factors, and antibodies (for targeting specific cells or tissues, neutralizing the activity of antigens, or performing various functions) on the outer membrane proteins.

However, OMVs naturally secreted by Gram-negative bacteria are produced in very small quantities, and the purification process is complex and difficult, resulting in low yields, making them unsuitable for mass production. They also contain unnecessary intracellular substances such as periplasmic proteins, inner membrane proteins, cytoplasmic proteins, excessive genetic materials, and various metabolites, which can reduce the efficiency of drug loading or induce adverse effects when administered *in vivo.* Therefore, it is necessary to develop methods to remove these substances.

To overcome the above problems, Gram-negative bacterial outer membrane-derived nanovesicles (OMNVs) have been artificially manufactured and developed as therapeutic agents, drug delivery systems, and/or vaccine delivery systems for various diseases, including cancer, for a long time.

Typically, OMNVs are manufactured by treating Gram-negative bacteria with ethylenediaminetetraacetic acid (EDTA) and lysozyme, breaking them down through sonication, separating the outer and inner membranes using high-speed centrifugation, removing the inner membrane by treating with detergents such as sodium lauryl sarcosinate (Sarkosyl), and then isolating the outer membrane components using high-speed centrifugation, followed by manufacturing through sonication or extrusion.

However, the above method for manufacturing OMNVs has many limitations for mass production for clinical use due to its complexity and the need to use various substances such as lysozyme. Therefore, it is necessary to develop methods to overcome these limitations.

### SUMMARY OF INVENTION

### PROBLEM TO BE SOLVED

The present inventors have conducted research to solve the conventional problems as described above. As a result, the inventors discovered that treating Gram-negative bacteria with Sarkosyl removes the inner membrane, allowing the extraction of the outer membrane without the need for ultracentrifugation, and using the extracted outer membrane to prepare nanovesicles, thereby completing the present invention.

The present invention also provides a method for treating or diagnosing cancer using Gram-negative bacterial outer membrane-derived nanovesicles (OMNVs), and a method for delivering therapeutic or diagnostic substances by loading them onto the exterior, interior, or outer membrane of the nanovesicles. Additionally, the invention provides a method for delivering OMNVs, which express fusion proteins of outer membrane proteins and various proteins such as cytokines, growth factors, and antibodies, to target cells or tissues, enhancing therapeutic effects, reducing adverse effects, thereby providing the methods for improving the effects of preventing/treating various diseases and reducing adverse effects. Furthermore, the invention provides a method for preventing and treating various diseases by co-administering OMNVs with antigens or administering OMNVs expressing antigens.

However, the technical problems to be achieved by the present invention are not limited to the above-mentioned problems, and other problems not mentioned will be clearly understood by those skilled in the art from the following description.

### MEANS FOR SOLVING THE PROBLEM

To achieve the aforementioned objectives of the present invention, the present invention provides a method for preparing Gram-negative bacterial outer membrane-derived nanovesicles (OMNVs), comprising steps of:
(a) obtaining an outer membrane by removing an inner membrane from Gram-negative bacteria; and
(b) preparing nanovesicles from a suspension comprising the outer membrane.

To achieve another objective of the present invention, the present invention provides a pharmaceutical composition for treating a disease comprising OMNVs, wherein the OMNVs have therapeutic activity.

Additionally, to achieve another objective of the present invention, the present invention provides a pharmaceutical composition for treating a disease consisting of OMNVs, wherein the OMNVs have therapeutic activity.

Furthermore, to achieve another objective of the present invention, the present invention provides a pharmaceutical composition for treating a disease consisting essentially of OMNVs, wherein the OMNVs have therapeutic activity.

To achieve another objective of the present invention, the present invention provides a vaccine composition for preventing or treating a disease comprising OMNVs.

To achieve another objective of the present invention, the present invention provides the use of OMNVs according to the present invention for manufacturing a pharmaceutical composition for treating a disease.

To achieve another objective of the present invention, the present invention provides a method for treating a disease comprising administering an effective amount of a pharmaceutical composition comprising OMNVs according to the present invention to a subject in need thereof.

Hereinafter, the present invention will be described in details.

The present invention provides a method for preparing OMNVs, comprising steps of:
(a) obtaining an outer membrane by removing an inner membrane from Gram-negative bacteria; and
(b) preparing nanovesicles from a suspension comprising the outer membrane.

In the present invention, the term "Gram-negative bacteria" refers to a group of bacteria that do not retain the color of crystal violet stain in Gram staining method. Gram-negative bacteria have a cell surface composed of an outer membrane, a periplasmic space containing proteins such as lipoproteins, a peptidoglycan layer, and an inner membrane (or cytoplasmic membrane). Among these, the outer membrane is composed of an asymmetric lipid bilayer, with the inner leaflet consisting of phospholipids and the outer leaflet consisting of lipopolysaccharides. Additionally, the outer membrane accommodates outer membrane proteins such as OmpA, OmpE, OmpC, LptD, and BamA.

In one embodiment of the present invention, the Gram-negative bacteria may be bacteria selected from the group consisting of *Escherichia* genus, *Helicobacter* genus, *Hemophilus* genus, *Neisseria* genus, *Cyanobacterium* genus, *Klebsiella* genus, *Acetobacter* genus, *Acinetobacter* genus, *Enterobacter* genus, *Chlamydia* genus, *Vibrio* genus, *Pseudomonas* genus, *Salmonella* genus, *Thiobacter* genus, *Borrelia* genus, *Burkholderia* genus, *Serratia* genus, and *Treponema* genus, but are not limited thereto. In a preferred embodiment of the present invention, the Gram-negative bacteria may be bacteria belonging to the *Escherichia* genus, *Salmonella* genus, or *Pseudomonas* genus.

The term "nanovesicles" in the present invention refers to structures where the inside and outside are separated by a lipid bilayer composed of cell membrane components of the originating bacteria, including bacterial cell membrane lipids and cell membrane proteins, and having the same topology, but with intracellular substances such as genetic materials and intracellular proteins removed, and being smaller in size than the original cells, but not limited thereto.

In the present invention, the nanovesicles are not particularly limited as long as their diameter is at the nanometer level, but for example, the diameter may be about 20-290 nm, 20-280 nm, 20-270 nm, 20-260 nm, 20-250 nm, 20-240 nm, 20-230 nm, 20-220 nm, 20-210 nm, 20-200 nm, 20-190 nm, 20-180 nm, 20-170 nm, 20-160 nm, 20-150 nm, 20-140 nm, 20-130 nm, 20-120 nm, 20-110 nm, 20-100 nm, 20-90 nm, 20-80 nm, 20-70 nm, 20-60 nm, 20-50 nm, 20-40 nm, 20-30 nm, 30-300 nm, 30-290 nm, 30-280 nm, 30-270 nm, 30-260 nm, 30-250 nm, 30-240 nm, 30-230 nm, 30-220 nm, 30-210 nm, 30-200 nm, 30-190 nm, 30-180 nm, 30-170 nm, 30-160 nm, 30-150 nm, 30-140 nm, 30-130 nm, 30-120 nm, 30-110 nm, 30-100 nm, 30-90 nm, 30-80 nm, 30-70 nm, 30-60 nm, 30-50 nm, 30-40 nm, 40-300 nm, 40-290 nm, 40-280 nm, 40-270 nm, 40-260 nm, 40-250 nm, 40-240 nm, 40-230 nm, 40-220 nm, 40-210 nm, 40-200 nm, 40-190 nm, 40-180 nm, 40-170 nm, 40-160 nm, 40-150 nm, 40-140 nm, 40-130 nm, 40-120 nm, 40-110 nm, 40-100 nm, 40-90 nm, 40-80 nm, 40-70 nm, 40-60 nm, 40-50 nm, 50-300 nm, 50-290 nm, 50-280 nm, 50-270 nm, 50-260 nm, 50-250 nm, 50-240 nm, 50-230 nm, 50-220 nm, 50-210 nm, 50-200 nm, 50-190 nm, 50-180 nm, 50-170 nm, 50-160 nm, 50-150 nm, 50-140 nm, 50-130 nm, 50-120 nm, 50-110 nm, 50-100 nm, 50-90 nm, 50-80 nm, 50-70 nm, 50-60 nm, 60-300 nm, 60-290 nm, 60-280 nm, 60-270 nm, 60-260 nm, 60-250 nm, 60-240 nm, 60-230 nm, 60-220 nm, 60-210 nm, 60-200 nm, 60-190 nm, 60-180 nm, 60-170 nm, 60-160 nm, 60-150 nm, 60-140 nm, 60-130 nm, 60-120 nm, 60-110 nm, 60-100 nm, 60-90 nm, 60-80 nm, 60-70 nm, 70-300 nm, 70-290 nm, 70-280 nm, 70-270 nm, 70-260 nm, 70-250 nm, 70-240 nm, 70-230 nm, 70-220 nm, 70-210 nm, 70-200 nm, 70-190 nm, 70-180 nm, 70-170 nm, 70-160 nm, 70-150 nm, 70-140 nm, 70-130 nm, 70-120 nm, 70-110 nm, 70-100 nm, 70-90 nm, 70-80 nm, 80-300 nm, 80-290 nm, 80-280 nm, 80-270 nm, 80-260 nm, 80-250 nm, 80-240 nm, 80-230 nm, 80-220 nm, 80-210 nm, 80-200 nm, 80-190 nm, 80-180 nm, 80-170 nm, 80-160 nm, 80-150 nm, 80-140 nm, 80-130 nm, 80-120 nm, 80-110 nm, 80-100 nm, 80-90 nm, 90-300 nm, 90-290 nm, 90-280 nm, 90-270 nm, 90-260 nm, 90-250 nm, 90-240 nm, 90-230 nm, 90-220 nm, 90-210 nm, 90-200 nm, 90-190 nm, 90-180 nm, 90-170 nm, 90-160 nm, 90-150 nm, 90-140 nm, 90-130 nm, 90-120 nm, 90-110 nm, 90-100 nm, 100-300 nm, 110-290 nm, 120-280 nm, 130-270 nm, 140-260 nm, 150-250 nm, 160-240 nm, 170-230 nm, 180-220 nm, or 190-210 nm.

**In** the present invention, the term "Gram-negative bacterial outer membrane-derived nanovesicles (OMNVs)" refers to artificially prepared nanovesicles using the outer membrane of Gram-negative bacteria according to the method comprising steps (a) and (b).

**In** the method for preparing nanovesicles according to the present invention, step (a) is the step of obtaining an outer membrane by removing the inner membrane from Gram-negative bacteria.

In one embodiment of the present invention, step (a) can be performed by treating Gram-negative bacteria with an anionic surfactant. The term "anionic surfactant" refers to a substance that, when dissociated in water, has an anion that adsorbs to the surface of the aqueous solution, thereby reducing surface tension. Non-limiting examples of the anionic surfactant include sodium dodecyl sulfate (SDS), Sarkosyl, lithium dodecyl sulfate, and sodium 1-octane sulfonic acid, but are not limited thereto. In a preferred embodiment of the present invention, the anionic surfactant may be Sarkosyl.

In one embodiment of the present invention, in step (a), the anionic surfactant can be treated at a final concentration of 0.1 to 5% (w/v) in a culture medium or suspension containing Gram-negative bacteria, preferably at a final concentration of 0.1 to 4% (w/v), more preferably at a final concentration of 0.1 to 3% (w/v), even more preferably at a final concentration of 0.1 to 2% (w/v), and most preferably at a final concentration of 0.5 to 2% (w/v).

In the present invention, the term "culture medium" refers to an inclusion of Gram-negative bacteria in a medium containing nutrients in which gram-negative bacteria can survive and proliferate, and the term "suspension" refers to a culture medium containing Gram-negative bacteria from which only the bacteria have been separated by means such as centrifugation, and then the separated Gram-negative bacteria are suspended in a medium or buffer. In a preferred embodiment of the present invention, the terms "culture medium" and "suspension" can be interpreted interchangeably.

In one embodiment of the present invention, in step (a), the anionic surfactant can be treated at a temperature of 4 to 60°C for 5 to 120 minutes, preferably at a temperature of 4 to 60°C for 5 to 60 minutes, more preferably at a temperature of 4 to 60°C for 10 to 60 minutes, even more preferably at a temperature of 4 to 60°C for 10 to 50 minutes, and most preferably at a temperature of 4 to 60°C for 20 to 40 minutes, but is not limited thereto.

In one embodiment of the present invention, in step (a), the anionic surfactant can be treated once or repeatedly. When the anionic surfactant is treated repeatedly in step (a), the process of treating the anionic surfactant for a certain period of time in a culture medium or suspension containing Gram-negative bacteria, removing the residual anionic surfactant in the supernatant by means such as centrifugation, suspending the Gram-negative bacteria in a medium or buffer, and then treating the anionic surfactant again can be repeated. In the present invention, the term "repeatedly" can mean 2 to 10 times, preferably 2 to 5 times, more preferably 2 to 4 times, and most preferably 2 to 3 times. When the anionic surfactant is treated repeatedly in step (a), the treatment conditions (i.e., treatment concentration, treatment time, treatment temperature, etc.) for each treatment can be the same or different.

In step (a), after treating the Gram-negative bacteria with the anionic surfactant, a process for separating the outer membrane from the inner membrane, cytoplasmic components, etc., can be additionally performed. For example, after step (a), any method for separating and purifying the outer membrane known in the art, such as centrifugation, filtration, dialysis, size exclusion chromatography, Capto Core chromatography, tangential flow filtration, etc., can be additionally performed, but is not limited thereto.

In the present invention, step (b) is the process of forming nanovesicles by applying energy to the outer membrane of Gram-negative bacteria separated in step (a). In one embodiment of the present invention, step (b) can be performed by applying one or more means selected from the group consisting of microfluidics, sonication, extrusion, treatment with an alkaline solution, treatment with a homogenizer or disperser, and nitrogen cavitation to a culture medium or suspension containing the outer membrane derived from Gram-negative bacteria, but is not limited thereto.

In one embodiment of the present invention, after step (b), a process of purifying or separating and collecting the nanovesicles derived from the outer membrane of Gram-negative bacteria contained in the culture medium or suspension can be additionally performed. The purification or separation can be performed by any method known in the art without limitation. For example, it can be separated or purified by a method selected from the group consisting of ultracentrifugation, density gradient ultracentrifugation, ultrafiltration, size exclusion chromatography, ion exchange chromatography, Capto Core chromatography, immunoaffinity separation, microfluidic separation, aqueous two-phase system, polymer-based precipitation, sonication, and extrusion, but is not limited thereto.

According to the method of the present invention, an increased yield can be provided compared to the conventional technique for separating naturally secreted outer membrane vesicles. For example, based on the same volume of bacterial culture medium, OMNVs can be obtained at a yield at least about 2 to 1000 times higher in terms of particle numbers than naturally secreted bacterial outer membrane vesicles. For example, based on the same volume of bacterial culture medium, OMNVs can be obtained at a level at least about 2 to 100 times higher in terms of total protein than naturally secreted bacterial outer membrane vesicles. For example, based on the same volume of bacterial culture medium, OMNVs can be obtained at a purity (total particle numbers per total protein amounts) at least about 2 to 100 times higher than naturally secreted bacterial outer membrane vesicles. Additionally, the OMNVs prepared according to the present invention are very unlikely to contain non-outer membrane-derived components such as surrounding cytoplasm, inner membrane, and cytoplasm.

In one embodiment of the present invention, the OMNVs prepared according to the method can be characterized by being deficient in at least one non-outer membrane-derived component of the bacteria from which they are derived. The term "non-outer membrane-derived component" includes peptidoglycan, surrounding cytoplasmic proteins, inner membrane proteins, nucleic acids, cytoplasmic proteins, and ribosomes.

In one embodiment of the present invention, the OMNVs prepared according to the method can be characterized by being deficient in at least 50%, preferably at least 70%, more preferably at least 90%, and most preferably 100% of at least one non-outer membrane-derived component compared to naturally secreted outer membrane vesicles of the bacteria from which they are derived.

In one embodiment of the present invention, the Gram-negative bacteria can be transformed.

In the present invention, the term "transformation" refers to the modification of the genotype of bacteria by the introduction of exogenous polynucleotides, meaning that exogenous polynucleotides are introduced into the bacteria regardless of the method used for the transformation. The exogenous polynucleotides introduced into the bacteria can be maintained either integrated into the bacterial genome or not integrated, and the present invention includes both cases. The exogenous polynucleotides can be inserted into a vector and used for the transformation of the bacteria.

In the present invention, the term "vector" is used for the purpose of replication or expression of the exogenous polynucleotides and generally includes one or more of signal sequences, origins of replication, one or more marker genes, enhancers, promoters, and transcription termination sequences. A plasmid, which is a type of vector, refers to a linear or circular double-stranded DNA molecule to which external polynucleotide fragments can be attached. Other forms of vectors include viral vectors (e.g., replication-defective retroviruses, adenoviruses, and adeno-associated viruses), where additional DNA fragments can be introduced into the viral genome. Certain vectors can autonomously replicate within the bacteria into which they are introduced (e.g., bacterial vectors including bacterial origin and episomal mammalian vectors). Other vectors (e.g., non-episomal mammalian vectors) are integrated into the bacterial genome by introduction into the bacteria and replicate along with the bacterial genome.

In the present invention, the vector can be understood to have the same meaning as "expression vector," which is a type of vector capable of expressing the polynucleotides. A polynucleotide sequence is "operably linked" to a regulatory sequence when the regulatory sequence affects the expression (e.g., level, timing, or location of expression) of the polynucleotide sequence. The regulatory sequence is a sequence that affects the expression (e.g., level, timing, or location of expression) of the nucleic acid to which it is operably linked. The regulatory sequence can exert its influence directly on the regulated nucleic acid or through the action of one or more other molecules (e.g., polypeptides that bind to the regulatory sequence and/or the nucleic acid). The regulatory sequence includes promoters, enhancers, and other expression control elements.

The vector can be introduced into the bacteria by methods known in the art, such as, but not limited to, transient transfection, microinjection, transduction, fusion, calcium phosphate precipitation, liposome-mediated transfection, DEAE dextran-mediated transfection, polybrenemediated transfection, electroporation, gene gun, and other known methods for introducing nucleic acids into bacteria, thereby transforming the bacteria.

In the present invention, the term "expression" means that a protein or polypeptide is produced in the bacteria.

In the present invention, the term "exogenous protein" refers to a protein or peptide that is not endogenously expressed in the bacteria but is expressed in the cytoplasm and/or cell membrane of the bacteria by artificial transformation. The exogenous protein can be induced to be expressed in the bacteria by transforming the bacteria with a vector into which a polynucleotide encoding the protein is inserted.

In a preferred embodiment of the present invention, the exogenous protein can include one or more selected from the group consisting of antigenic proteins and/or antigenic peptides that can be expressed in or exposed to the outer side of the outer membrane of the bacteria, antigenic proteins or antigenic peptides that can be exposed to the inner side of the outer membrane, immunostimulating proteins, immunosuppressive proteins, cell adhesion molecules, antibodies, targeting proteins, cell membrane fusion proteins, cytokines, enzymes, growth factors, extracellular domains of membrane receptors, marker proteins, fragments thereof, and fusion proteins thereof, but is not limited thereto.

In one embodiment of the present invention, the Gram-negative bacteria can be characterized by reduced or knocked-out expression of endogenous proteins. The term "endogenous protein" refers to a protein that is inherent to the Gram-negative bacteria or naturally expressed within the Gram-negative bacteria.

In the present invention, the endogenous proteins whose expression can be reduced or knocked out can include non-essential proteins that are functionally redundant with other endogenous proteins and/or secreted proteins (e.g., for bacterial growth and/or viability, such as in a biomanufacturing environment). In one embodiment, the endogenous proteins whose expression can be reduced or knocked out can be non-essential or associated with secretory functions. In one embodiment, the endogenous proteins whose expression can be reduced or knocked out can be associated with immune function, disease resistance, bacterial communication, and/or secretion. In a preferred embodiment, the endogenous proteins whose expression can be reduced or knocked out can be proteins related to bacterial toxicity.

In some embodiments, reducing the expression of endogenous proteins in bacteria includes removing copies of the genes encoding the endogenous proteins, for example, knocking them out. In some embodiments, reducing the expression of endogenous proteins in bacteria includes removing all (e.g., both) copies of the genes encoding the endogenous proteins from the bacterial genome, for example, knocking them out. In some embodiments, reducing the expression of endogenous proteins in bacteria includes removing regulatory nucleic acid sequences operably linked to the genes encoding the endogenous proteins, for example, promoters or enhancers, for example, knocking them out. In some embodiments, reducing the expression of endogenous proteins in bacteria includes removing all (e.g., both) copies of the regulatory nucleic acid sequences operably linked to the genes encoding the endogenous proteins, for example, promoters or enhancers, for example, knocking them out.

In the present invention, removing the expression of the endogenous proteins, for example, knocking them out, includes introducing deletions, substitutions, or insertion mutations into the genes encoding the endogenous proteins or the regulatory nucleic acid sequences operably linked to the genes encoding the endogenous proteins, for example, promoters or enhancers, which can reduce the expression of the endogenous proteins.

Removing copies of the genes encoding the endogenous proteins or the regulatory elements operably linked to the genes, for example, knocking them out, can be achieved by any gene editing system known in the art. Exemplary gene editing systems include clustered regularly interspaced short palindromic repeats (CRISPR) systems, zinc finger nucleases (ZFN), and transcription activator-like effector nucleases (TALEN). In some embodiments, removing copies of the genes encoding the endogenous proteins or the regulatory elements operably linked to the genes, for example, knocking them out, includes using CRISPR-Cas9 molecules, for example, Cas9 molecules, in combination with RNA specific to the genes encoding the endogenous proteins or the regulatory nucleic acid sequences operably linked to the genes encoding the endogenous proteins, for example, promoters or enhancers (e.g., gRNA, sgRNA, and/or trans-activating crRNA).

In some embodiments, reducing the expression of endogenous bacterial proteins includes reducing the expression of mRNA transcripts encoding the endogenous proteins, such as by deletion in the bacteria. In some embodiments, reducing the expression of mRNA transcripts encoding the endogenous proteins, such as by deletion, includes using, for example, siRNA that can bind to mRNA, which encodes the endogenous proteins or regulatory nucleic acid sequences operably linked thereto, such as promoters or enhancers. Those skilled in the art will know tools and techniques for designing and delivering siRNA to bacteria. Such tools and/or techniques include, but are not limited to, Dharmacon Horizon siDesign tools, InvivoGen siRNA Wizard, GenScript siRNA construct services, IDT Custom Dicer-Substrate siRNA, Sigma-Aldrich siRNA design services, or those taught by www.rnaiweb.com/RNAi/siRNA_Design/.

In one embodiment of the present invention, the transformed bacteria may be transformed to attenuate toxicity, target specific cells or tissues, fuse with the cell membrane of target cells, express substances for treating and/or diagnosing diseases, and simultaneously inhibit specific substances and express specific substances, but are not limited thereto.

In a preferred embodiment of the present invention, the Gram-negative bacteria may be characterized by being transformed to attenuate toxicity. For example, the bacteria may be transformed to attenuate the toxicity of lipopolysaccharides mediating the host inflammatory responses, or may have reduced expression or deletion of endotoxin-producing genes. Specifically, bacteria transformed to have one or more genotypes selected from the group consisting of *ΔmsbB, ΔkdsA, ΔkdsB, ΔlpxB, ΔkdtA, ΔlpxC, AlpxD, Δssc, ΔlpxA,* and *ΔhtrB,* but are not limited thereto.

In one embodiment of the present invention, the Gram-negative bacteria may be transformed by substance treatment or gene introduction and may be transformed two or more times.

In one embodiment of the present invention, the membrane of the OMNVs may further comprise components other than the outer membrane of the bacteria.

The components other than the outer membrane may include targeting substances, cell membrane fusion substances (fusogens), cyclodextrins, polyethylene glycol, hyaluronic acid, etc. Additionally, the components other than the outer membrane may be added by various methods, including chemical modification of the outer membrane.

For example, the membrane components of the OMNVs may be chemically modified using thiol groups (-SH) or amine groups (-NH₂), or the membrane components of the OMNVs may be chemically modified by chemically binding proteins, polyethylene glycol, or hyaluronic acid to the OMNVs. To this end, the method for preparing OMNVs of the present invention may further comprise a step of chemically modifying the membrane components of the OMNVs after step (b).

The present invention also provides OMNVs prepared by the aforementioned method.

In one embodiment of the present invention, the OMNVs according to the present invention may be characterized by being deficient in at least one non-outer membrane component of the bacteria from which they are derived. The "non-outer membrane components" include peptidoglycan, peripheral cytoplasmic proteins, inner membrane proteins, nucleic acids, cytoplasmic proteins, and ribosomes.

In one embodiment of the present invention, the OMNVs according to the present invention may be characterized by being deficient in at least 50%, preferably at least 70%, more preferably at least 90%, and most preferably 100% of at least one non-outer membrane component compared to naturally secreted outer membrane vesicles of the bacteria from which they are derived.

In one embodiment of the present invention, the OMNVs according to the present invention may be characterized by being treated by the following means to reduce adverse effects when administered *in vivo*:
(1) OMNVs may be prepared using genetically transformed bacteria to attenuate toxicity. For example, OMNVs may be prepared using bacteria transformed to attenuate the toxicity of lipopolysaccharides mediating the host inflammatory responses.
(2) The toxicity of the bacteria may be reduced by using drugs that inhibit the activity of endotoxins. An example of such a drug is polymyxin B. The drug may be co-administered with OMNVs, or OMNVs may be prepared from bacteria treated with the drug during culture.
(3) Adverse effects may be reduced by using drugs with anti-inflammatory and/or anticoagulant activities. Such drugs include aspirin. Co-administration of OMNVs and aspirin can prevent adverse effects such as inflammatory responses and blood coagulation caused by OMNVs. Additionally, OMNVs may be prepared from bacteria treated with the drug during culture.
(4) The membrane components of the OMNVs may be chemically modified for use. For example, the membrane components of the OMNVs may be chemically modified using thiol groups or amine groups, or the membrane components of the OMNVs may be chemically modified by chemically binding proteins, polyethylene glycol, or hyaluronic acid to the OMNVs.
(5) The use of sterilized OMNVs can prevent infection by live bacteria. For example, sterilized OMNVs can be obtained through sterilization using ultraviolet and gamma rays or removal of bacteria using filtration.

The method for reducing adverse effects of OMNVs according to the present invention is not limited to the above examples and each of the methods can be used alone or in combination.

In one embodiment of the present invention, the OMNVs according to the present invention may be characterized by exhibiting therapeutic activity for diseases by themselves.

Therefore, the present invention provides a pharmaceutical composition for treating diseases comprising the OMNVs according to the present invention.

Additionally, the present invention provides a pharmaceutical composition for treating diseases consisting of the OMNVs according to the present invention.

The present invention provides a pharmaceutical composition for treating diseases consisting essentially of the OMNVs according to the present invention.

In the present invention, the "disease" may include various diseases, including cancer. In one embodiment of the present invention, the cancer may be selected from the group consisting of thyroid cancer, liver cancer, osteosarcoma, oral cancer, brain tumor, gall bladder cancer, colon cancer, lymphoma, bladder cancer, leukemia, small intestine cancer, tongue cancer, esophageal cancer, renal cancer, gastric cancer, breast cancer, pancreatic cancer, lung cancer, skin cancer, testicular cancer, penile cancer, prostate cancer, ovarian cancer, and cervical cancer, but is not limited thereto. In another embodiment of the present invention, the disease may be selected from the group consisting of hypertension, osteoporosis, irritable bowel syndrome, acute coronary syndrome, stroke, diabetes, atherosclerosis, obesity, peptic ulcer, Alzheimer's disease, emphysema, skin diseases, skin infections, respiratory infections, genitourinary infections, bone and joint infections, central nervous system infections, and sepsis, but is not limited thereto.

In one embodiment of the present invention, the composition may further comprise a substance that enhances therapeutic effects or reduces adverse effects.

The substance that enhances therapeutic effects or reduces adverse effects may be selected from the group consisting of anticancer agents, immunostimulators, STING agonists, anti-inflammatory agents, endotoxin inhibitors, peptides, proteins, toxins, nucleic acids, beads, microparticles, and nanoparticles, but is not limited thereto.

The "anticancer agent" blocks the processes of replication, transcription, and translation of DNA in cancer cells. The types of anticancer agents that can be used as therapeutic substances in the present invention are not particularly limited. Anticancer agents can be selected based on general principles considered when selecting anticancer agents, such as the type of cancer cells, absorption rate of the anticancer agent (treatment period and administration route), location of the tumor, and size of the tumor. The anticancer agents that can be used in the present invention include DNA crosslinking agents such as cyclophosphamide, chlormethine, uramustine, melphalan, chlorambucil, ifosfamide, bendamustine, carmustine, lomustine, streptozotocin, busulfan, thiotepa, cisplatin, and carboplatin; anti-cancer antibiotics such as dactinomycin (actinomycin D), doxorubicin (adriamycin), epirubicin, idarubicin, mitoxantrone, plicamycin, daunorubicin, mitomycin C, and bleomycin; plant alkaloids such as vincristine, vinblastine, paclitaxel, docetaxel, etoposide, teniposide, topotecan, and irinotecan; antibodies such as herceptin and rituximab, but are not limited thereto.

The "anti-inflammatory agent" may be selected from the group consisting of dexamethasone, indomethacin, ibuprofen, clobetasol propionate, diflorasone diacetate, halobetasol propionate, amcinonide, fluocinonide, mometasone furoate, desoximetasone, diclofenac, and piroxicam, but is not limited thereto.

The "peptides" and "proteins" may include various growth factors such as VEGF and EGF, cytokines such as IL-1, IFN-gamma, and IL-10, various antibody therapeutics, various peptides or proteins, DNase, and various proteins and peptides that can inhibit cancer growth and metastasis and suppress inflammatory responses, without limitation.

The "toxins" are collectively referred to as those derived from various organisms that can exhibit toxicity when absorbed into the body, and can induce cell death through the toxins. The types of toxins in the present invention are not particularly limited.

The "nucleic acids" may be selected from the group consisting of DNA, RNA, aptamers, locked nucleic acids (LNA), peptide nucleic acids (PNA), and morpholinos, but are not limited thereto.

The "nanoparticles" may be selected from the group consisting of iron oxide, gold, carbon nanotubes, and magnetic beads, but are not limited thereto.

In one embodiment of the present invention, the substance that enhances therapeutic effects or reduces adverse effects may be loaded into the OMNVs.

In the present invention, the term "loading" means displaying the necessary substance to the surface of OMNV membrane or encapsulating the substance inside the OMNVs, but is not limited thereto.

The pharmaceutical composition of the present invention may further comprise a pharmaceutically acceptable carrier in addition to the active ingredient. That is, it can be used by mixing one or more of saline, sterile water, Ringer's solution, buffered saline, cyclodextrin, dextrose solution, maltodextrin solution, glycerol, ethanol, liposome, and other components, and may further comprise other conventional additives such as antioxidants and buffers as needed. Additionally, diluents, dispersants, detergents, binders, and/or lubricants may be additionally added to formulate injectable formulations (aqueous solutions, suspensions, and emulsions), pills, capsules, granules, or tablets. Furthermore, it can be preferably formulated according to each component using methods disclosed in the relevant technical field. The pharmaceutical composition of the present invention is not particularly limited in formulation but is preferably formulated as an injectable or inhalable formulation.

The method of administering the pharmaceutical composition of the present invention is not particularly limited, but may be administered orally or parenterally, such as intravenously, subcutaneously, intraperitoneally, intranasally, by inhalation, or topically, depending on the intended method. The dosage varies depending on the patient's weight, age, gender, health condition, diet, administration time, administration method, excretion rate, and severity of the disease. The daily dosage means the amount of the therapeutic substance of the present invention sufficient to treat the reduced disease status by administering it to a subject in need of treatment. The effective amount of the therapeutic substance varies depending on the specific compound, disease status, and its severity, and the subject in need of treatment, and can be routinely determined by those skilled in the art. As a non-limiting example, the dosage of the composition of the present invention for humans may vary depending on the patient's age, weight, gender, administration form, health condition, and disease severity, and generally ranges from 0.1 to 1000 mg/day, preferably 1 to 500 mg/day, based on an adult patient weighing 70 kg, and may be administered once or several times a day at regular intervals.

In one embodiment of the present invention, the pharmaceutical composition may further comprise a drug that suppresses the toxicity caused by the OMNVs. Additionally, the drug may be loaded into the OMNVs. The drug may include a drug that suppresses the toxicity caused by endotoxins, such as polymyxin B.

The present invention also provides a vaccine composition for preventing or treating a disease comprising the OMNVs according to the present invention. The term "vaccine" means artificially injecting an antigen into the human body to activate the immune system to prevent or treat a disease.

According to one embodiment of the present invention, the OMNVs according to the present invention have been confirmed to have excellent activity in inducing innate immunity by themselves and can be used as an immune adjuvant (i.e., vaccine adjuvant) that increases the immune response in the body when administered with an antigen.

The term "immune adjuvant", "vaccine adjuvant", or "adjuvant" means a pharmaceutical or immunological agent administered to enhance the immune response of a vaccine, i.e., to increase the immune response in the body caused by the antigen.

In one embodiment of the present invention, the vaccine composition may further comprise an antigen.

The term "antigen" can be interpreted synonymously with "immunogen," meaning it can induce the formation of antibodies and/or a cellular immune response, specifically activating a particular immune response, and includes proteins, recombinant proteins, glycoproteins, peptides, polysaccharides, lipopolysaccharides, or polynucleotides of pathogens.

In one embodiment of the present invention, the antigen may be derived from one or more infectious pathogens selected from bacteria, viruses, and fungi. For example, the antigen may be glycoprotein E (gE) antigen of varicella zoster virus; glycoprotein E (gE) antigen of Japanese encephalitis virus; an inactivated antigen of seasonal influenza virus; haemagglutinin antigen and neuraminidase antigen of influenza virus; spike antigen of coronavirus; pertussis toxin antigen, filamentous haemagglutinin antigen, and pertactin antigen of *Bordetella pertussis*; an antigen of human papilloma virus (HPV); capsular polysaccharide antigen of groups A, B, C, Y, and W-135 of *Helicobacter pylori*; tetanus toxoid antigen of *Clostridium tetani*; diphtheria toxoid antigen of *Corynebacterium diphtheriae*; type 3 capsular polysaccharide antigen of *Streptococcus pneumoniae*; an antigen of *Mycobacterium tuberculosis*; GP-120 and GP-160 antigens of human immunodeficiency virus (HIV); cholera toxin B subunit antigen of *Vibrio cholerae*; staphylococcal enterotoxin B antigen of *Staphylococcus*; Shigella polysaccharides antigen of *Shigella*; vesicular stomatitis virus glycoprotein antigen of vesicular stomatitis virus; an antigen of cytomegalovirus (CMV); an antigen of hepatitis A/B/C/D/G virus; an antigen of respiratory syncytial virus (RSV); or an antigen of herpes simplex virus, but is not limited thereto.

In one embodiment of the present invention, the antigen may be a cancer antigen. For example, the cancer antigen may be HPV-derived antigen, carcinoembryonic antigen, prostate-specific antigen (PSA), prostate-specific membrane antigen (PSMA), mucin-1 (MUC-1), BCR/ABL, alpha-fetoprotein (AFP), Epstein-Barr virus (EBV)-derived antigen, HBV-derived antigen, HCV-derived antigen, cancer antigen-125 (CA-125), cancer antigen-72-4 (CA-72-4), cancer antigen-15-3 (CA-15-3), cancer antigen-19-9 (CA-19-9), or a mutant Ras protein, Raf protein, Src protein, Myc protein, EGFR, PDGFR, VEGFR, p53, PTEN, or HER2/neu, but is not limited thereto.

In one embodiment of the present invention, the antigen may be loaded into the OMNVs according to the present invention or included in the composition along with a separate carrier.

The vaccine composition may be administered in combination with commonly used adjuvants, such as aluminum hydroxide, aluminum phosphate, alum, MF59, virosome, AS04 (a mixture of aluminum hydroxide and monophosphoryl lipid A, MPL), AS03 (a mixture of DL-α-tocopherol, squalene, and polysorbate 80), CpG DNA, flagellin, Poly I:C, AS01 (a mixture of QS-21, MPL, and liposome), AS02 (a mixture of QS-21, MPL, and oil-in-water emulsion), immune stimulating complexes (ISCOMs), and ISCOM matrix, but is not limited thereto. Additionally, the vaccine composition may be administered in combination with additional drugs to enhance the efficacy of the vaccine or reduce adverse effects.

In one embodiment of the present invention, the vaccine composition may be for preventing or treating infectious diseases caused by bacteria, viruses, or fungi. In the present invention, the infection may be selected from the group consisting of skin infections, respiratory infections, genitourinary infections, bone and joint infections, central nervous system infections, and sepsis, but is not limited thereto.

In one embodiment of the present invention, the vaccine composition may be for preventing or treating cancer. In the present invention, the cancer may be selected from the group consisting of thyroid cancer, liver cancer, osteosarcoma, oral cancer, brain tumor, gallbladder cancer, colon cancer, lymphoma, bladder cancer, leukemia, small intestine cancer, tongue cancer, esophageal cancer, renal cancer, gastric cancer, breast cancer, pancreatic cancer, lung cancer, skin cancer, testicular cancer, penile cancer, prostate cancer, ovarian cancer, and cervical cancer, but is not limited thereto.

In one embodiment of the present invention, the vaccine composition may be for preventing or treating diseases selected from the group consisting of hypertension, osteoporosis, irritable bowel syndrome, acute coronary syndrome, stroke, diabetes, atherosclerosis, obesity, peptic ulcer, Alzheimer's disease, chronic obstructive pulmonary disease, asthma, skin diseases, and autoimmune diseases, but is not limited thereto.

The route of administration of the vaccine adjuvant or the vaccine composition comprising the same according to one embodiment of the present invention may be any general route as long as it can reach the target tissue. Such routes of administration may include transdermal, mucosal administration (e.g., oral, nasal, rectal, or vaginal) or parenteral administration, such as intraperitoneal, intravenous, intramuscular, subcutaneous, or intra-synovial cavity administration, but are not limited thereto.

The vaccine composition according to the present invention may be formulated in a suitable form together with a pharmaceutically acceptable carrier. Pharmaceutically acceptable carriers may include, for example, carriers for parenteral administration such as water, suitable oils, saline, water-soluble glucose, and glycol, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. Additionally, the composition according to the present invention may appropriately include suspending agents, solubilizing agents, stabilizers, isotonic agents, preservatives, anti-adsorption agents, detergents, diluents, excipients, pH adjusters, analgesics, buffers, antioxidants, etc., as necessary depending on the method of administration or formulation. Pharmaceutically acceptable carriers and formulations suitable for the present invention, including those exemplified above, are detailed in the literature [Remington's Pharmaceutical Sciences, the latest edition].

The dosage of the vaccine composition for a patient may vary depending on many factors, including the patient's height, body surface area, age, the specific compound being administered, gender, administration time and route, general health, and other drugs being administered simultaneously. Pharmaceutically active DNA may be administered in an amount of 100 ng/kg body weight to 10 mg/kg body weight, more preferably 1 to 500 µg/kg body weight, and most preferably 5 to 50 µg/kg body weight, with the dosage being adjusted considering the above factors.

Furthermore, the vaccine composition of the present invention is administered in a pharmaceutically effective amount.

The present invention also provides the use of OMNVs according to the present invention for manufacturing a pharmaceutical composition for treating a disease.

The present invention also provides a method for treating a disease comprising administering an effective amount of a pharmaceutical composition OMNVs according to the present invention to a subject in need thereof.

The term "pharmaceutically effective amount" as used in this document refers to an amount sufficient to treat a disease with a reasonable benefit/risk ratio applicable to medical treatment, and the effective dosage level can be determined based on factors such as the type and severity of the condition, age, gender, drug activity, sensitivity to the drug, administration time, administration route and excretion rate, treatment duration, concurrent medications, and other factors well known in the medical field. The vaccine composition of the present invention can be administered in a dosage of 0.1 mg/kg to 1 g/kg, and more preferably in a dosage of 1 mg/kg to 500 mg/kg. Meanwhile, the dosage can be appropriately adjusted according to the age, gender, and condition of the patient.

The 'effective amount' of the present invention refers to an amount that, when administered to a subject, exhibits an effect of improving, treating, detecting, diagnosing, inhibiting or reducing cancer and the disease of the present invention, and the 'subject' may be an animal, preferably a mammal, particularly an animal including a human, and may also be cells, tissues, organs, etc., derived from animals. The subject may be a patient in need of the effect.

The 'treatment' of the present invention comprehensively refers to improving symptoms caused by cancer or the aforementioned disease, which may include curing, substantially preventing, or improving the condition, and includes alleviating, curing, or preventing one or most of the symptoms derived from the disease, but is not limited thereto.

The term "comprising" as used herein is synonymous with "including" or "characterized by," and does not exclude additional components or steps not specifically mentioned in the composition or method according to the present invention. The term "consisting of" means excluding additional elements, steps, or components not specifically described. The term "consisting essentially of" means that the scope of the composition or method may include substances or steps that do not substantially affect the basic characteristics of the described substances or steps.

### BRIEF DESCRIPTION OF THE DRAWINGS

Figure 1 shows a schematic diagram of a method for preparing Gram-negative bacterial outer membrane-derived nanovesicles (OMNVs).

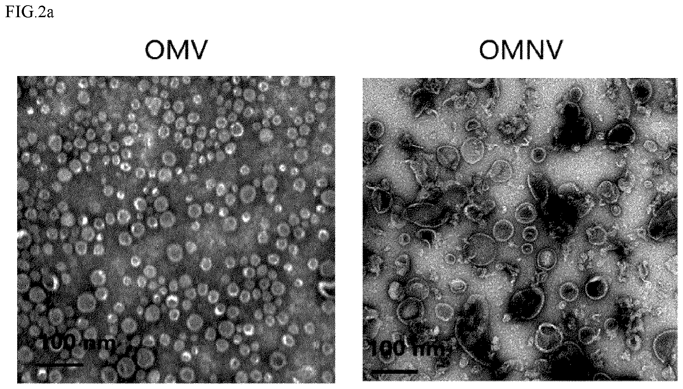

Figures 2a to 2i show characterization of Gram-negative bacterial outer membrane vesicles (OMVs) and OMNVs. (a) Transmission electron microscope images, (b) size distribution determined by dynamic light scattering analysis, (c-d) yield ((c)-total particle numbers per culture volume, and (d)-total protein amounts per culture volume), (e) total particle numbers per total protein amounts, (f) SDS-PAGE results, and (g) Western blot results using anti-OmpA antibody. In addition, there are also (h) the results of measuring AmCyan fluorescence value per culture volume and (i) total nucleic acid amount in whole cell lysates and OMNVs. a: Scale bars = 100 nm.
Figures 3a to 3d show the results of treating OMVs and OMNVs to (a) HEK-Blue^{™}hTLR2 and (b) HEK-Blue^{™}hTLR4, and the degree of responses is depicted as graphs. Additionally, there are also the results of measuring the concentration of (c) TNF-α and (d) IL-6 in the conditioned medium by ELISA after treating OMVs and OMNVs to mouse macrophage RAW264.7.
Figures 4a to 4e show characterization of OMNVs manufactured from various Gram-negative bacteria. (a) Transmission electron microscope images, (b) size distribution determined by dynamic light scattering analysis, (c-d) yield (c-total particle numbers per culture volume, d-total protein amounts per culture volume), and (e) total particle numbers per total protein amounts. a: Scale bars = 200 nm.
Figures 5a to 5b show the results of treating OMNVs manufactured from various Gram-negative bacteria to (a) HEK-Blue^{™}hTLR2 and (b) BEK-Blue^{™}hTLR4, and the degree of responses is depicted as graphs. Additionally, there are also the results of measuring the concentration of (c) TNF-α and (d) IL-6in the conditioned medium by ELISA after treating OMNVs derived from various Gram-negative bacteria to RAW264.7.
Figure 6 shows the results of adding polyethylene glycol on the surface of OMNVs.
Figures 7a to 7b show the results of confirming whether OMNVs loaded with daunorubicin (DNR), an anticancer drug, during manufacturing (OMNV_{DNR}) can deliver daunorubicin to target cells by (a) fluorescence microscopy and (b) flow cytometry. a: Scale bars = 20 µm.
Figures 8a to 8b show the results of confirming whether OMNVs loaded with daunorubicin by incubation after manufacturing (OMNV_{DNR-Inc}) can deliver daunorubicin to target cells by (a) fluorescence microscopy and (b) flow cytometry. a: Scale bars = 20 µm.
Figures 9a to 9b show the results of confirming whether OMNVs loaded with daunorubicin by sonication after manufacturing (OMNV_{DNR-Son}) can deliver daunorubicin to target cells by (a) fluorescence microscopy and (b) flow cytometry. a: Scale bars = 20 µm.
Figures 10a to 10b show the results of confirming whether OMNVs loaded with daunorubicin by freezing-thawing after manufacturing (OMNV_{DNR-FT}) can deliver daunorubicin to target cells by (a) fluorescence microscopy and (b) flow cytometry. a: Scale bars = 20 µm.
Figure 11 shows the results of measuring IL-6 release by ELISA after treating OMNVs loaded with dexamethasone (DEX), an anti-inflammatory drug (OMNV_{DEX}) to RAW264.7.
Figures 12a to 12b show the results of confirming whether OMNVs loaded with GFP (OMNV_{GFP}) can deliver GFP to target cells by (a) fluorescence microscopy and (b) flow cytometry. a: Scale bars = 20 µm.
Figure 13 shows the results of confirming adverse effects after intraperitoneal administration of OMNVs into mice.
Figures 14a to 14e show the results of determining *in vivo* distribution over time after intraperitoneal administration of OMNVs labeled with Cy7 (^{Cy7}OMNV) into mice. (a, b) The distribution over time in the whole body of the mouse by IVIS and (c) the distribution in major organs 24 hours after administration were observed, and (d-e) the degree of distribution in major organs was shown in graphs.
Figures 15a to 15b show the graphical results of the change in size of cancer tissue over time after administrating OMNVs into cancer after introducing mouse bladder cancer cell line MB49 into mice.
Figures 16a to 16d show the results of determining (a) the cell viability after treating OMNVs to dendritic cells, and measuring the concentration of (b) TNF-α, (c) IL-6, and (d) IL-12p40 in the conditioned medium by ELISA.
Figures 17a to 17b show the results of confirming whether dendritic cells uptake OMNVs labeled with Alexa 594 (Alexa ⁵⁹⁴OMNV) by treating the OMNVs to dendritic cells by (a) fluorescence microscopy and (b) flow cytometry. a: Scale bars = 5 µm.
Figures 18a to 18e show (a) the experimental schedule of intraperitoneally administering a mixture of OMNVs and an antigen (GFP or SARS-CoV-2 S protein) with an adjuvant (Alum) or CuSO₄ into mice, and (b-e) the graphical results of the serum IgG titer against GFP or S protein 7, 14, and 21 days after administration.
Figures 19a to 19e show (a) the experimental schedule of intraperitoneally administering a mixture of OMNVs and an antigen (GFP or SARS-CoV-2 S protein) with an adjuvant (Alum) or CuSO₄ into mice, and (b-e) the graphical results of the serum IgG titer against GFP or S protein 21 and 49 days after administration.
Figures 20a to 20e show (a) the experimental schedule of intraperitoneally administering a mixture of OMNVs and an antigen (GFP or SARS-CoV-2 S protein) with an adjuvant (Alum) or CuSO₄ into mice, and (b-e) the graphical results of the T cell response (IFN-γ secretion) against GFP or S protein 49 days after administration.
Figures 21a to 21e show (a) the experimental schedule of intraperitoneally or intramuscularly administering a mixture of OMNVs and an antigen (HSN1 hemagglutinin) with an adjuvant (Alum) into mice, and (b) the graphical results of the serum IgG titer against H5N1 hemagglutinin 7, 14, 21, and 45 days after administration (b, c - intraperitoneal administration; d, e - intramuscular administration).
Figures 22a to 22e show (a) the plasmid map for expressing an antigen (S1 RBD; SARS-CoV-2 Spike protein 1 receptor-binding domain or HSN1 hemagglutinin) in bacteria based on the outer membrane protein display system, (b) the yield, average diameter, and purity when isolating OMNVs expressing the antigen (^{S1 RBD-OmpA}OMNV, ^{S1 RBD-LOlB}OMNV, ^{H5N1-LolB}OMNV), and (c-e) Western blot results using anti-His₆ antibody for OMNVs the antigen.
Figures 23a to 23f show the results of measuring the concentration of TNF-α (a, d), IL-6 (b, e), and IL-12p40 (c, f) in conditioned medium by ELISA after treating OMNVs expressing the antigen based on the outer membrane protein display system to dendritic cells.
Fig. 24a to 24e show (a) the experimental schedule for intraperitoneally administering OMNVs expressing antigens based on the outer membrane protein display system into mice, (b, c) the serum IgG titer against S1 RBD on days 5, 11, and 17 after administration, and (d) the serum IgG titer against S1 RBD and (e) T-cell response (IFN-γ secretion) against S1 RBD on day 49 after administration.
Fig. 25a to 25c show (a) the experimental schedule for intramuscularly administering OMNVs expressing antigens based on the outer membrane protein display system into mice, and (b, c) the serum IgG titer against S1 RBD or HSN1 hemagglutinin on days 5, 11, and 17 after administration.
Fig. 26a to 26f show the survival rate (a-c) and body weight change (d-f) of mice when OMNVs expressing antigens based on the outer membrane protein display system are intraperitoneally administered into mice.
Fig. 27a to 27b show (a) the schematic diagram of the method for loading proteins into OMNVs and (b) the amount of protein loaded into OMNVs.
Fig. 28a to 28e show characterization of OMNVs isolated by various methods. (a)transmission electron microscopy images, (b) the size distribution determined by dynamic light scattering analysis, (c, d) yield ((c)-total particle numbers per culture volume, (d)-total protein amounts per culture volume), and (e) total particle numbers per total protein amounts. a: Scale bars = 200 nm.
Fig. 29a to 29d show the degree of responses after treating OMNVs isolated by various methods to (a) HEK-Blue^{™}hTLR2 and (b) HEK-Blue^{™}hTLR4. There are also the concentration of (c) TNF-α and (d) IL-6 in the conditioned medium measured by ELISA after treating OMNVs isolated by various methods to RAW264.7.
Fig. 30 shows the change in the size of the tumor tissue over time when OMNVs isolated by various methods are intratumorally administered after introducing the mouse bladder cancer cell line MB49 into mice.
Fig. 31 shows a schematic diagram of various manufacturing methods and applications of OMNVs.

### DETAILED DESCRIPTION OF THE INVENTION

Hereinafter, the present invention will be described in more detail through examples. These examples are merely illustrative of the present invention, and it will be apparent to those skilled in the art that the scope of the present invention is not limited by these examples.

### Example 1. Preparation of Gram-negative Bacterial Outer Membrane Nanovesicles (OMNVs)

To prepare OMNVs, *Escherichia coli* BL21 (DE3) *ΔmsbB*-AmCyan was cultured in vegetable peptone-based lysogeny broth (VP-LB), and the culture was centrifuged at 6,000 × g for 20 minutes at 4°C to obtain the bacteria by removing the supernatant. The obtained bacteria were suspended in Tris-based saline (TBS; 50 mM Tris-HCl (pH 8.0), 138 mM NaCl, 2.7 mM KCl) at a ratio of 100 mL per 1 L of culture medium. The suspension was centrifuged at 6,000 × g for 20 minutes at 4°C to wash the bacterial cells by removing the supernatant.

The washed bacterial cells were rapidly frozen using liquid nitrogen, then suspended in 30 mL of Buffer A (10 mM Tris-HCl (pH 8.0), 5% sucrose), and treated with Sarkosyl at the final concentration of 1% at room temperature for 30 minutes. The suspension was centrifuged at 10,000 × g for 20 minutes at 4°C to remove the supernatant. Then, the pellet was suspended in 30 mL of Buffer A and treated with Sarkosyl at a final concentration of 0.25% at 4°C for 10 minutes. The suspension was centrifuged at 10,000 × g for 20 minutes at 4°C to remove the supernatant. The pellet was suspended in 50 mL of Buffer A, and the process of centrifuging the suspension at 10,000 × g for 20 minutes at 4°C and removing the supernatant was repeated three times to remove Sarkosyl and isolate Gram-negative bacterial outer membrane.

Gram-negative bacterial outer membrane was suspended in 50 mL of Buffer A, and OMNVs were prepared by passing through a microfluidizer processor (Microfluidics, MA) at 10,000 psi for four cycles. The solution passed through the microfluidizer was centrifuged at 6,000 × g for 20 minutes at 4°C to obtain the supernatant, and substances larger than 500 kDa were concentrated using tangential flow filtration, and dialysis was performed using Buffer A to obtain about 7 mL of sample. Finally, 3 mL of 2.5 M sucrose, 3 mL of 0.8 M sucrose, and 7 mL of the sample were sequentially placed in an ultracentrifuge tube, and ultracentrifuged at 200,000 × g for 2 hours at 4°C to finally obtain OMNVs from the layer between 2.5 M sucrose and 0.8 M sucrose.

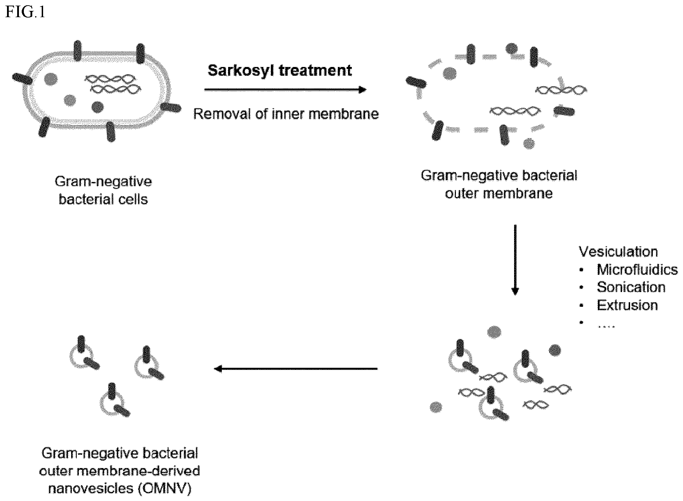

The method for preparing OMNVs is schematically shown in Figure 1.

### Example 2. Isolation of Gram-Negative Bacterial Outer Membrane Vesicles (OMVs)

The bacteria used to isolate OMVs were *E. coli* BL21 (DE3) *ΔmsbB*-AmCyan. The bacteria were cultured in VP-LB, and the culture was centrifuged at 6,000 × g for 20 minutes at 4°C. The supernatant was filtered using a 0.45 µm pore-sized filter, and the filtrate was concentrated to substances larger than 100 kDa using tangential flow filtration. The concentrate was ultracentrifuged at 100,000 × g for 2 hours at 4°C, and the pellet was suspended in 50% iodixanol. In an ultracentrifuge tube, 2 mL of 50% iodixanol suspension, 1.5 mL of 40% iodixanol, and 1 mL of 10% iodixanol were sequentially placed, and ultracentrifuged at 200,000 × g for 2 hours at 4°C to isolate OMVs from the layer between 40% iodixanol and 10% iodixanol.

### Example 3. Characterization of OMVs and OMNVs

### 3-1. Analysis of the Morphology and Size of OMVs and OMNVs

To analyze the morphology of the OMNVs prepared by the method of Example 1 and the OMVs isolated by the method of Example 2, they were observed using a transmission electron microscope. The OMVs and OMNVs were adsorbed on a glow-discharged carbon-coated copper grid for 20 minutes. The grid was washed with distilled water, stained with 1% uranyl acetate for 2 seconds, and observed with a transmission electron microscope (JEM1011, JEOL, Japan). As a result, as shown in Figure 2a, it was confirmed that both the OMVs and OMNVs were composed of lipid bilayers and had a diameter of 50-100 nm.

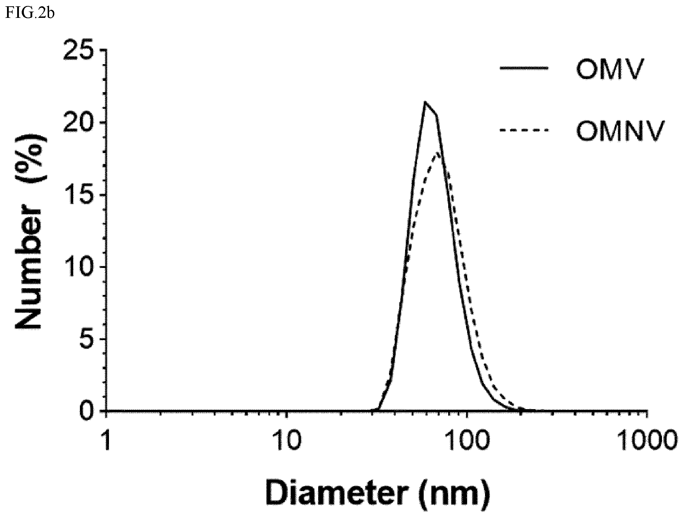

In addition, to measure the size of the OMVs and OMNVs, they were diluted to a concentration of 1 µg/mL in HEPES-buffered saline (HBS, 20 mM HEPES (pH 7.4), 150 mM NaCl) and analyzed with a dynamic light scattering particle size analyzer (Zetasizer Nano ZS, Malvern Instruments, UK). As a result, as shown in Figure 2b, it was confirmed that the average diameters of the OMVs and OMNVs were 70.1 ± 11.4 nm and 74.3 ± 15.8 nm, respectively.

### 3-2. Analysis of Total Particle Number and Total Protein Amount of OMVs and OMNVs

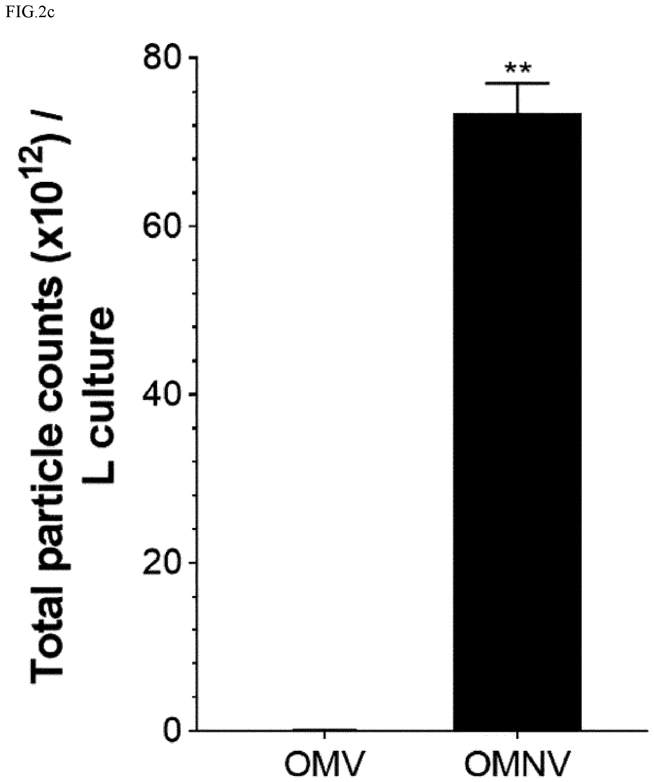

To measure total particle numbers of the OMVs and OMNVs, they were diluted to the concentration of 1 µg/mL in HBS, placed in a chamber, and analyzed with a nanoparticle tracking analyzer (Nanosight LM-10, Malvern Instruments, UK). As a result, as shown in Figure 2c, it was confirmed that the OMVs and OMNVs could be obtained at an average of (1.33 ± 0.24) × 10¹¹ particles and (7.35 ± 3.54) × 10¹³ particles per 1 L of culture, respectively, confirming that the OMNVs could be obtained about 550 times more than the OMVs based on total particle numbers.

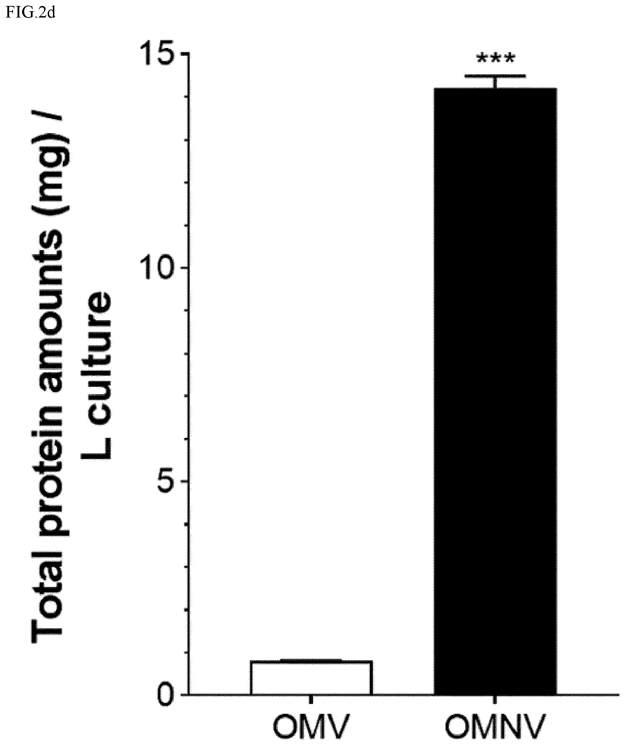

In addition, total protein amounts of the OMVs and OMNVs was quantified by Bradford assay. As a result, as shown in Figure 2d, it was confirmed that the OMVs and OMNVs could be obtained at an average of 0.812 ± 0.001 mg and 14.200 ± 0.283 mg per 1 L of culture, respectively, confirming that the OMNVs could be obtained about 17 times more than the OMVs based on total protein amounts.

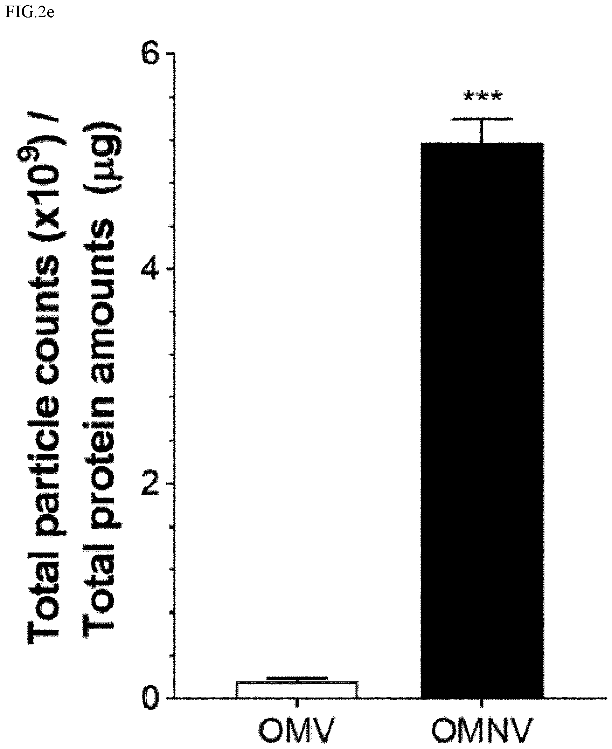

Furthermore, to confirm the purity of the OMVs and OMNVs, total particle numbers per total protein amounts were calculated. As a result, as shown in Figure 2e, it was confirmed that the OMVs and OMNVs had purities of (0.164 ± 0.024) × 10⁹ particles/µg and (5.177 ± 0.220) × 10⁹ particles/µg, respectively, confirming that the OMNVs had a purity about 32 times higher than the OMVs.

### 3-3. Analysis of Protein Distribution and the Presence of Outer Membrane Proteins in OMVs and OMNVs

First, to prepare whole cell lysates, *E. coli* BL21 (DE3) *ΔmsbB*-AmCyan culture was centrifuged at 6,000 × g for 20 minutes at 4°C. After removing the supernatant, the pellet was suspended in RIPA buffer (50 mM Tris-HCl (pH 8.0), 150 mM NaCl, 1% NP-40, 0.1% SDS, 0.5% sodium deoxycholate, 1 mM EDTA, 1 mM PMSF). Then, the suspension was sonicated to obtain whole cell lysates.

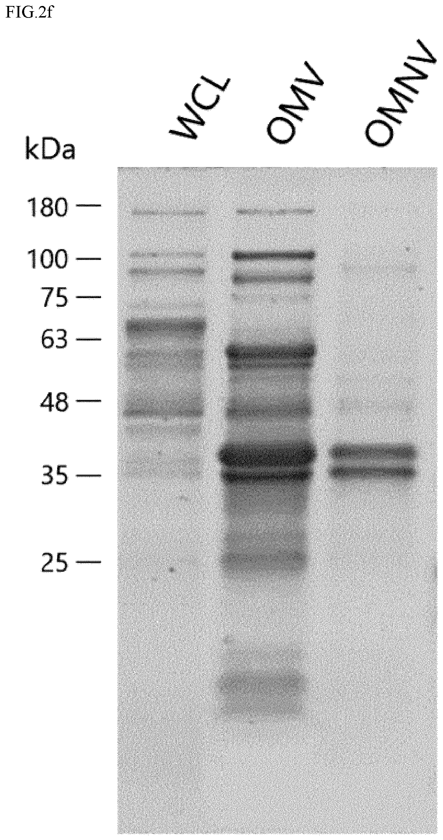

To verify the protein distribution of whole cell lysates, OMVs, and OMNVs, a total of 5 µg of whole cell lysates, OMVs, and OMNVs were subjected to non-reducing sodium dodecyl sulfate-polyacrylamide gel electrophoresis (SDS-PAGE) and stained with Coomassie Brilliant Blue. As shown in Figure 2f, unlike whole cell lysates and OMVs, which showed various protein bands, OMNVs exhibited a limited number of protein bands.

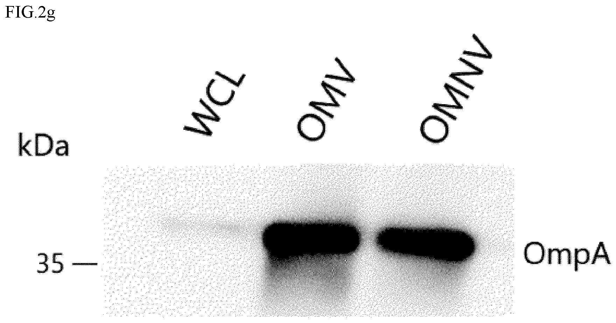

To verify the presence of outer membrane proteins in whole cell lysates, OMVs and OMNVs, a total of 1 µg of whole cell lysates, OMVs, and OMNVs were subj ected to non-reducing electrophoresis and transferred to a polyvinylidene fluoride (PVDF) membrane using semi-dry transfer. The membrane was then blocked with 3% non-fat milk/TBS-T (0.05% Tween 20) solution for 1 hour, followed by incubation with a primary antibody specific to OmpA protein at room temperature for 2 hours. After washing the membrane three times with TBS-T (0.05% Tween 20), a peroxidase-conjugated secondary antibody was applied and incubated at room temperature for 1 hour. The membrane was washed three times with TBS-T (0.05% Tween 20) again, and the expression of OmpA protein was detected using enhanced chemiluminescence (ECL) substrate. As shown in Figure 2g, OmpA was more highly expressed in OMVs and OMNVs compared to whole cell lysates.

### 3-4. Measurement of AmCyan Fluorescence and Total Nucleic Acid Amount in Whole Cell Lysates and OMNVs

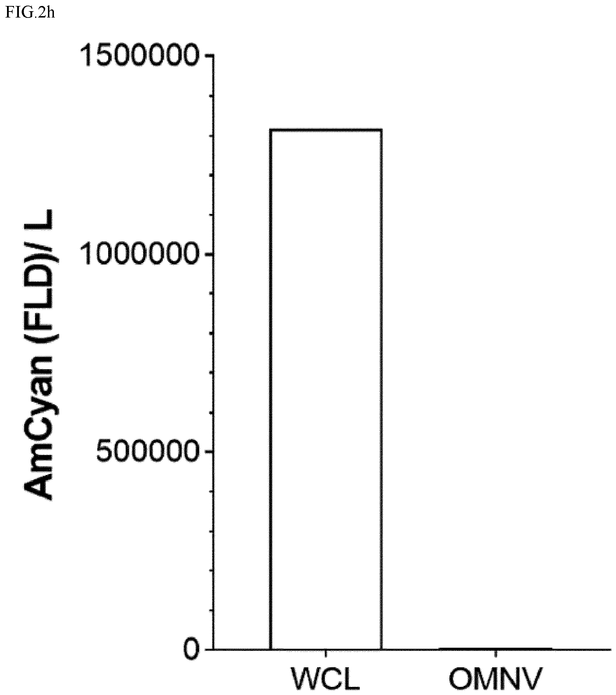

To verify the presence of cytoplasmic proteins in whole cell lysates and OMNVs, the relative amount of the cytoplasmic fluorescent protein AmCyan was measured. Fluorescence was measured using a spectrophotometer at an excitation wavelength of 457 nm and an emission wavelength of 491 nm. As shown in Figure 2h, OMNVs contained almost no cytoplasmic fluorescent proteins compared to whole cell lysates.

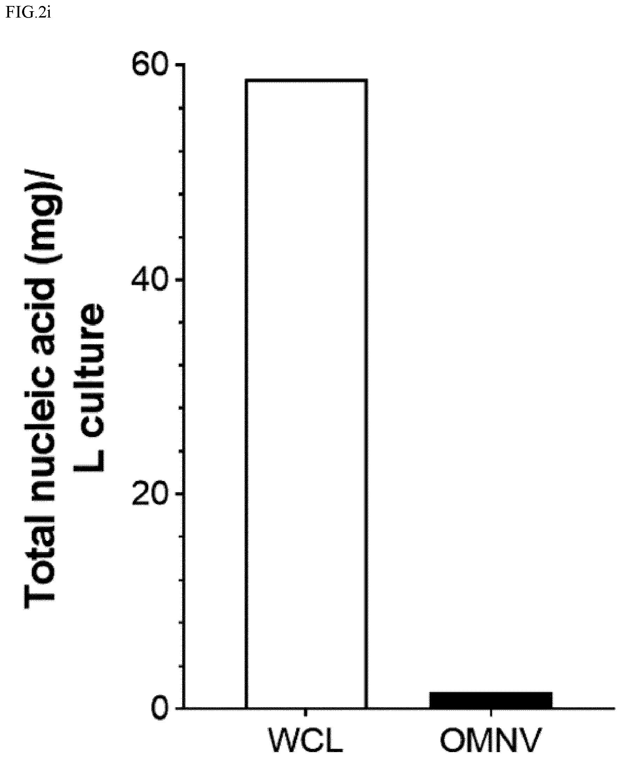

To verify total nucleic acid contents in whole cell lysates and OMNVs, absorbance at the wavelength of 260 nm was measured using a micro-spectrophotometer. As shown in Figure 2i, OMNVs contained almost no nucleic acids compared to whole cell lysates.

In summary, a method for producing and purifying high-purity OMNVs with a production yield increased by tens to hundreds of times compared to OMVs, and efficiently removing unnecessary substances such as cytoplasmic proteins and nucleic acids by disrupting the bacterial inner membrane, was established.

### Example 4. Measurement of Innate Immune Inducing Activity of OMVs and OMNVs

To measure innate immune inducing activity of the OMNVs prepared by the method of Example 1 and the OMVs isolated by the method of Example 2, OMVs and OMNVs were treated to BEK-Blue^{™}hTLR2 and HEK-Blue^{™}hTLR4, and the degree of responses was determined. Additionally, cytokine release was measured by treating them to mouse macrophages RAW264.7.

To measure the degree of responses of HEK-Blue^{™}hTLR2 and HEK-Blue^{™}hTLR4 to OMVs and OMNVs, each cell (2×10⁵ cells/well) was attached to a well plate for 24 hours. Then, various concentrations (0.00001, 0.0001, 0.001, 0.01, 0.1, 1, 10, 100, 1000, 10000 ng/mL) of OMVs and OMNVs were treated to each cell for 18 hours, and the conditioned medium was collected. The collected conditioned medium was reacted with a chemiluminescence substrate to measure RLU, and RLU was normalized using the RLU of the conditioned medium from the cells not treated either with OMVs or OMNVs.

To quantify the degree of responses of RAW264.7 to OMVs and OMNVs, RAW264.7 (5×10⁴ cells/well) was attached to a well plate for 24 hours. Then, various concentrations (0, 0.1, 0.3, 1, 3, 10, 30, 100, 300, 1000 ng/mL) of OMVs and OMNVs were treated to RAW264.7 for 24 hours, and conditioned medium was collected. mTNF-α and mIL-6 in the collected conditioned medium were quantified by ELISA.

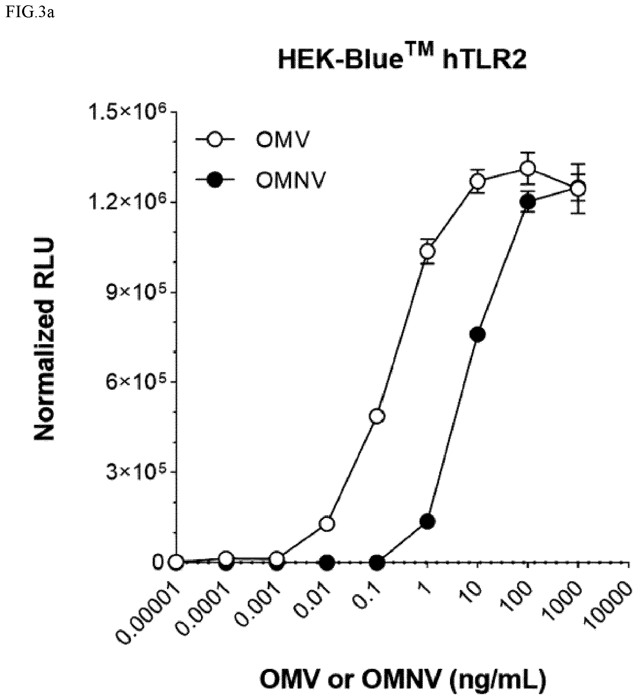

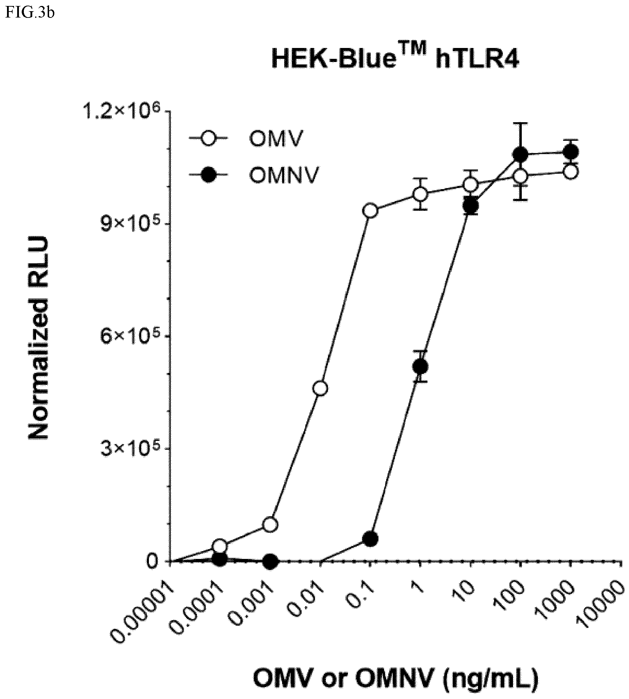

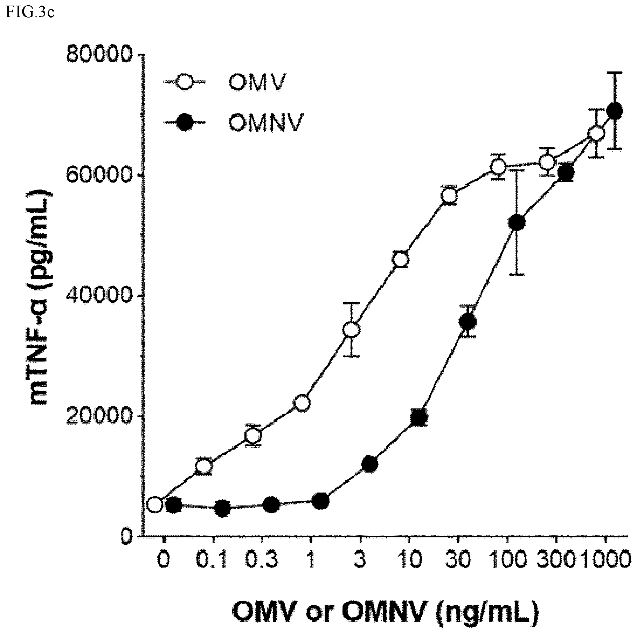

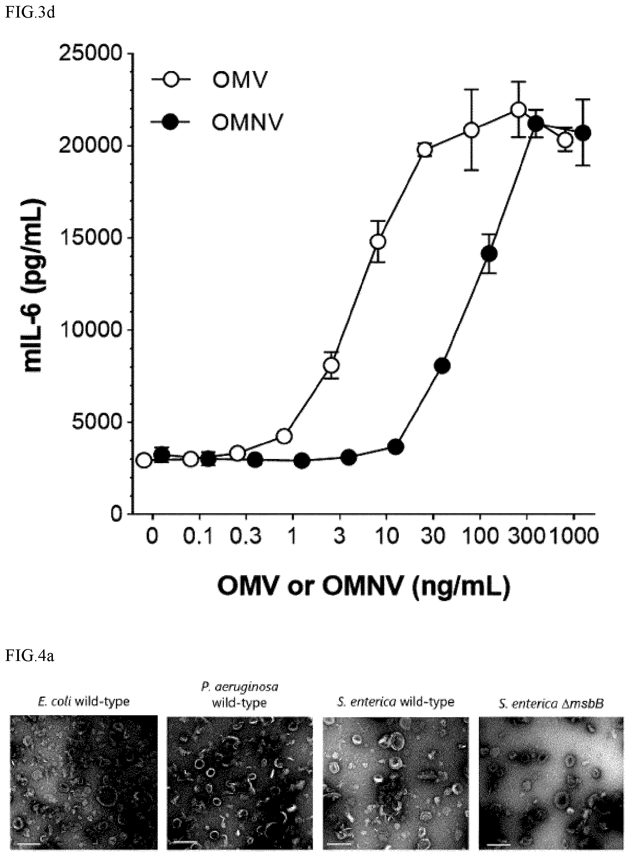

As shown in Figures 3a to d, OMVs and OMNVs could induce innate immunity in dose-dependent manners, and at the same concentration, OMVs induced innate immunity with a higher degree than OMNVs did. These results suggest that OMNVs induce innate immunity while having less adverse effects compared to OMVs.

### Example 5. Characterization of OMNVs Prepared from Various Types of Gram-negative Bacteria

### 5-1. Morphology and Size Determination of OMNVs prepared from various types of Gram-negative bacteria

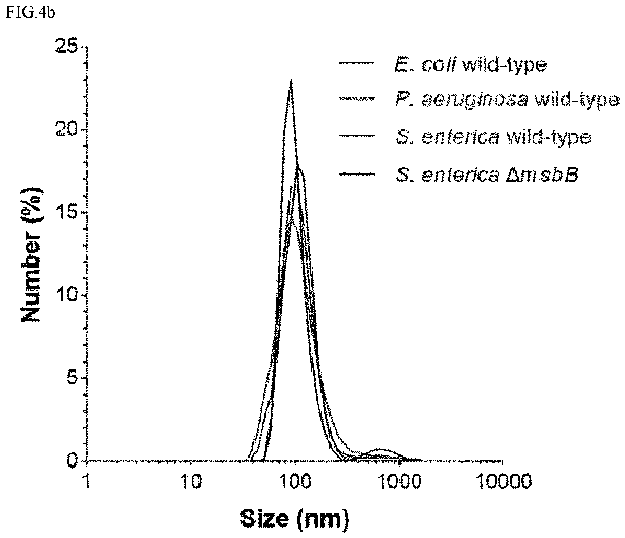

Referring to the method of Example 1, OMNVs were prepared by culturing *E. coli* BL21 (DE3) wild-type, *Pseudomonas aeruginosa* PAO1 wild-type, *Salmonella enterica* wild-type, and *S. enterica ΔmsbB.* To analyze the morphology and size of OMNVs prepared from various types of Gram-negative bacteria, transmission electron microscopy and dynamic light scattering analysis were performed referring to Example 3-1. As shown in Figure 4a, it was confirmed that all the OMNVs prepared from various types of Gram-negative bacteria were composed of lipid bilayers. Additionally, as shown in Figure 4b, the average diameters of OMNVs from *E. coli* BL21 (DE3) wild-type, *P. aeruginosa* PAO1 wild-type, *S. enterica* wild-type, and *S. enterica ΔmsbB* were 125.5 ± 40.2 nm, 124.0 ± 39.3 nm, 144.6 ± 48.9 nm, and 154.8 ± 32.1 nm, respectively.

### 5-2. Determination of Total Particle Number and Total Protein Amounts of OMNVs Prepared from Various Types of Gram-negative Bacteria

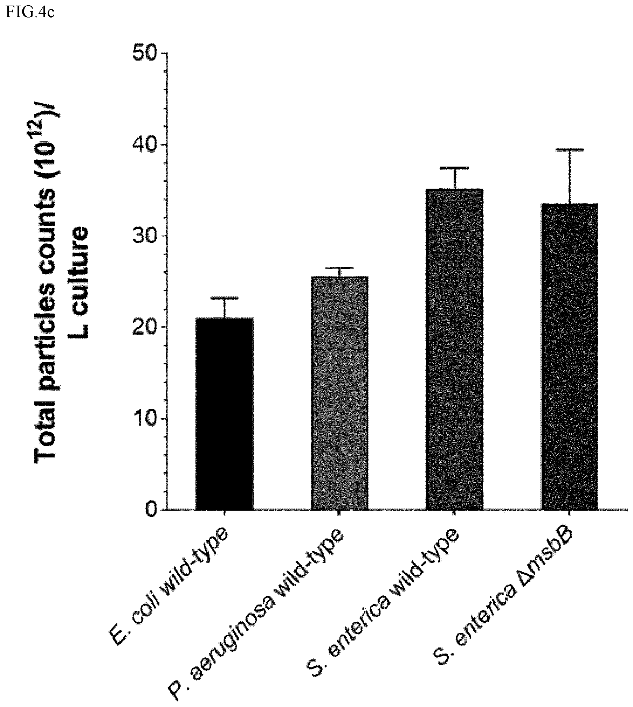

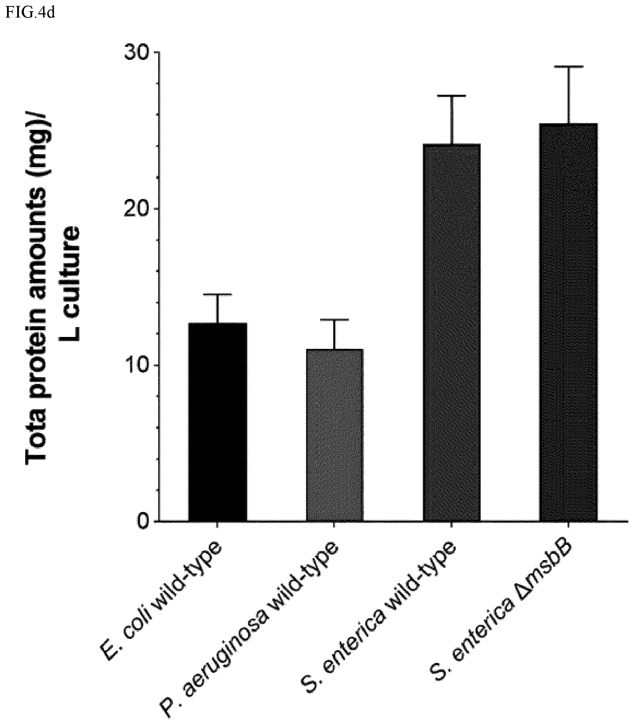

To determine total particle numbers and total protein amounts of OMNVs prepared from various types of Gram-negative bacteria, analysis and quantification were performed using a nanoparticle tracking analyzer and Bradford assay referring to Example 3-2. As shown in Figure 4c, it was confirmed that the average number of particles per liter of culture medium for OMNVs from *E. coli* BL21 (DE3) wild-type, *P. aeruginosa* PAO1 wild-type, *S. enterica* wild-type, and S. *enterica ΔmsbB* were (2.10 ± 0.22) × 10¹³ particles, (2.56 ± 0.09) × 10¹³ particles, (3.52 ± 0.23) × 10¹³ particles, and (3.36 ± 0.59) × 10¹³ particles, respectively. Additionally, as shown in Figure 4d, the average total protein amounts per liter of culture medium for OMNVs from *E. coli* BL21 (DE3) wild-type, *P. aeruginosa* PAO1 wild-type, *S. enterica* wild-type, and *S. enterica ΔmsbB* were 12.733 ± 1.803 mg, 11.043 ± 1.866 mg, 24.151 ± 3.077 mg, and 25.479 ± 3.619 mg, respectively.

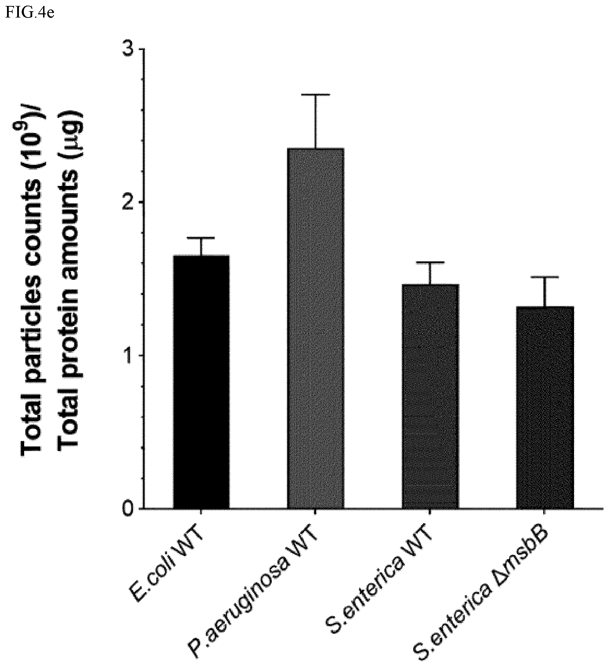

Furthermore, to determine the purity of OMNVs prepared from various types of Gram-negative bacteria, total particle numbers per total protein amounts was calculated. As shown in Figure 4e, the purities of OMNVs from *E. coli* BL21 (DE3) wild-type, *P. aeruginosa* PAO1 wild-type, *S. enterica* wild-type, and *S. enterica ΔmsbB* were (1.657 ± 0.112) × 10⁹ particles/µg, (2.353 ± 0.349) × 10⁹ particles/µg, (1.467 ± 0.140) × 10⁹ particles/µg, and (1.320 ± 0.193) × 10⁹ particles/µg, respectively.

### Example 6. Analysis of Innate Immunity Inducing Activity of OMNVs Prepared from Various Gram-Negative Bacteria

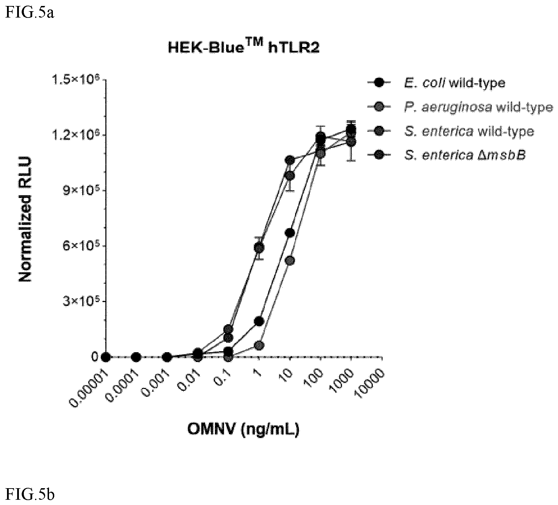

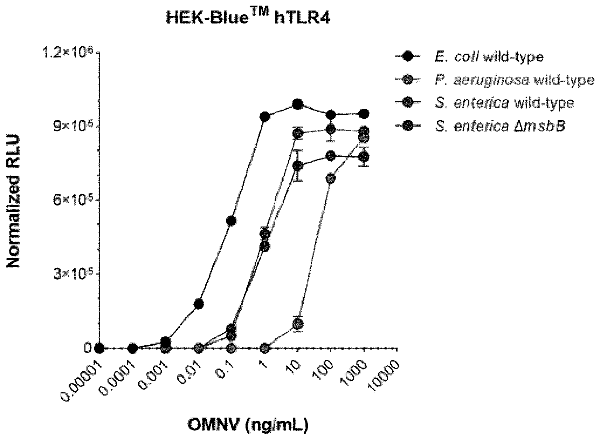

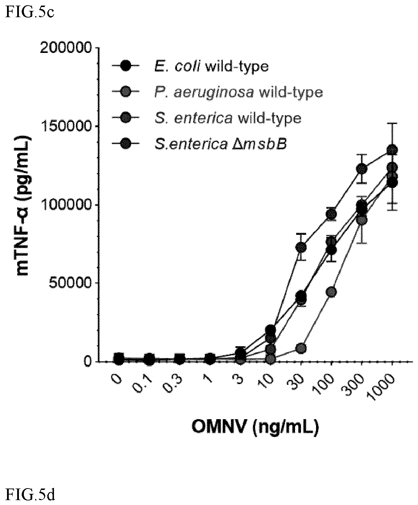

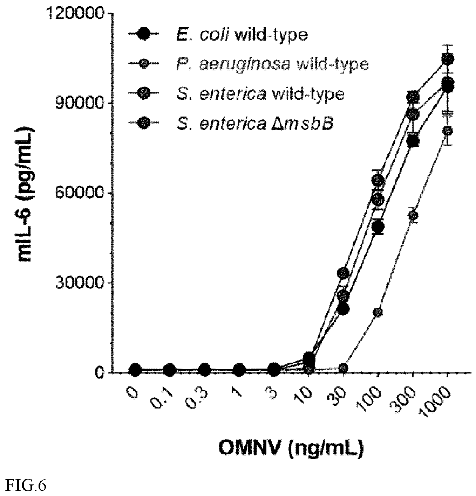

The innate immunity inducing activity of OMNVs prepared from various Gram-negative bacteria, prepared by the method of Example 5, was analyzed by the method of Example 4. As a result, as shown in Figures 5a to 5d, it was confirmed that OMNVs prepared from various Gram-negative bacteria can induce innate immunity in dose-dependent manners.

### Example 7. Preparation of OMNVs Containing Polyethylene Glycol

Polyethylene glycol was incorporated into the surface of OMNVs prepared by the method of Example 1 to prepare OMNVs containing polyethylene glycol on the surface.

The OMNVs prepared by the method of Example 1 were diluted to 1 mg/mL using HEPES-buffered saline (20 mM HEPES (pH 7.4), 150 mM NaCl), and then mixed so that the final concentrations of cholesterol-PEG and cholesterol-PEG-biotin were 20 µg/mL and 2 µg/mL, respectively. The mixture was then incubated at 4°C for 30 minutes, and PEGylated OMNVs were obtained through size-exclusion chromatography (^{PEGylation}OMNV).

To measure PEGylation of ^{PEGylation}OMNV, various concentrations (0, 100, 300, 1000 ng/mL) of OMNV or ^{PEGylation}OMNV were attached to a 96-well plate at room temperature for 2 hours. Then, blocking was performed with 1% BSA/PBS at room temperature for 1 hour, and streptavidin-HRP was treated at room temperature for 30 minutes. Finally, luminescence was measured using a multimode plate reader with BM chemiluminescence substrate to confirm PEGylation.

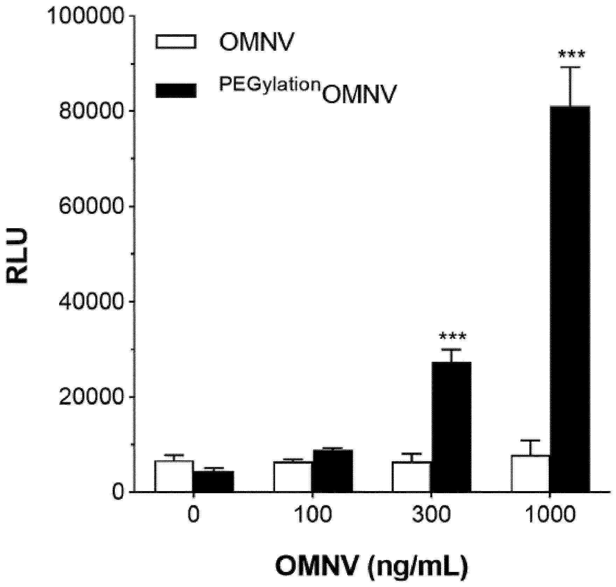

As a result, as shown in Figure 6, it was found that cholesterol-polyethylene glycol-biotin was bound to the OMNVs in the case of ^{PEGylationo}OMNV, showing high RLU. On the other hand, OMNV showed low RLU.

### Example 8. Drug Delivery Using OMNVs

### 8-1. Delivery of Anticancer Drugs Using OMNVs-1: OMNVs Loaded with Anticancer Drugs During Preparation

To determine whether daunorubicin (DNR), an anticancer drug, can be delivered using OMNVs prepared by the method of Example 1, daunorubicin-loaded nanovesicles were prepared according to the method of Example 1. In the step of preparing the outer membrane of Gram-negative bacteria by treating with Sarkosyl, 500 µg/mL of daunorubicin was added. Then, serial extrusion was performed to prepare daunorubicin-loaded OMNVs (OMNV_{DNR}).

To verify whether daunorubicin can be delivered to target cells using OMNV_{DNR}, Alexa 488 NHS Ester was incubated with OMNV_{DNR}, and only the fluorescently labeled OMNV_{DNR} was separated by size-exclusion chromatography (^{Alexa 488}OMNV_{DNR}). RAW264.7 cells were cultured on gelatin-treated glass, and the cells were treated with 1 µg/mL of ^{Alexa 488}OMNV_{DNR} for 24 hours, then the nuclei of the cells were stained with Hoechst and observed under a fluorescence microscope. Additionally, RAW264.7 cells were treated with various concentrations (0, 10, 100, 1000 ng/mL) of ^{Alexa 488}OMNV_{DNR}, and flow cytometry was performed after 24 hours.

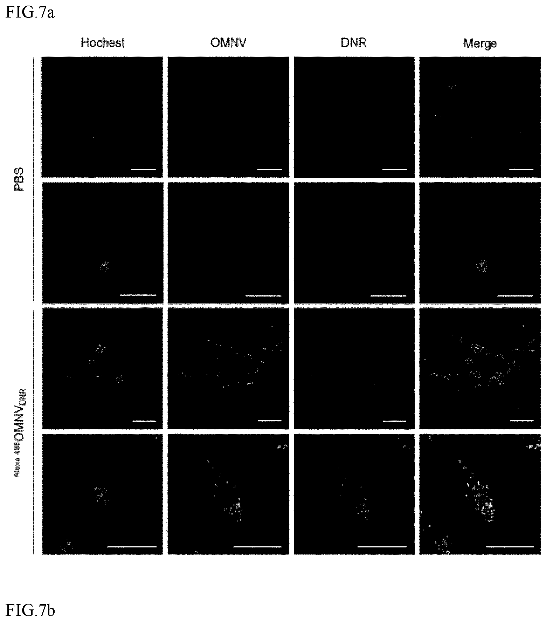

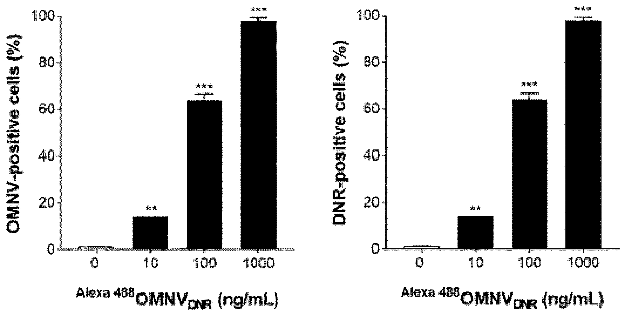

As a result, as shown in Figures 7a to 7b, OMNV_{DNR} delivered daunorubicin to target cells.

### 8-2. Delivery of Anticancer Drugs Using OMNVs-2: OMNVs Loaded with Anticancer Drugs by Various Methods After Preparation

To determine whether daunorubicin (DNR) can be delivered to target cells even when daunorubicin is loaded by various methods after preparing OMNVs by the method of Example 1, OMNVs were prepared by the method of Example 1, and then daunorubicin was loaded by incubation, sonication, and freezing-thawing methods.

First, to load daunorubicin into OMNVs by incubation, OMNVs (1 mg/mL) and daunorubicin (500 µg/mL) were mixed and incubated at 37°C for 6 hours. Additionally, to load daunorubicin into OMNVs by sonication, OMNVs (1 mg/mL) and daunorubicin (500 µg/mL) were mixed and sonicated 10 times at amplitude 50%, cycle 0.5, 4°C. Finally, to load daunorubicin into OMNVs by freezing-thawing, OMNVS (1 mg/mL) and daunorubicin (500 µg/mL) were mixed, frozen in liquid nitrogen for 3 minutes, and then thawed at 37°C for 3 minutes, repeating this 10 times. Size exclusion-chromatography was performed to isolate daunorubicin-loaded OMNVs from the mixtures that underwent incubation, sonication, and freezing-thawing processes. Finally, OMNVs were prepared, and daunorubicin-loaded OMNVs by incubation, sonication, and freezing-thawing methods were named OMNV_{DNR-Inc}, ONMV_{DNR-Son}, and OMNV_{DNR-FT}, respectively.

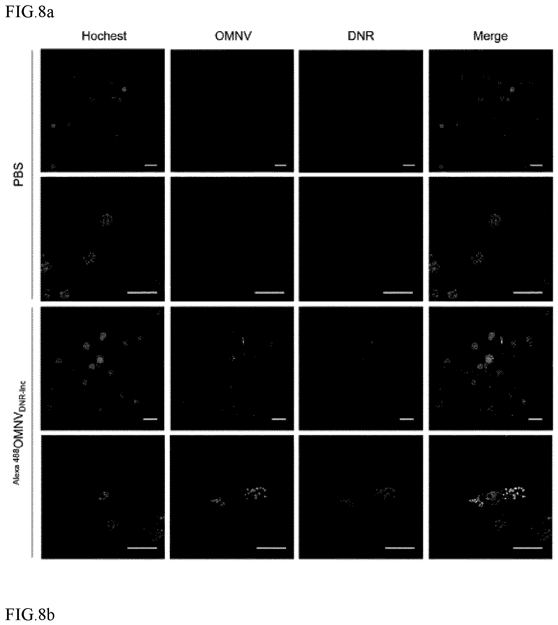

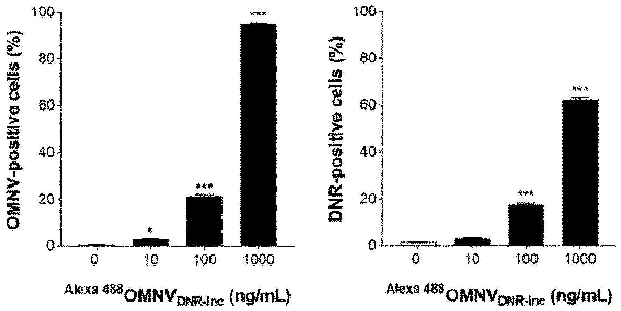

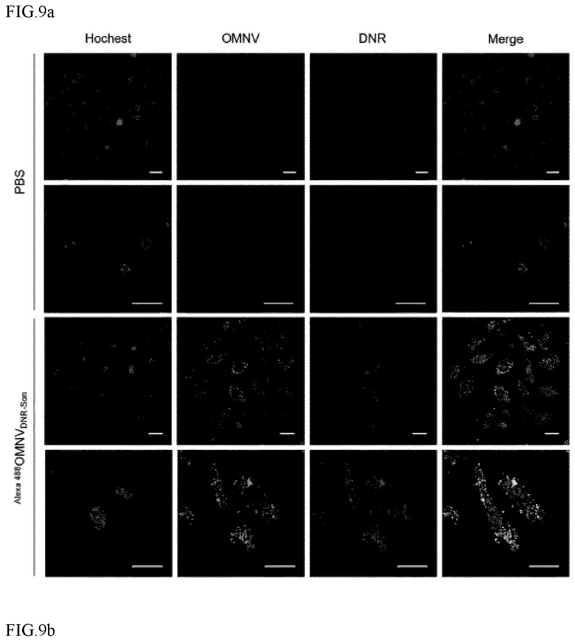

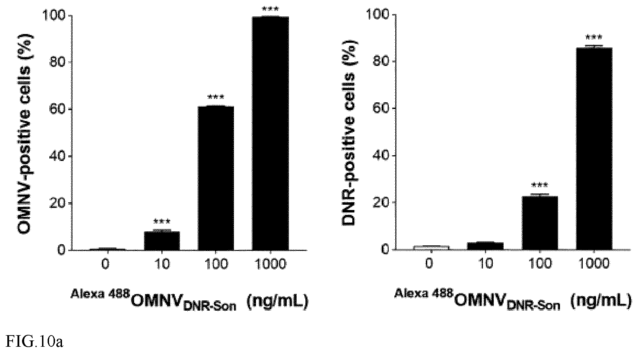

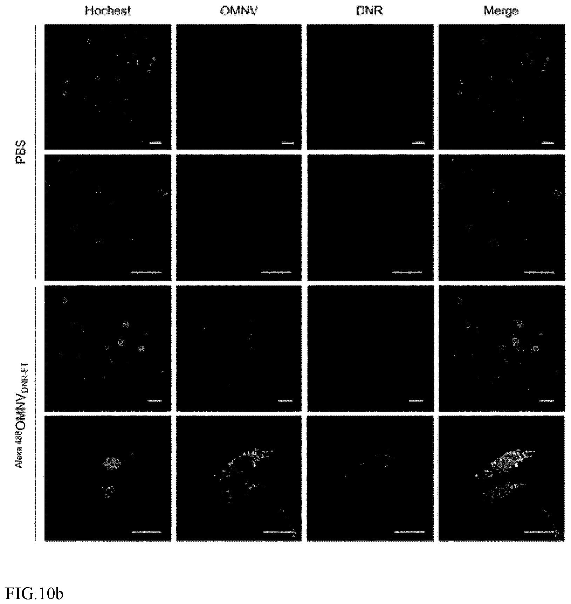

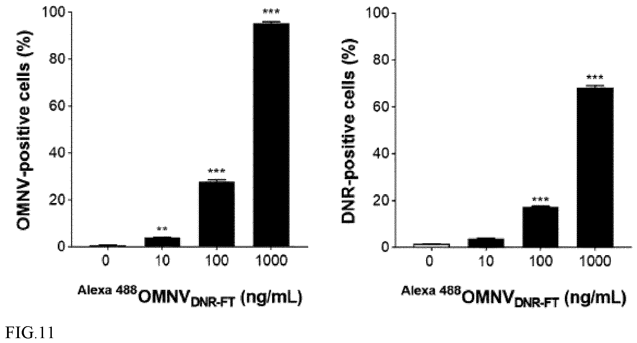

To verify whether daunorubicin can be delivered to target cells using OMNV_{DNR-Inc}, OMNV_{DNR-Son}, and OMNV_{DNR-FT}, OMNV_{DNR-Inc}, OMNV_{DNR-Son}, and OMNV_{DNR-FT} were incubated with Alexa 488 NHS ester and the fluorescently labeled ones were isolated by the method of Example 8-1 (^{Alexa488}OMNV_{DNR-Inc}, ^{Alexa 488}OMNV_{DNR-Son}, ^{Alexa 488}OMNV_{DNR-FT}). These OMNVs were treated to RAW264.7 by the method of Example 8-1, then flow cytometry and fluorescence microscopy were performed. As a result, as shown in Figures 8a to 8b, 9a to 9b, and 10a to 10b, OMNV_{DNR-Inc}, OMNV_{DNR-Son}, and OMNV_{DNR-FT} delivered daunorubicin to target cells.

### 8-3. Delivery of Anti-inflammatory Drugs Using OMNVs

To determine whether dexamethasone (DEX), an anti-inflammatory drug, can be delivered using OMNVs prepared by the method of Example 1, dexamethasone-loaded OMNVs were prepared according to the method of Example 1. In the step of preparing the outer membrane of Gram-negative bacteria by treating with Sarkosyl, 500 µg/mL of dexamethasone was added, and extrusion was performed to prepare dexamethasone-loaded OMNVs (ONMV_{DEX}).

To verify whether anti-inflammatory responses can be induced using OMNV_{DEX}, RAW264.7 (5×10⁴ cells/well) was attached to a well plate for 24 hours. Then, the cells were treated with various concentrations (10, 30, 100, 300, 1000 ng/mL) of OMNV_{DEX} and OMNV prepared in Example 1 for 18 hours, and the conditioned medium was obtained. mIL-6 was quantified by ELISA from the obtained conditioned medium.

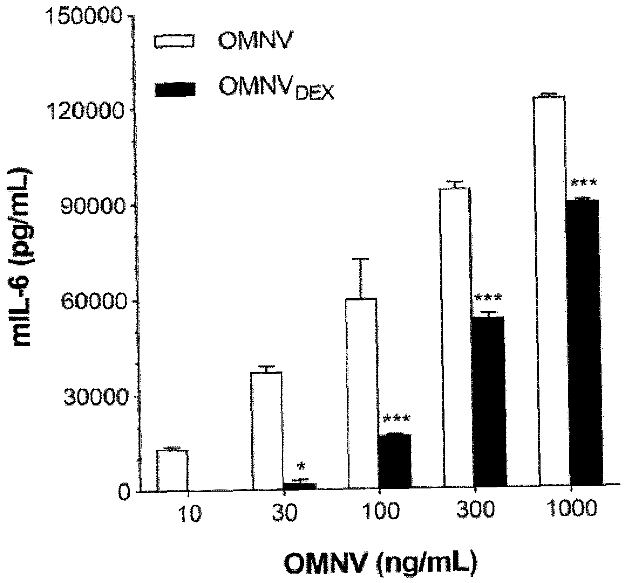

As a result, as shown in Figure 11, OMNV_{DEX} can inhibit IL-6 secretion from macrophages in a dose-dependent manner.

### Example 9. Delivery of Proteins Using OMNVs

To determine whether proteins can be delivered using OMNVs prepared by the method of Example 1, GFP-loaded OMNVs were prepared according to the method of Example 1. In the step of preparing the outer membrane of Gram-negative bacteria by treating with Sarkosyl, 500 µg/mL of GFP was added, and serial extrusion was performed to prepare GFP-loaded OMNVs (OMNV_{GFP}).

To verify whether GFP can be delivered to target cells using OMNV_{GFP}, Alexa 594 NHS Ester was incubated with OMNV_{GFP}, and the fluorescently labeled OMNV_{GFP} was isolated by size-exclusion chromatography (^{Alexa 594}OMNV_{GFP}). RAW264.7 was cultured on gelatin-treated glass, and the cells were treated with ^{Alexa 594}OMNV_{GFP} ( 1 ng/mL) for 24 hours, then the nuclei of the cells were stained with Hoechst and observed under a fluorescence microscope. Additionally, RAW264.7 cells were treated with various concentrations (0, 10, 100, 1000 ng/mL) of Alexa ⁵⁹⁴OMNV_{GFP} and flow cytometry was performed after 24 hours.

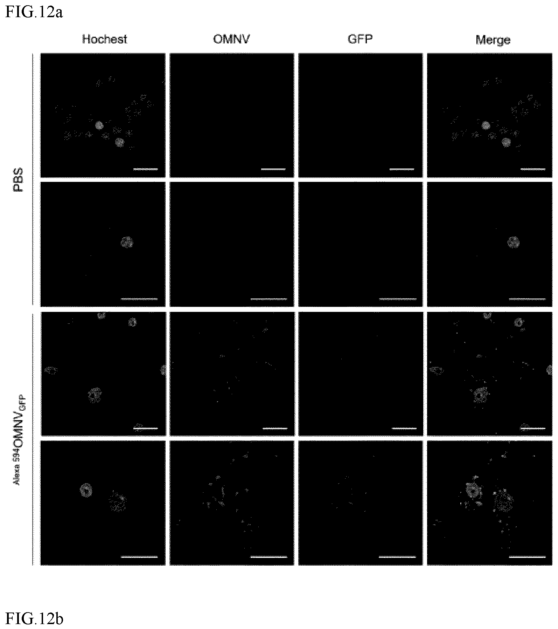

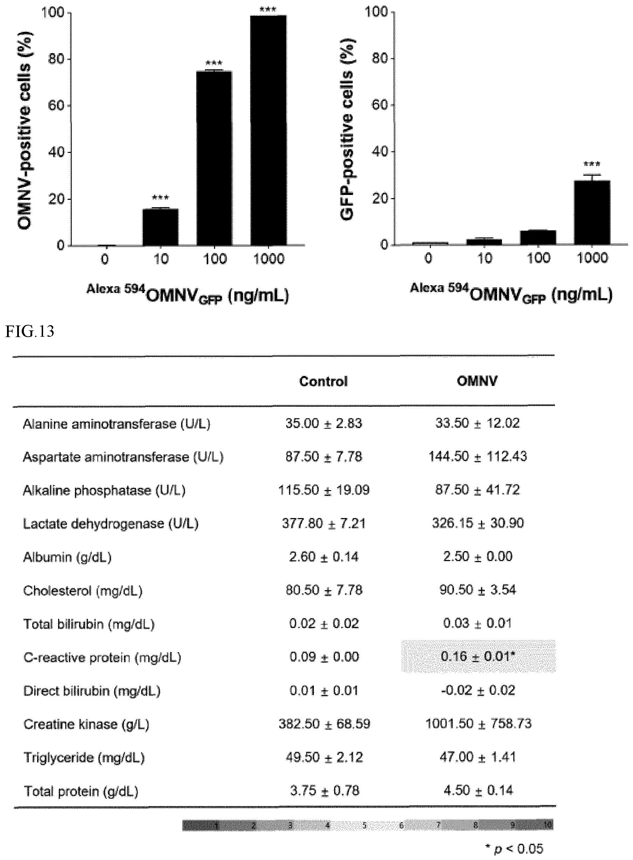

As a result, as shown in Figures 12a to 12b, OMNV_{GFP} delivered GFP to target cells.

In summary, various drugs and proteins can be delivered to target cells using OMNVs.

### Example 10. Analysis of Adverse Effects After Administrating OMNVs

To verify the adverse effects after administrating OMNVs prepared by the method of Example 1, OMNVs (50 µg/head) were intraperitoneally administered into C57BL/6 (male, 6 weeks old) mice, and the serum was prepared from the blood of the mice after 24 hours. Then, the serum was subjected to blood biochemical analysis using an automatic chemistry analyzer (BS-390, Mindray, China).

As a result, as shown in Figure 13, the mice administered with OMNVs showed no significant differences from the control group in most items, and only the concentration of C-reactive protein was slightly increased significantly.

In summary, the OMNVs have almost no adverse effects, making them very useful for developing therapeutic and diagnostic methods for cancer, methods for delivering substances for disease treatment and diagnosis, and vaccine delivery methods for disease prevention and treatment, including cancer and infectious diseases.

### Example 11. Analysis of In Vivo Distribution After Administrating OMNVs

To analyze *in vivo* distribution after administrating OMNVs prepared by the method of Example 1, Cy7-labeled OMNVs (^{Cy7}OMNV) were prepared, and free Cy7 was removed by size-exclusion chromatography. Then, ^{Cy7}OMNV was intraperitoneally administered into mice, and whole-body distribution was measured over time using an *in vivo* imaging system (IVIS). Additionally, at 24 hours after administration, the major organs (the thymus, spleen, heart, liver, lungs, kidneys, skin at the injection site, small intestine) were harvested, and the distribution within the organs was analyzed using the IVIS equipment. ^{Cy7}Dye Control was used as a control group.

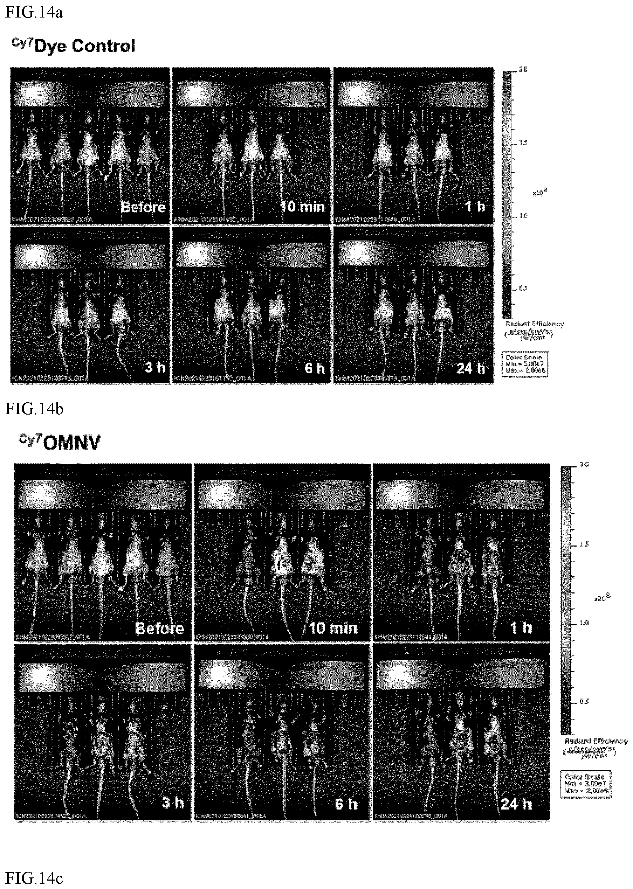

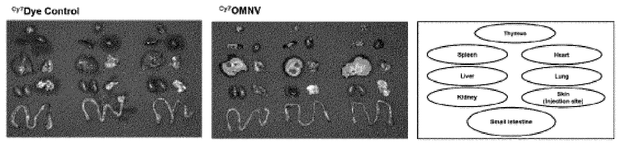

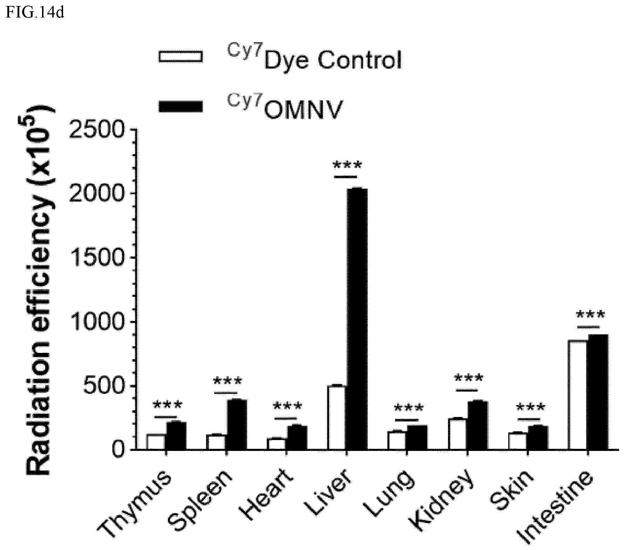

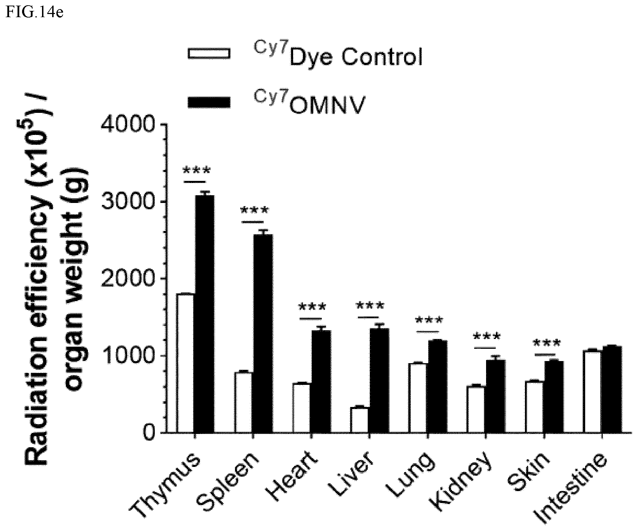

As a result, as shown in Figure 14a, ^{Cy7}Dye Control could not be detected in the whole-body distribution, whereas, as shown in Figure 14b, ^{Cy7}OMNV was determined to spread around the peritoneal cavity over time. Additionally, as shown in Figures 14c and 14d, ^{Cy7}Dye Control could not be detected in the organ distribution, whereas ^{Cy7}OMNV showed strong signals in the liver and small intestine, and when the signal was normalized for organ weight, strong signals were detected in the thymus, spleen, heart, and liver.

### Example 12. Analysis of Anti-tumor Activity of OMNVs

To determine anti-tumor activity of OMNVs prepared by the method of Example 1, MB49 (mouse bladder cancer cells, 1×10⁶ cells/head) were subcutaneously introduced into C57BL/6 (male, 7 weeks old) mice at, and the size of the tumor tissue was measured over time using a vernier caliper (size = 0.5 × width × length × length). When the size of the tumor tissue reached approximately 100 mm³, the OMNVs were intratumorally administered three times at 3-day intervals (0, 1, or 10 µg/head per administration), and the size of the tumor tissue was measured over time.

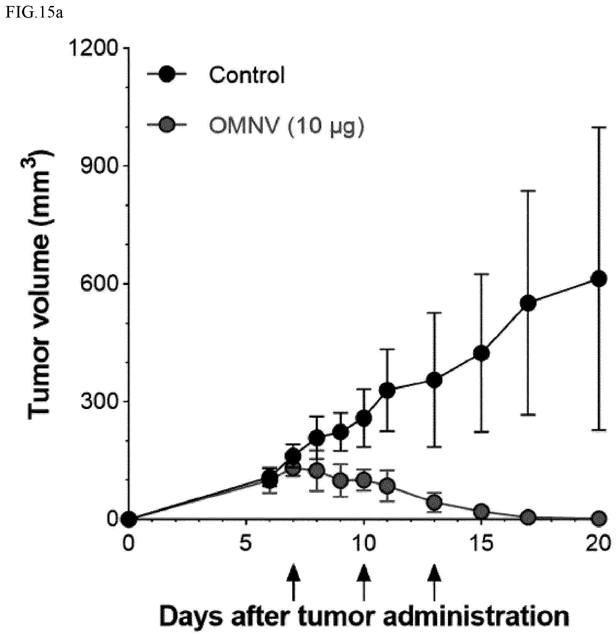

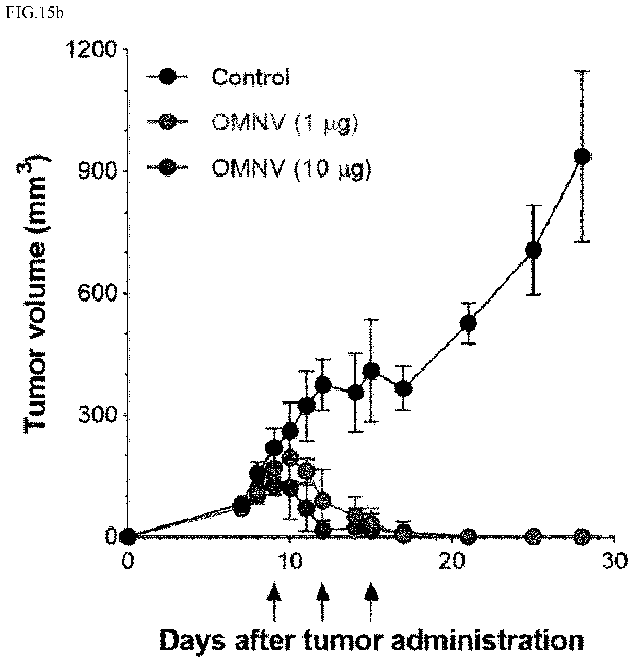

As a result, as shown in Figures 15a and 15b, the OMNVs showed anti-tumor activity.

### Example 13. Analysis of Interaction Between OMNVs and Dendritic Cells

### 13-1. Analysis of the Effect of OMNVs on the Survival Rate and Cytokine Release of Dendritic Cells

To determine the effect of OMNVs prepared by the method of Example 1 on the survival rate and cytokine release of dendritic cells, bone marrow-derived dendritic cells (BMDCs) were isolated, and the OMNVs were treated to confirm the survival rate and cytokine release of dendritic cells.

The femur and tibia of C57BL/6 (male, 4 weeks old) mice were harvested, and DMEM (2.5% FBS) was administered into the bone marrow to isolate bone marrow cells, which were cultured using RPMI1640 (10% FBS, 50 µM 2-mercaptoethanol, 20 ng/mL rmGM-CSF) to isolate bone marrow-derived dendritic cells. The isolated bone marrow-derived dendritic cells (1×10⁵ cells/well) were attached to a well plate for 24 hours, and the cells were treated with various concentrations (0, 1, 3, 10, 30, 100, 300, 1000 ng/mL) of OMNVs for 24 hours. The cell survival rate was confirmed through an MTT assay, and mTNF-α, mIL-6, and mIL-12p40 were quantified from the conditioned medium using ELISA.

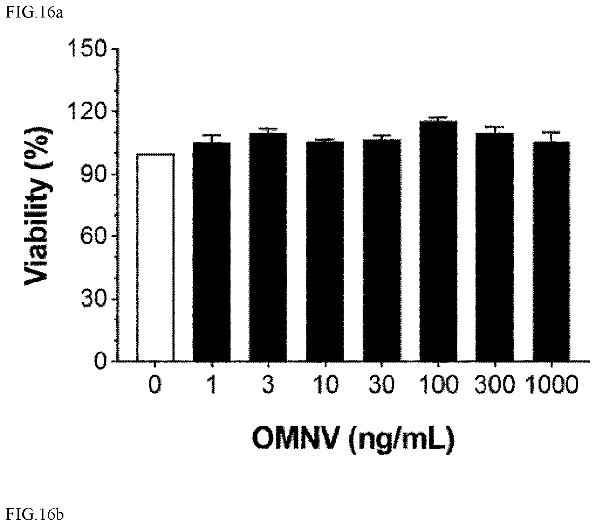

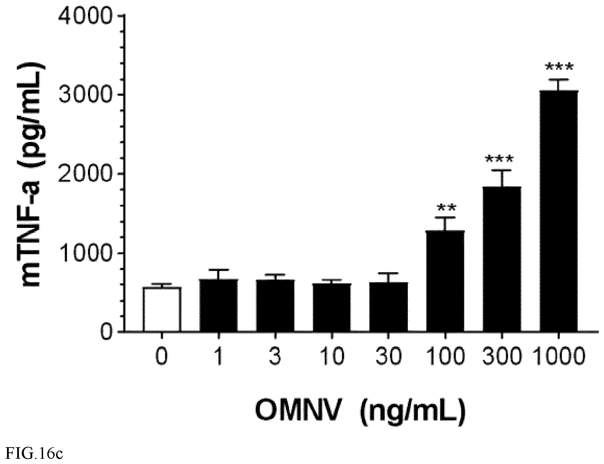

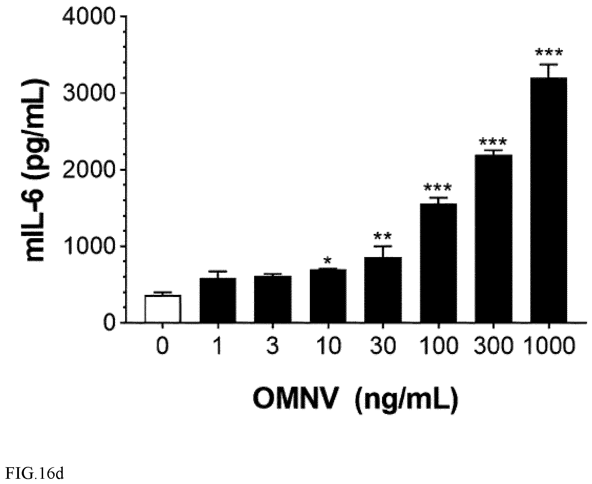

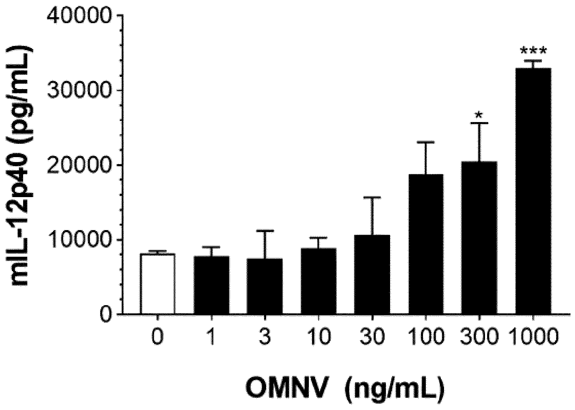

As a result, as shown in Figure 16a, the OMNVs did not affect the survival rate of dendritic cells, and as shown in Figures 16b, 16c, and 16d, they were determined to induce the release of mTNF-α, mIL-6, and mIL-12p40 in a dose-dependent manner.

### 13-2. Analysis of Uptake of OMNVs by Dendritic Cells

To verify whether dendritic cells efficiently uptake the OMNVs prepared by the method of Example 1, Alexa 594-labeled OMNVs (^{Alexa594}OMNV) were prepared, and free Alexa 594 was removed by size-exclusion chromatography. Then, Alexa ⁵⁹⁴OMNV was treated to dendritic cells, and the uptake by dendritic cells was determined through fluorescence microscopy and flow cytometry. Alexa ⁵⁹⁴Dye Control was used as a control group.

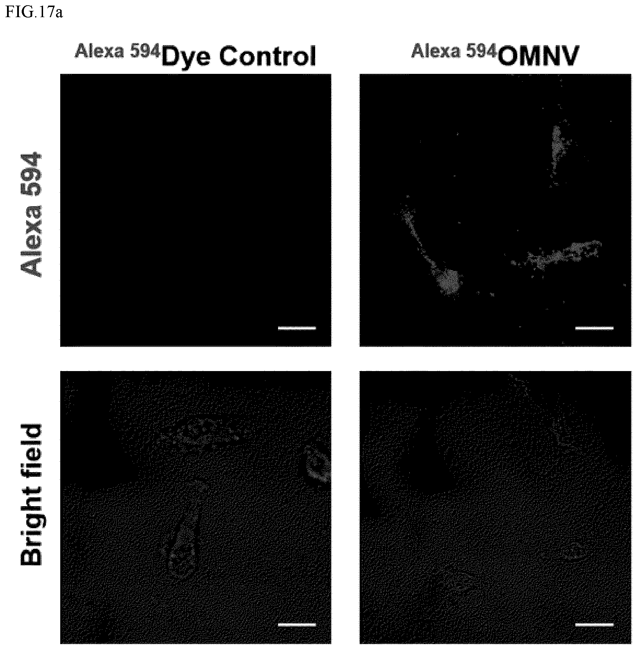

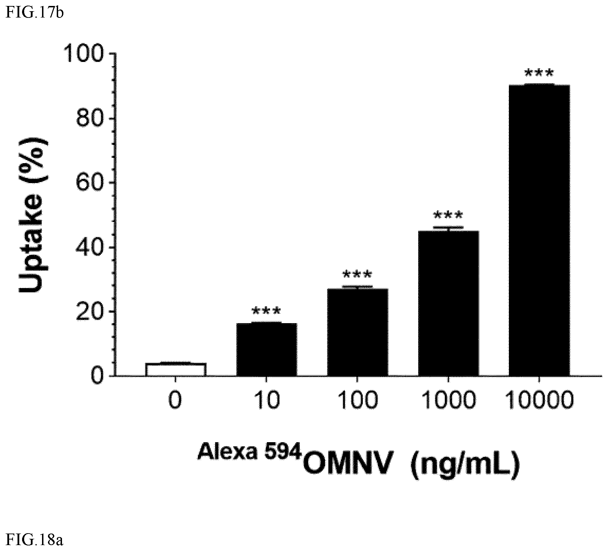

As a result, as shown in Figures 17a and 17b, dendritic cells were determined to uptake Alexa ⁵⁹⁴OMNV in a dose-dependent manner, whereas Alexa ⁵⁹⁴Dye Control was not uptaken.

### Example 14. Vaccination Using OMNVs and Antigens

### 14-1. Analysis of Short-term Immunity-inducing Activity of OMNVs

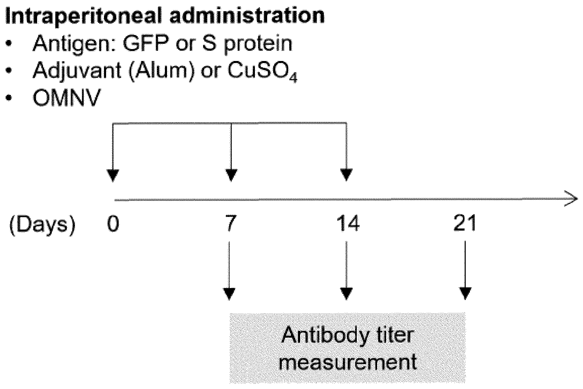

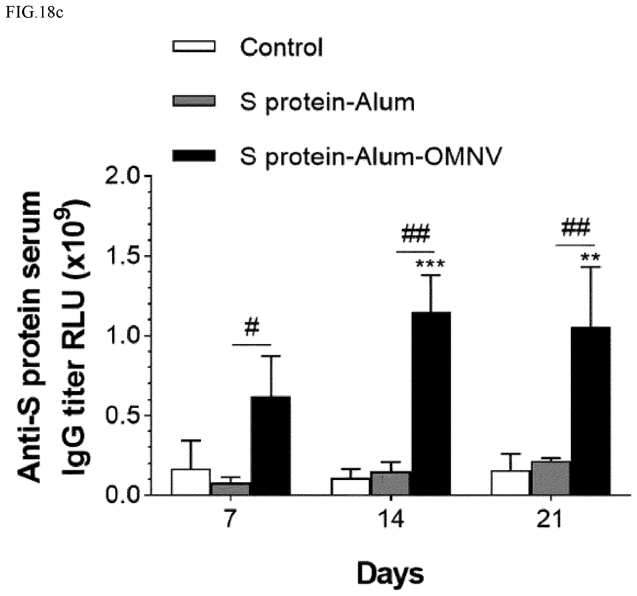

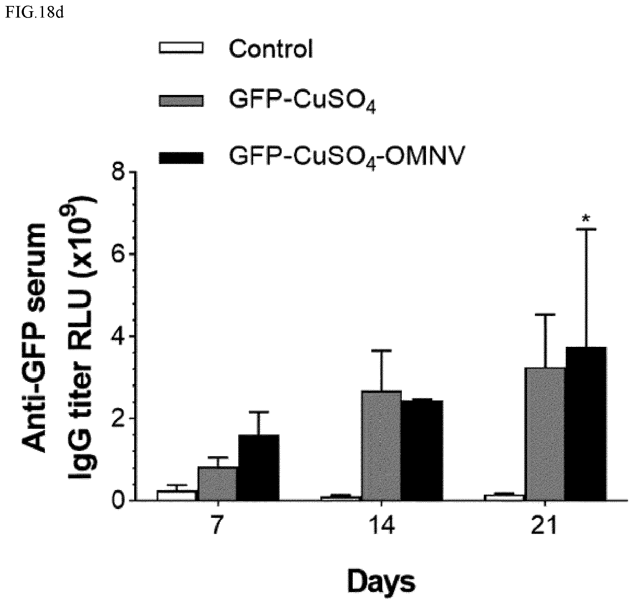

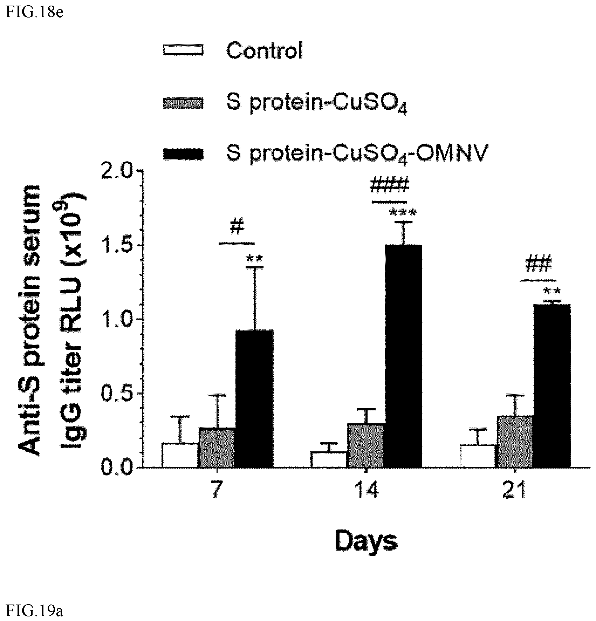

To determine the short-term immunity-inducing activity of OMNVs prepared by the method of Example 1, antigens (GFP or SARS-CoV-2 S protein) and adjuvants (Alum or CuSO₄) were mixed with OMNVs and intraperitoneally administered into C57BL/6 (male, 4 weeks old) mice three times at a week intervals. The blood was collected on days 7, 14, and 21 after the first administration (day 0), and the serum was separated. The experimental process is shown in Figure 18a.

Antigens (GFP or SARS-CoV-2 S protein) were incubated overnight at room temperature in a 96 black well plate (100 ng/well) and washed three times with PBS-T (0.05% Tween 20). Then, blocking was performed with 1% BSA/PBS at room temperature for 1 hour, followed by three times of washing with PBS-T (0.05% Tween 20). Mouse serum was diluted in 1% BSA/PBS and treated to the well plate at room temperature for 2 hours, followed by three times of washing with PBS-T (0.05% Tween 20). Then, a secondary antibody (anti-mouse IgG-HRP) conjugated with peroxidase was diluted in 1% BSA/PBS and treated to the well plate at room temperature for 1 hour, followed by three times of washing with PBS-T (0.05% Tween 20). Finally, luminescence was measured using a multimode plate reader with BM chemiluminescence substrate to confirm antigen-specific IgG titer.

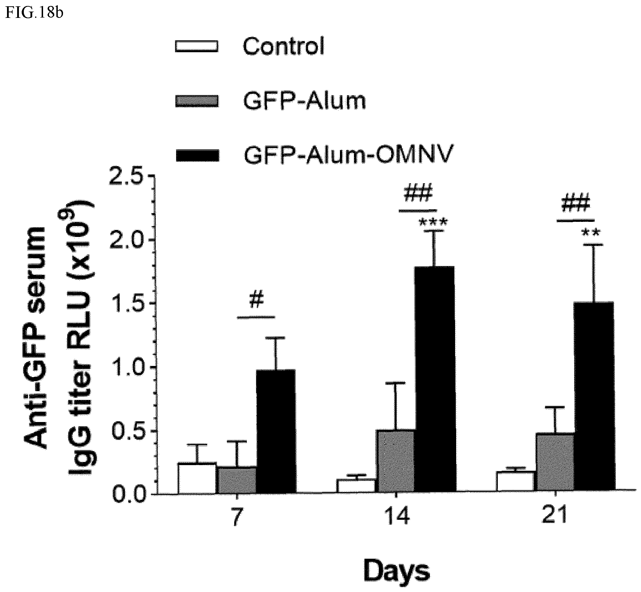

As a result, as shown in Figures 18b to 18e, the group admnistered with antigens, adjuvants (Alum or CuSO₄), and OMNVs showed higher antigen-specific IgG titer compared to the control group and the group administered with only antigens and adjuvants (Alum or CuSO₄). This result verifies that co-administration of OMNVs with antigens and adjuvants (Alum or CuSO₄) can more effectively induce short-term immunity.

### 14-2. Analysis of Long-term Immunity-inducing Activity of OMNVs

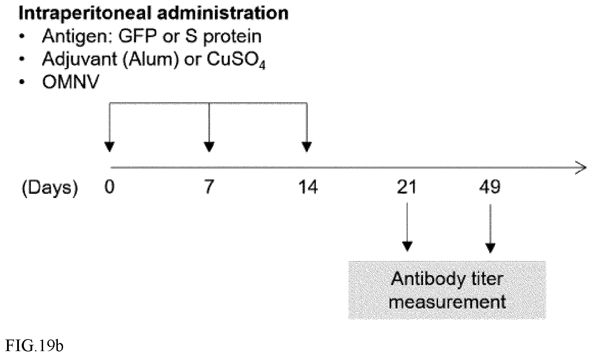

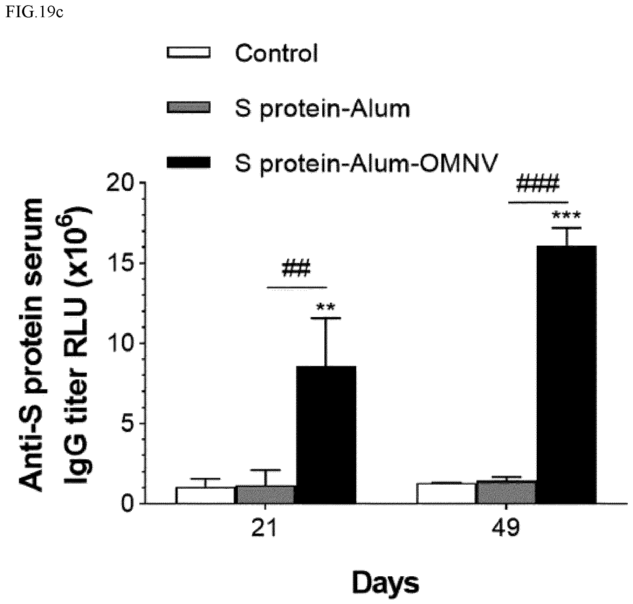

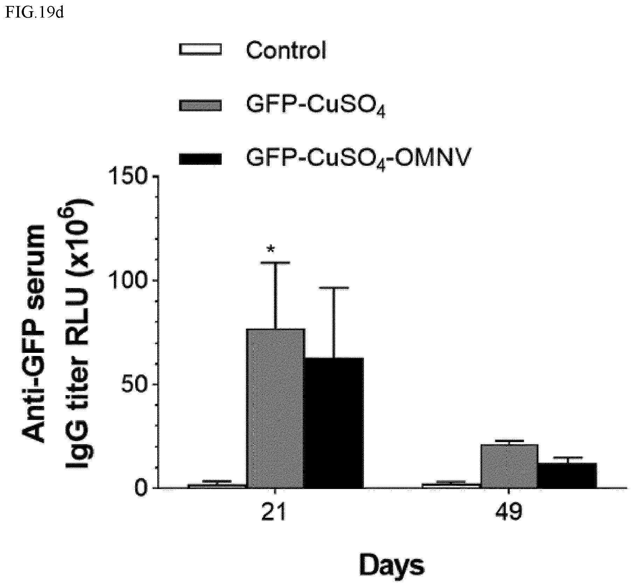

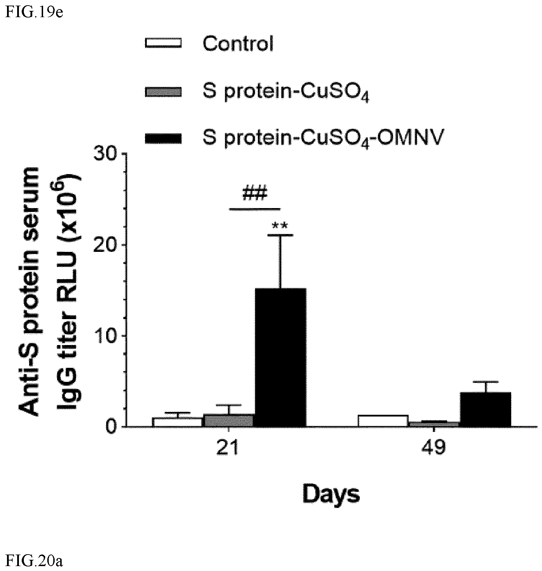

To confirm the long-term immunity-inducing activity of OMNVs, antigens (GFP or SARS-CoV-2 S protein) and adjuvants (Alum or CuSO₄) were mixed with OMNVs and intraperitoneally administered into C57BL/6 (male, 4 weeks old) mice three times at a week intervals. The blood was collected on days 21 and 49 after the first administration (day 0), and the serum was separated. The experimental process is shown in Figure 19a.

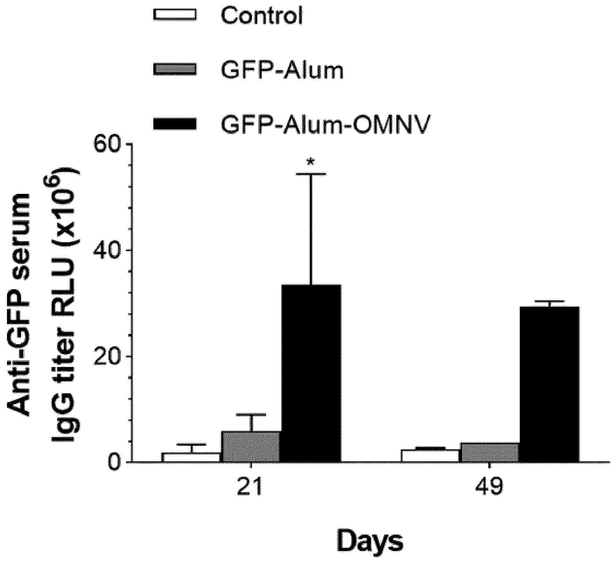

The separated serum was measured for antigen-specific IgG titer as shown in Example 14-1. As a result, as shown in Figures 19b to 19e, the case where the antigen, adjuvant (Alum), or CuSO₄ and OMNVs were administered together showed a higher antigen-specific IgG titer than the control group as well as the case where only the antigen and adjuvant (Alum) or CuSO₄ were administered. This result verifies that co-administration of OMNVs with antigen and adjuvant (Alum) or CuSO₄ can more effectively induce long-term antigen-specific IgG titer.

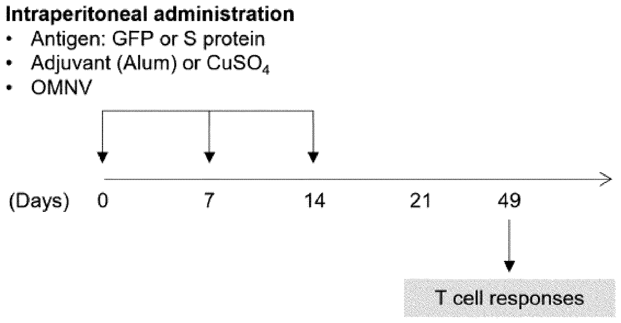

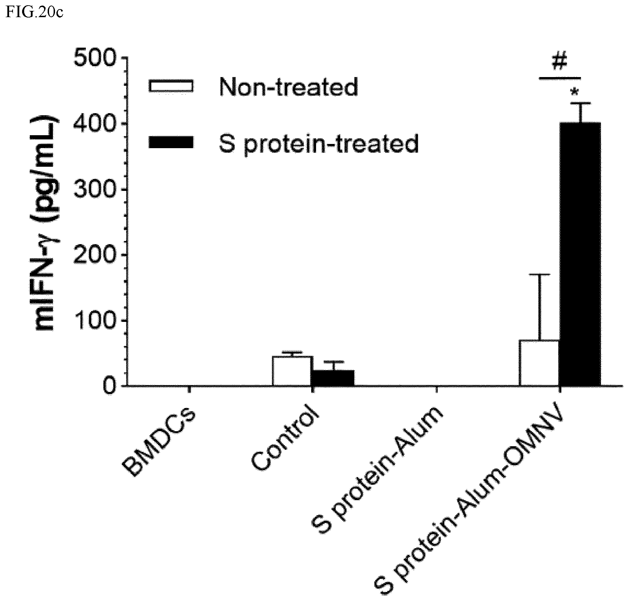

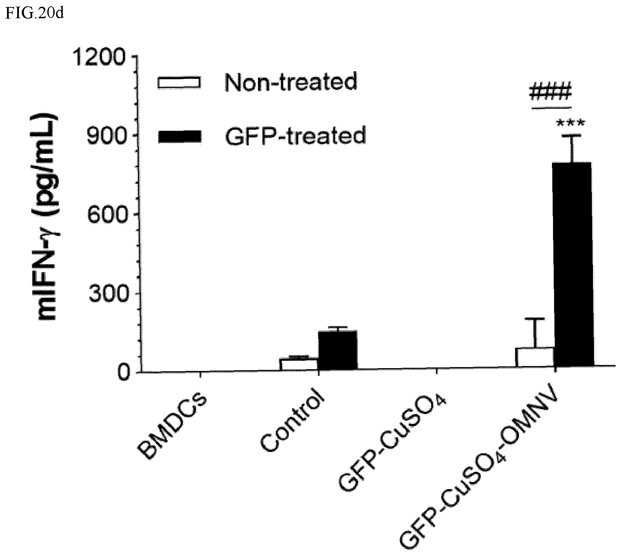

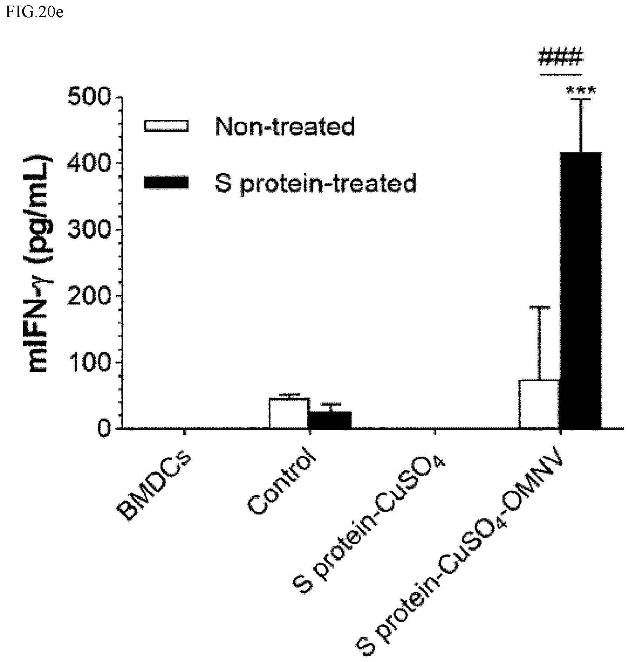

In addition, to confirm the effect of OMNVs on long-term T cell activation, antigen (GFP or SARS-CoV-2 S protein) and adjuvant (Alum) or CuSO₄ and OMNVs were mixed and intraperitoneally administered into C57BL/6 (male, 4 weeks old) three times at a week intervals. When the first administration was day 0, the spleen was harvested from the mouse on day 49. T cells were separated from the isolated spleen, and the conditioned medium was obtained by treating the antigen to the cells for 72 hours. mIFN-γ was quantified from the obtained conditioned medium by ELISA. The experimental process is shown in Figure 20a.

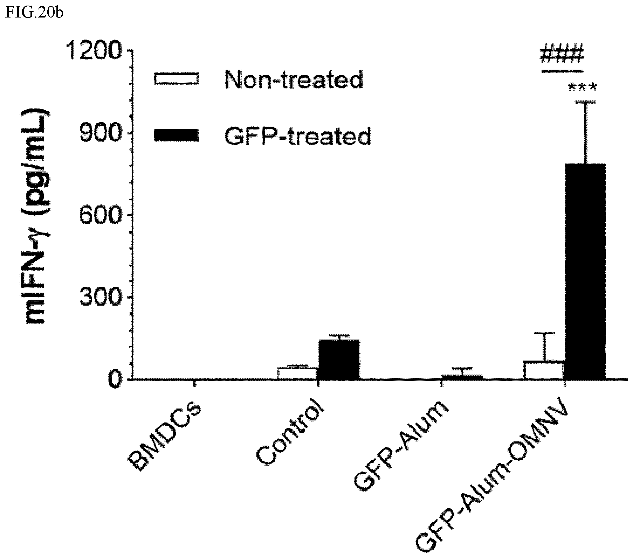

As a result, as shown in Figures 20b to 20e, the case where the antigen, adjuvant (Alum), or CuSO₄ and OMNVs were administered together showed higher antigen-specific mIFN-γ secretion than the control group as well as the case where only the antigen and adjuvant (Alum) or CuSO₄ were administered. This result confirms that co-administration of OMNVs with antigen and adjuvant can more effectively induce long-term antigen-specific Th1 response.

In summary, co-administration of OMNVs with antigen and adjuvant (Alum) or CuSO₄ can more effectively induce long-term immunity.

### 14-3. Analysis of Immunity-inducing Activity According to the Administration Route of OMNVs

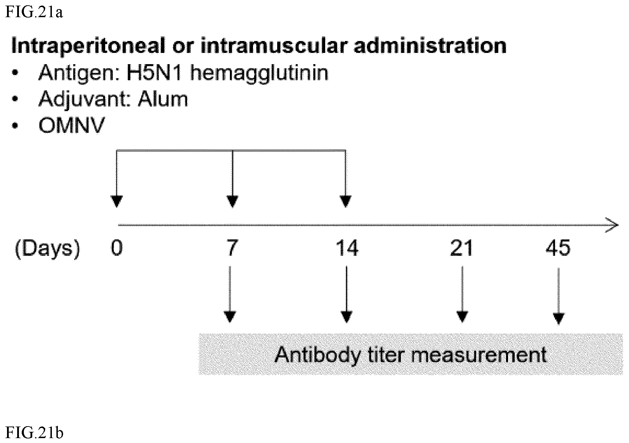

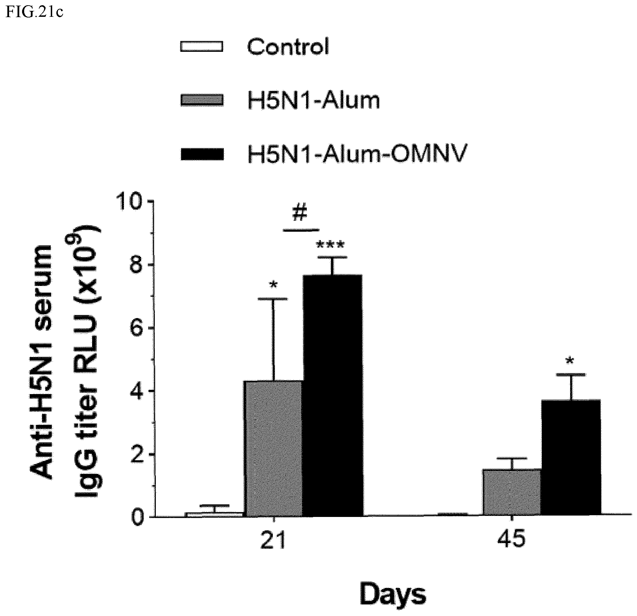

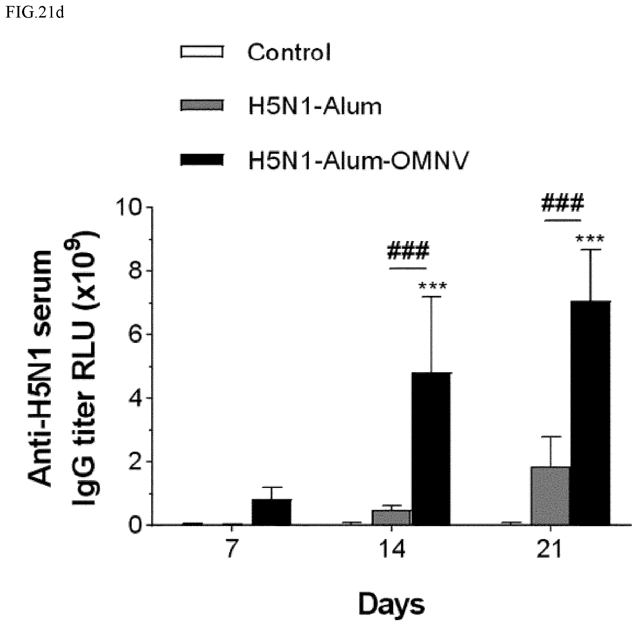

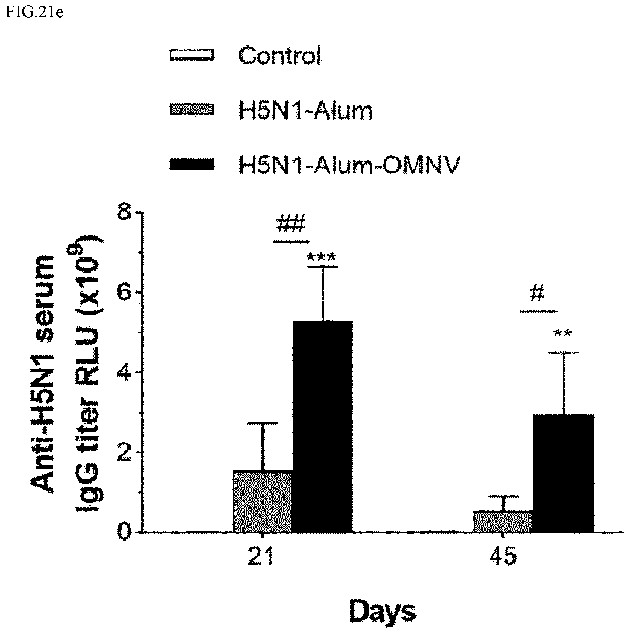

To determine the immunity-inducing activity according to the administration route of OMNVs, antigen (H5N1 hemagglutinin) and adjuvant (Alum) and OMNVs were mixed and intraperitoneally or intramuscularly administered into C57BL/6 (male, 4 weeks old) three times at a week intervals. When the first administration was day 0, the blood was collected on days 21 and 45, and the serum was separated. The experimental process is shown in Figure 21a.

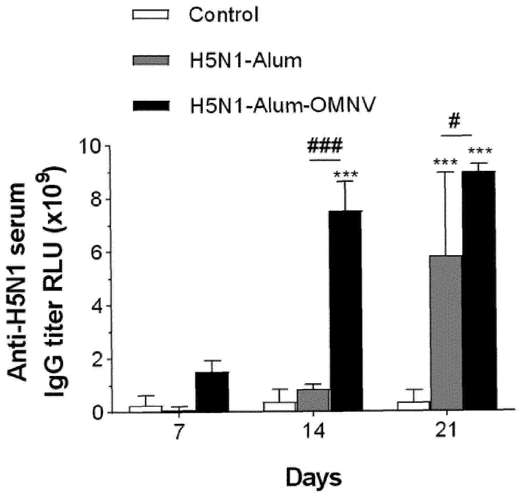

The separated serum was measured for antigen-specific IgG titer as shown in Example 14-1. As a result, as shown in Figures 21b to 21e, both administration routes showed higher antigen-specific IgG titer when the antigen, adjuvant, and OMNVs were administered together compared to the control group as well as the case where only the antigen and adjuvant were administered. This result confirms that co-administration of OMNVs with antigen and adjuvant intraperitoneally or intramuscularly can effectively induce long-term antigen-specific IgG titer.

In summary, OMNVs have almost no adverse effects, making them very useful for developing treatments and vaccines for various diseases, including cancer and infectious diseases, as methods for cancer treatment and diagnosis, delivery methods for therapeutic and diagnostic substances, and vaccine delivery methods for disease prevention and treatment.

In addition, OMNVs can effectively induce the production of neutralizing antibodies both short-term and long-term, making them useful as vaccines to prevent viral infections, cancer development such as cervical cancer caused by viral infections, or bacterial infections.

### Example 15. Preparation of OMNVs Expressing Antigens Based on the Outer Membrane Protein Display System

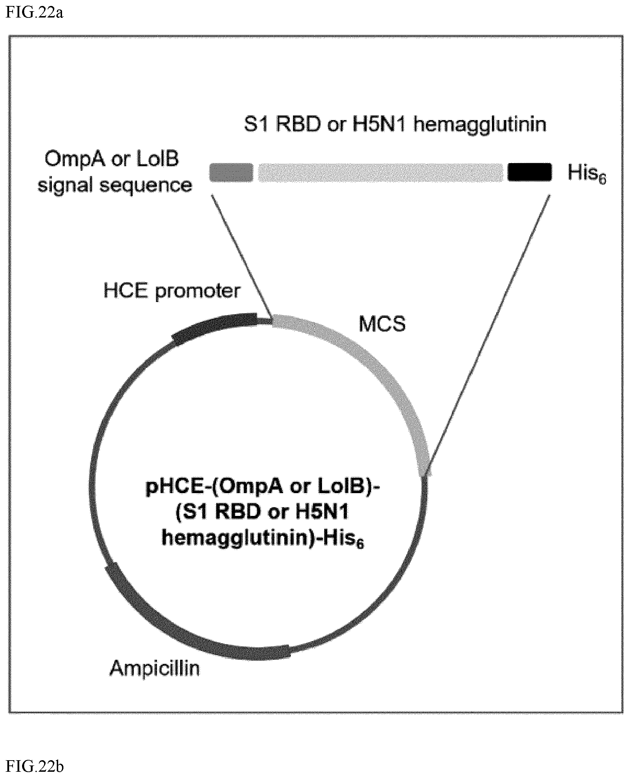

To prepare OMNVs expressing antigens based on the outer membrane protein display system, as shown in Figure 22a, signal sequences of outer membrane proteins (OmpA or LolB), antigens (S1-RBD (SARS-CoV-2 Spike protein 1 receptor-binding domain) or H5N1 hemagglutinin), and His₆-tag were inserted into the multiple cloning site of the pHCE vector, and *E. coli* BL21 (DE3) *ΔmsbB* was transformed to overexpress the antigen on the outer membrane of Gram-negative bacteria. Using the transformed Gram-negative bacteria, OMNVs expressing the antigen were prepared by the method of Example 1 (^{S1 RBD-OmpA}OMNV, ^{S1 RBD-LolB}OMNV, ^{H5N1-LolB}OMNV).

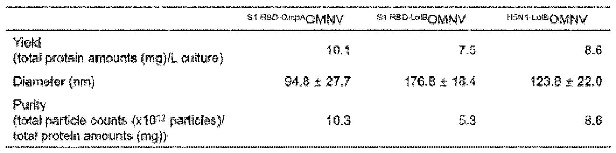

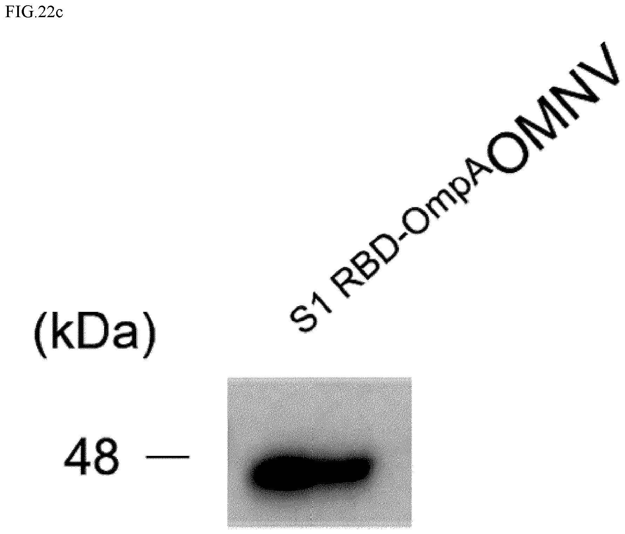

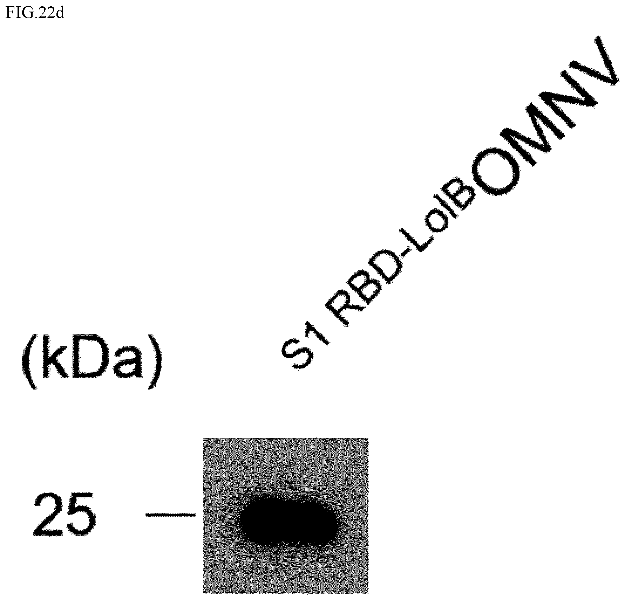

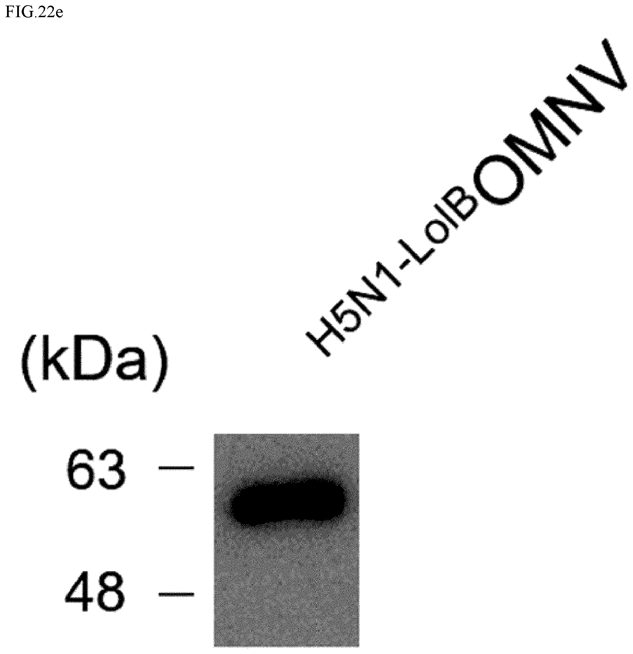

To analyze the characteristics of OMNVs expressing the antigen, the size, yield, and purity of OMNVs expressing the antigen were measured by the methods of Examples 3-1 and 3-2. The results are shown in Figure 22b. Meanwhile, to confirm the presence of the antigen in the OMNVs, Western blot was performed using a His₆-tag specific primary antibody by the method of Example 3-3. As shown in Figures 22c, 22d, and 22e, the His₆-tag specific band was confirmed, indicating the presence of the antigen in the OMNVs.

### Example 16. Analysis of Activity of Inducing Cytokine Release from Dendritic Cells by OMNVs expressing antigens based on the outer membrane protein display system

To confirm activity of inducing cytokine release from dendritic cells by OMNVs expressing antigens based on the outer membrane protein display system prepared by the method of Example 15, the OMNVs expressing the antigen were treated to bone marrow-derived dendritic cells by the method of Example 13-1 to obtain conditioned medium. mTNF-α, mIL-6, and mIL-12p40 were quantified from the obtained conditioned medium by ELISA.

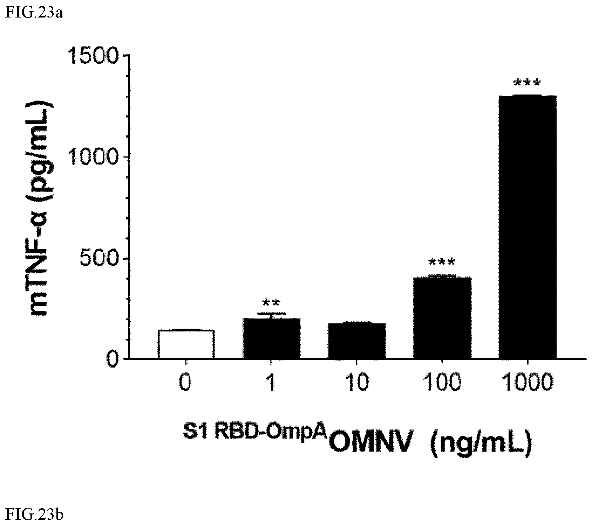

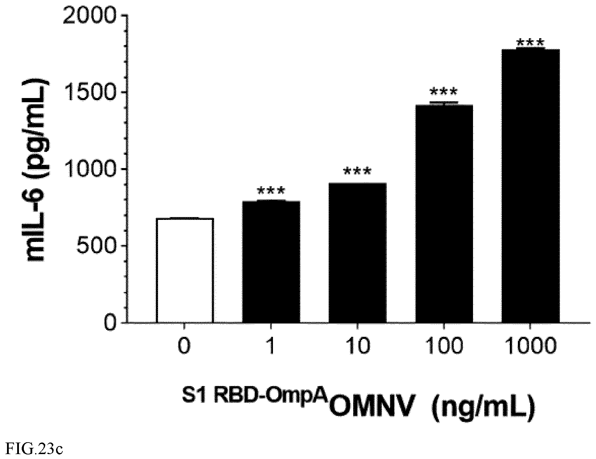

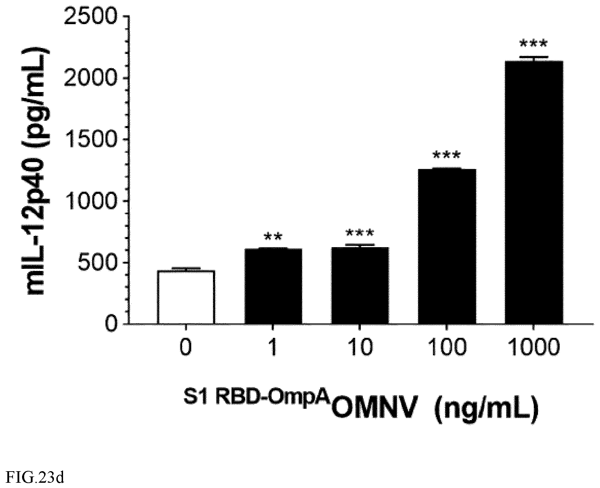

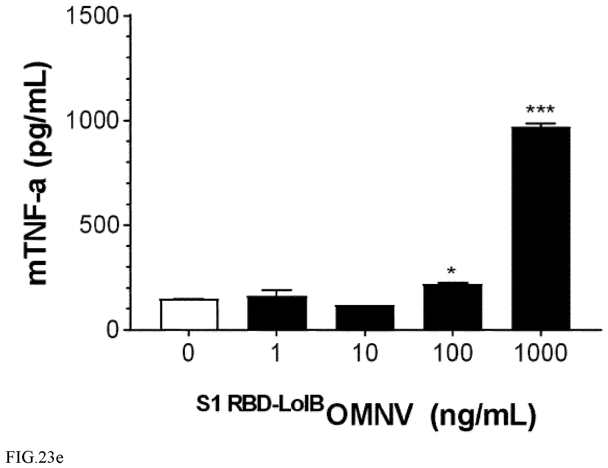

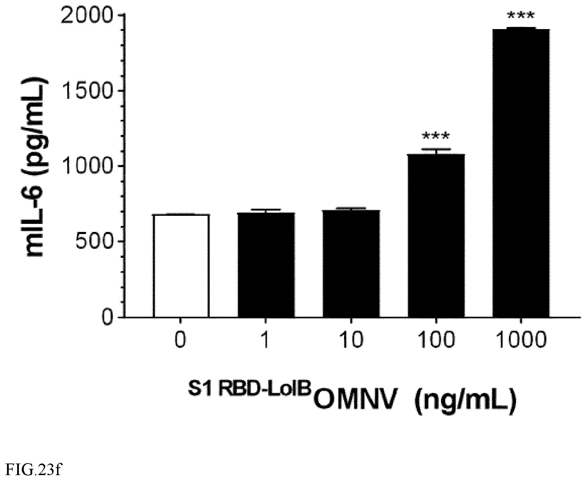

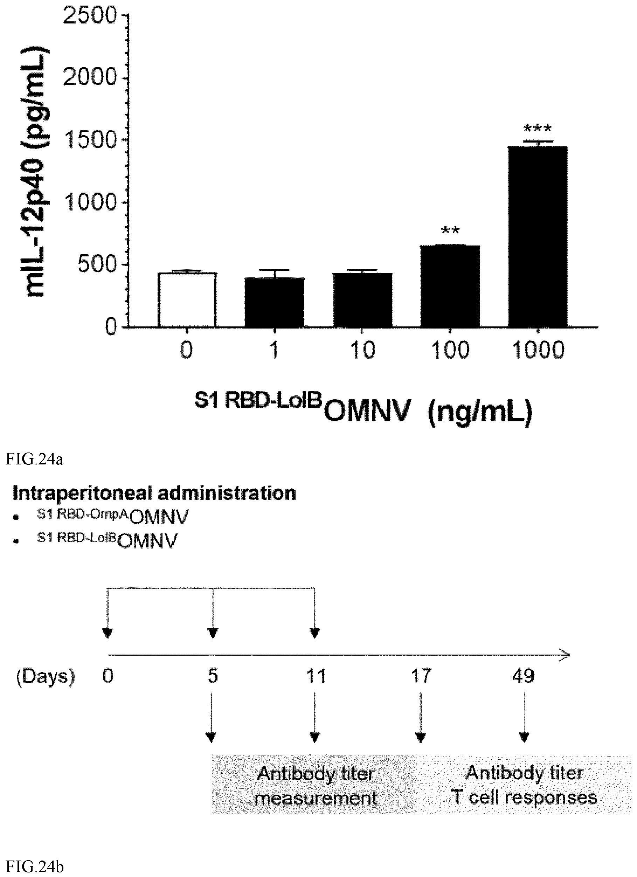

As shown in Figures 23a to 23f, OMNVs expressing antigens based on the outer membrane protein display system can induce the release of mTNF-α, mIL-6, and mIL-12p40 from dendritic cells in a dose-dependent manner.

### Example 17. Vaccination Using OMNVs Expressing Antigens Based on the Outer Membrane Protein Display System

### 17-1. Intraperitoneal Administration Model

To determine the short-term and long-term immunity-inducing activity of OMNVs expressing antigens based on the outer membrane protein display system prepared by the method of Example 15, OMNVs expressing the antigen were intraperitoneally administered into C57BL/6 (male, 4 weeks old) three times at 5-6 day intervals. When the first administration was day 0, the blood was collected on days 5, 11, and 17, and the serum was separated. The experimental process is shown in Figure 24a.

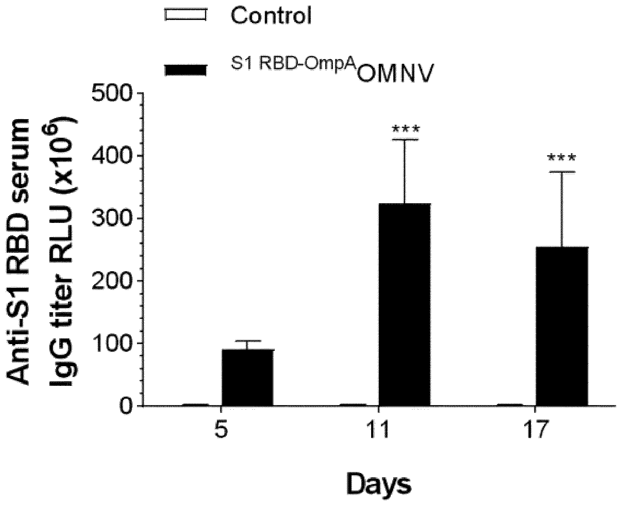

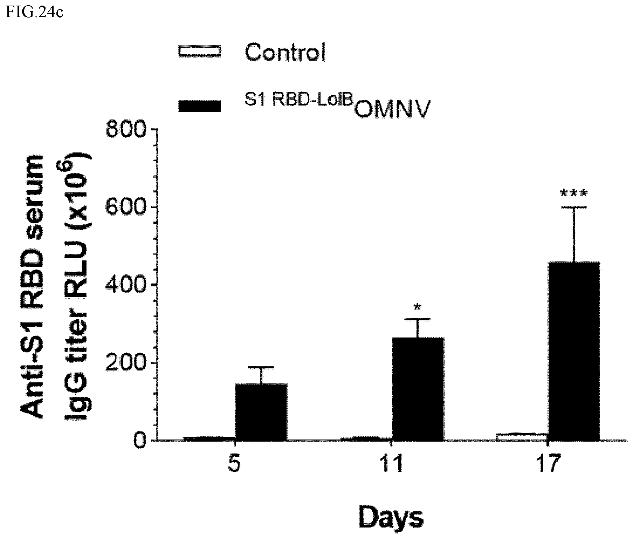

Then, antigen-specific IgG titer was measured by the method of Example 14-1. As shown in Figures 24b and 24c, the case where OMNVs expressing the antigen were administered showed higher antigen-specific IgG titer compared to the control group.

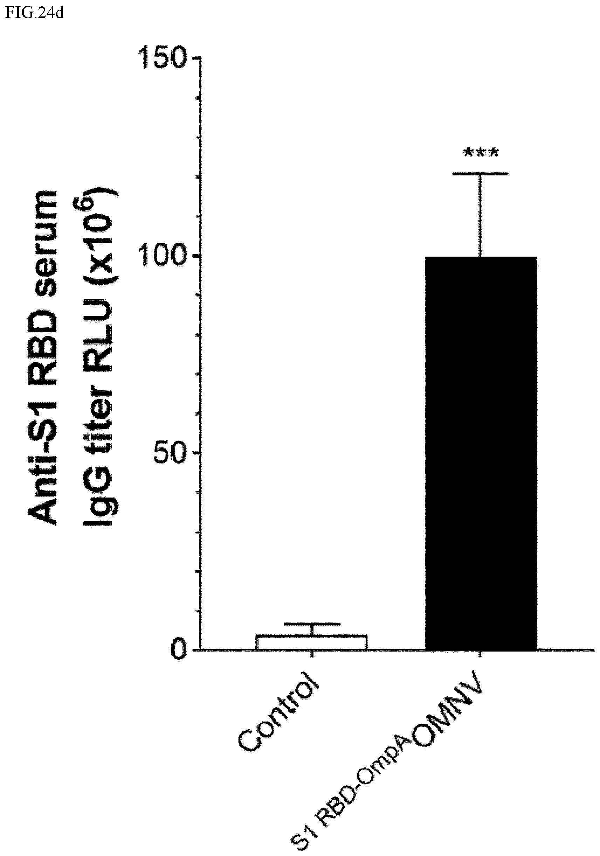

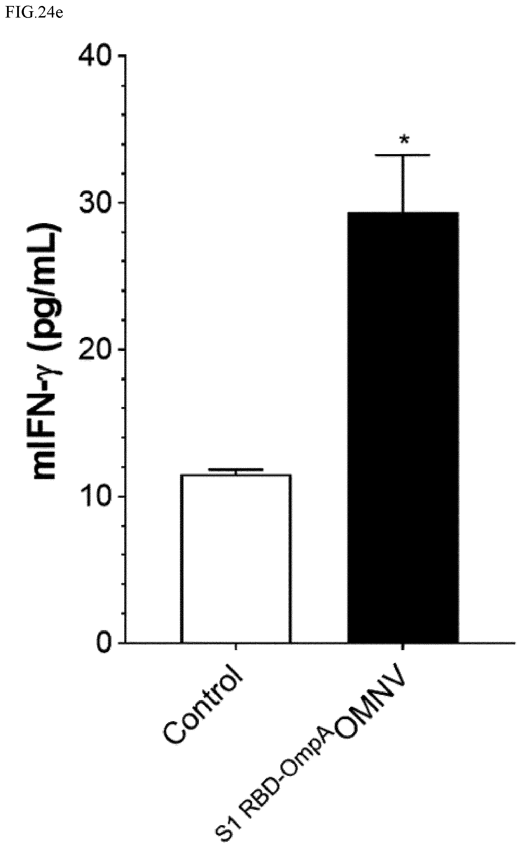

In addition, long-term immunity-inducing activity of OMNVs expressing antigens based on the outer membrane protein display system was determined by harvesting the serum and spleen from the mouse on day 49 by the method of Example 14-2. As shown in Figures 24d and 24e, the case where OMNVs expressing the antigen were administered showed higher antigen-specific IgG titer and mIFN-γ release compared to the control group.

### 17-2. Intramuscular Administration Model

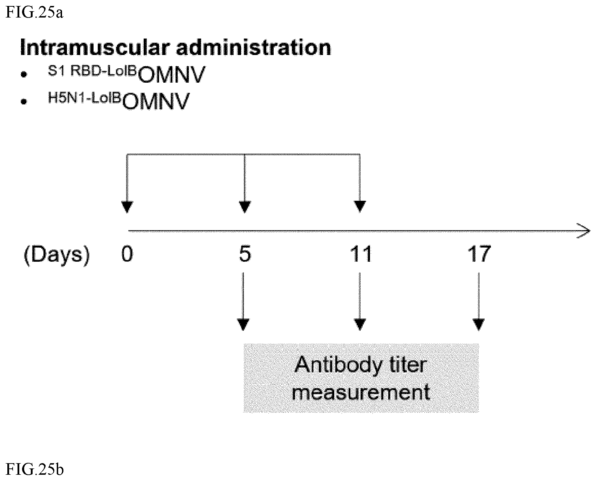

To verify the short-term and long-term immunity-inducing activity of OMNVs expressing an antigen, the OMNVs were intramuscularly administered into C57BL/6 (male, 4 weeks old) mice three times at intervals of 5-6 days. When the first administration was day 0, blood was collected on days 5, 11, and 17, and serum was separated. The experimental process is shown in Figure 25a.

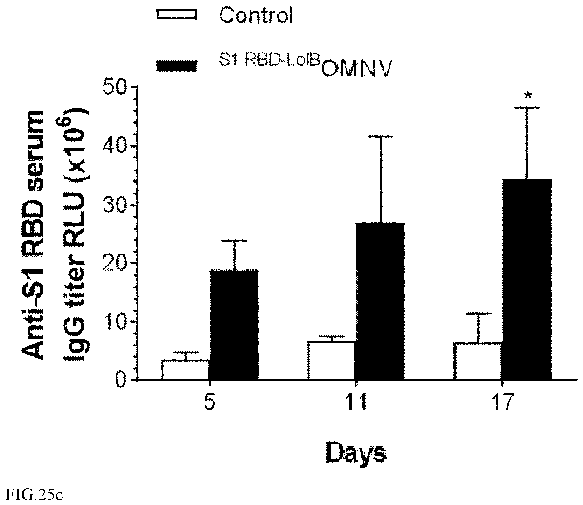

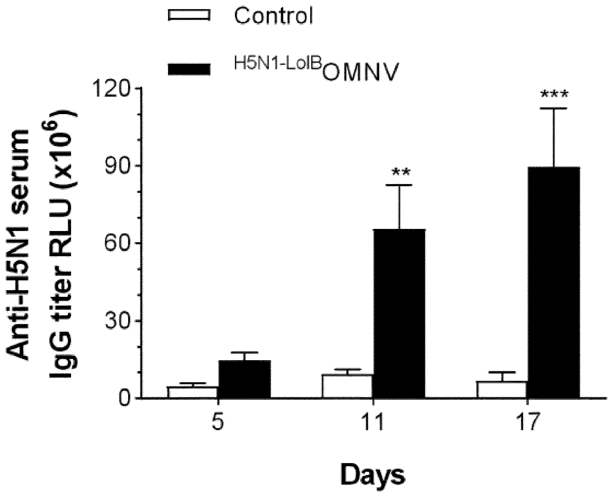

Next, the antigen-specific IgG titer was analyzed according to the method of Example 14-1. As a result, as shown in Figures 25b and 25c, the case where OMNVs expressing an antigen were administered showed a higher antigen-specific IgG titer compared to the control group.

### Example 18. Safety Analysis of OMNVs Expressing an Antigen Based on the Outer Membrane Protein Display System

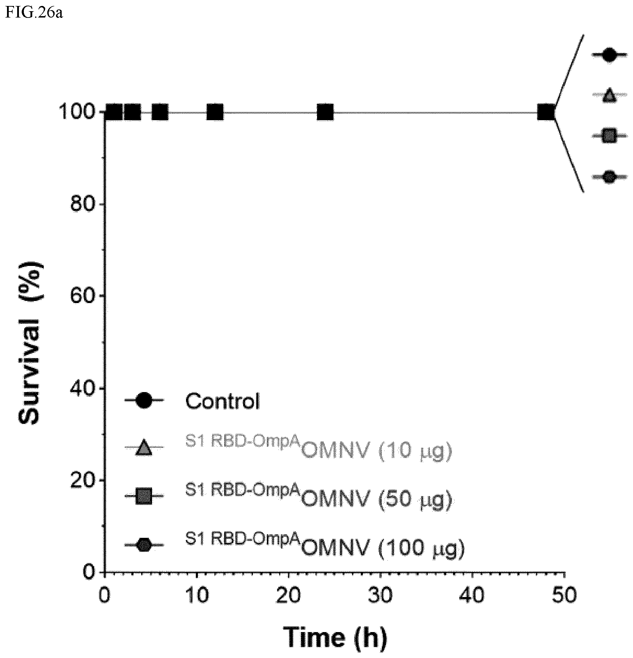

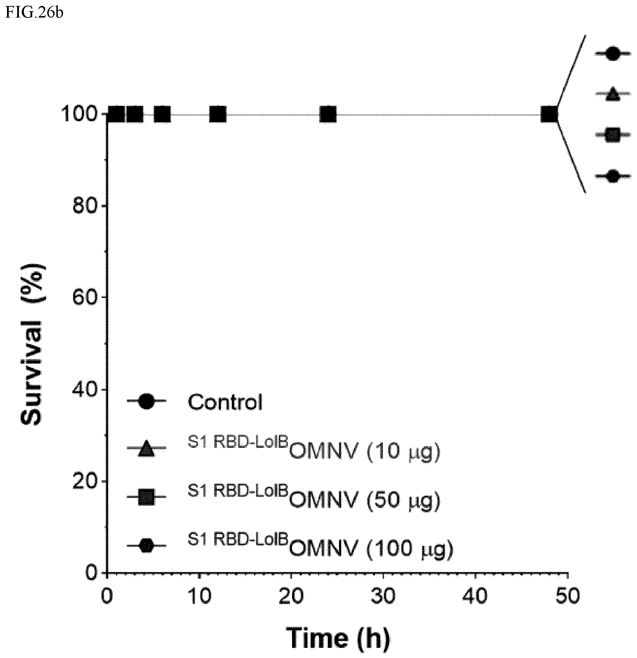

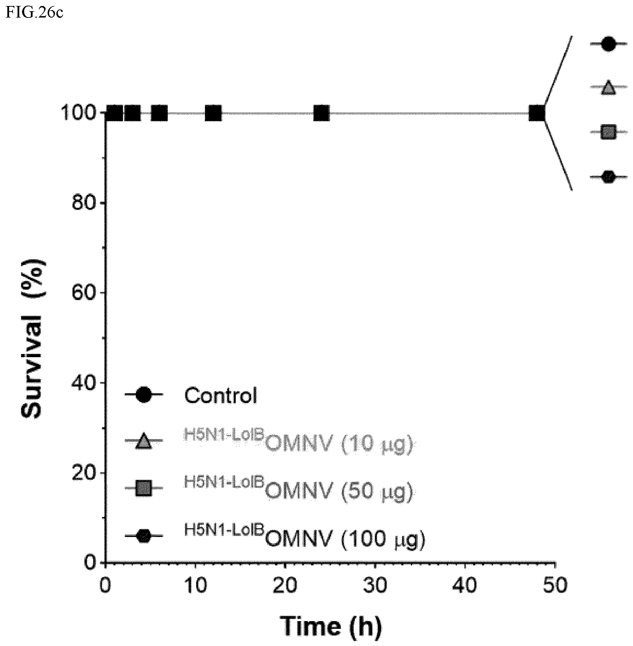

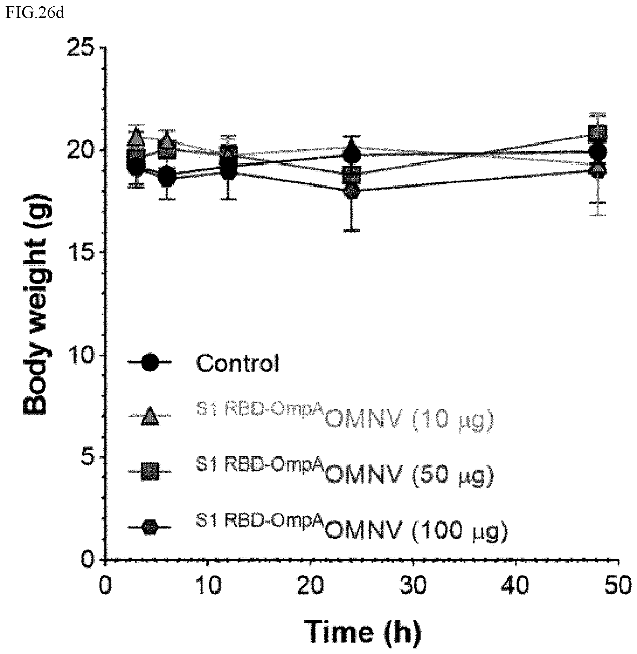

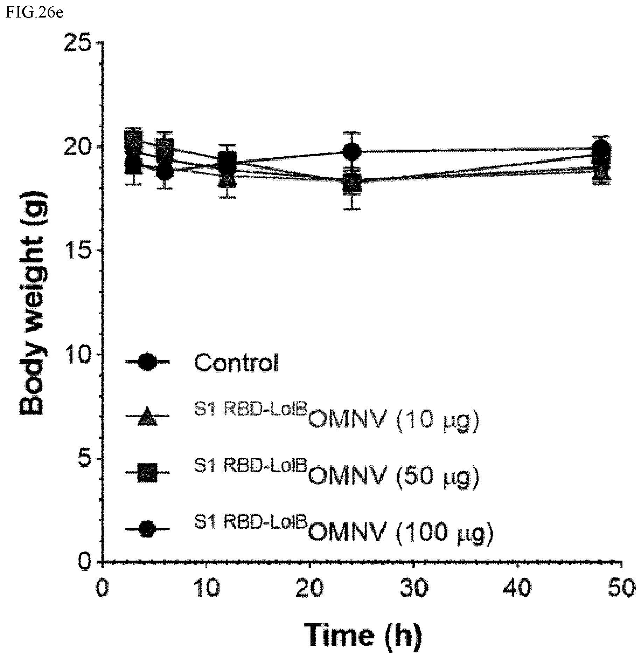

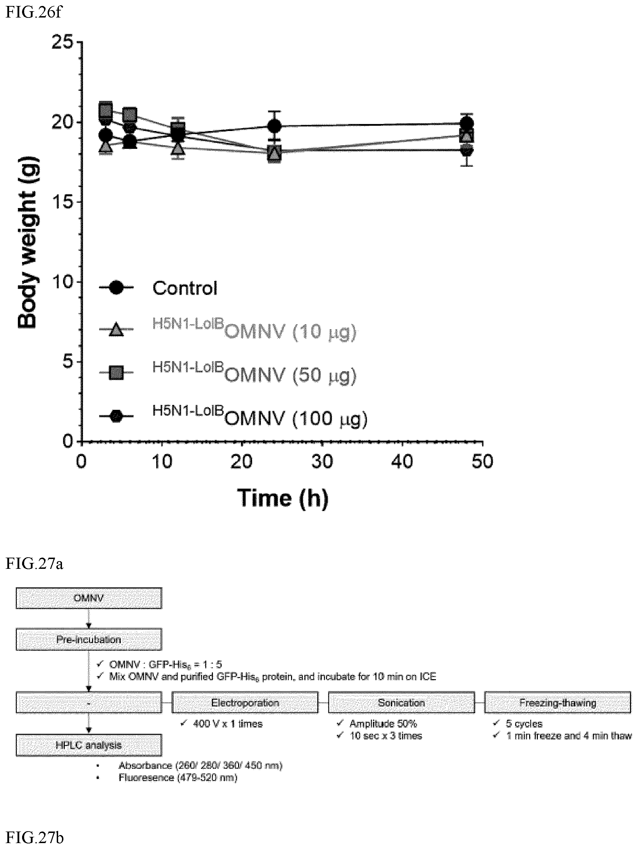

To determine the safety of OMNVs expressing an antigen based on the outer membrane protein display system prepared by the method of Example 15, the OMNVs were intraperitoneally administered into mice (0, 10, 50, 100 µg/head, single administration). The survival and body weight of the mice were observed for 48 hours after administration. As shown in Figures 26a to f, no significant differences were observed between the control group and the group administered with OMNVs expressing an antigen.

Combining the results of Examples 15, 16, 17, and 18, OMNVs expressing various proteins such as peptides, cytokines, growth factors, antibodies, antigenic peptides, and antigenic proteins using the outer membrane protein display system utilizing outer membrane proteins of Gram-negative bacteria such as OmpA, LolB, OmpF, and ClyA, can be very useful in developing methods for treating and diagnosing cancer, methods for delivering substances for treating and diagnosing diseases, methods for delivering vaccines for preventing and treating diseases, with minimal adverse effects, thereby very useful in developing treatments and vaccines for various diseases, including cancer and infectious diseases. The various proteins can be used to enhance the therapeutic and preventive efficacy of outer membrane vesicles and the efficacy of vaccine delivery vehicles.

### Example 19. Method of Loading Proteins into OMNVs

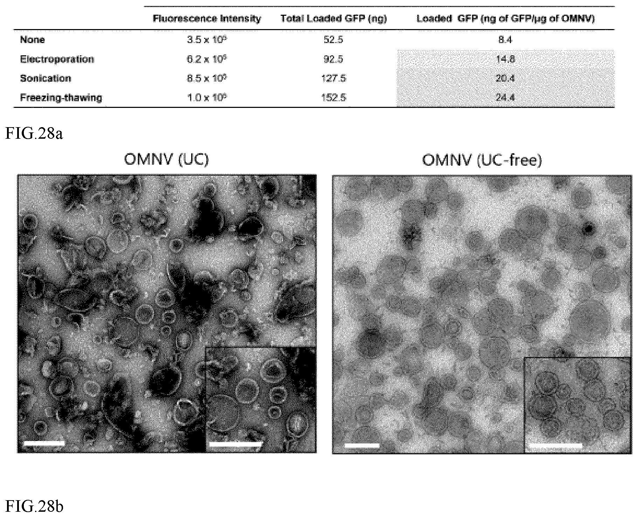

To develop a method for loading therapeutic substances, diagnostic substances, immuneenhancing substances, or vaccine antigens into OMNVs, OMNVs were mixed with GFP as a model substance according to the schematic diagram in Figure 27a. GFP was loaded using control, electroporation, sonication, and freeze-thawing methods, and the amount of loaded GFP was analyzed by size-exclusion chromatography (using a standard curve made with purified GFP). As shown in Figure 27b, approximately 8.4 ng, 14.8 ng, 20.4 ng, and 24.4 ng of GFP were loaded into 1 µg of OMNVs by control, electroporation, sonication, and freeze-thawing methods, respectively.

Additionally, to increase the efficiency of loading therapeutic substances, diagnostic substances, immunostimulatory substances, or vaccine antigens into OMNVs, the outer membrane or the purified OMNVs treated with Sarkosyl can be treated with a well-known alkaline solution to open the membrane, load the therapeutic substances, diagnostic substances, immunostimulatory substances, or vaccine antigens, and then reseal the membrane.

### Example 20. Preparation of OMNVs by Various Methods

To prepare OMNVs without using ultracentrifugation, *E. coli* BL21 (DE3) *ΔmsbB-*AmCyan was cultured in VP-LB using the method of Example 1, and the outer membrane of Gram-negative bacteria was isolated using Sarkosyl. Then, OMNVs were prepared using a microfluidic processor, and centrifugation, tangential flow filtration, and dialysis were performed to obtain approximately 7 mL of sample. Finally, OMNVs were obtained from the fraction obtained through Capto Core 700 (OMNV (UC-free)). Meanwhile, OMNVs were also prepared using *E. coli* BL21 (DE3) *ΔmsbB* by the method of Example 1 (OMNV (UC)).

### Example 21. Comparison of Characteristics According to the Method of Preparing OMNVs

### 21-1. Analysis of Morphology and Size According to the Method of Preparing OMNVs

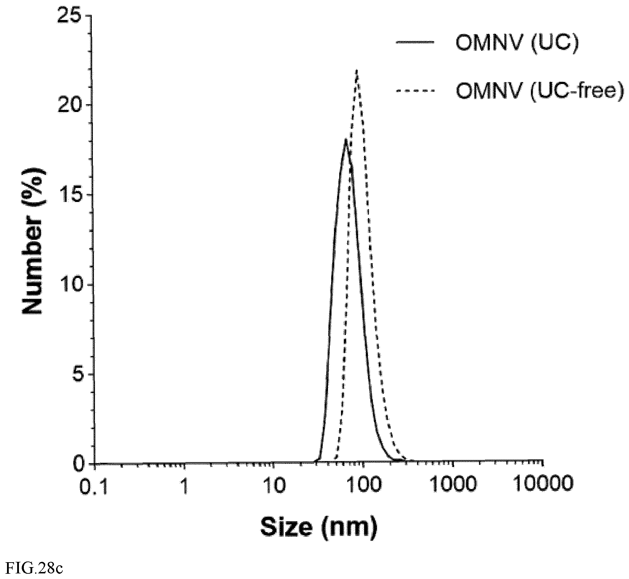

To analyze the morphology and size of OMNV (UC) and OMNV (UC-free) prepared by the method of Example 20, OMNV (UC) and OMNV (UC-free) were observed and analyzed using a transmission electron microscope and a dynamic light scattering analyzer with reference to Example 3-1. As shown in Figure 28a, both OMNV (UC) and OMNV (UC-free) were composed of lipid bilayers. Additionally, as shown in Figure 28b, the average diameters of OMNV (UC) and OMNV (UC-free) were measured to be 74.3 ± 15.8 nm and 105.1 ± 10.7 nm, respectively.

### 21-2. Analysis of Total Particle Numbers and Total Protein Amounts According to the Method of Preparing OMNVs

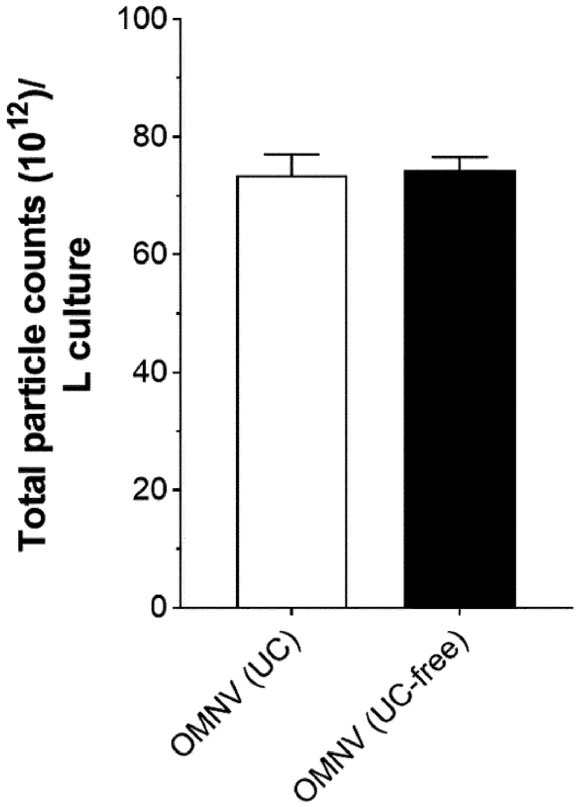

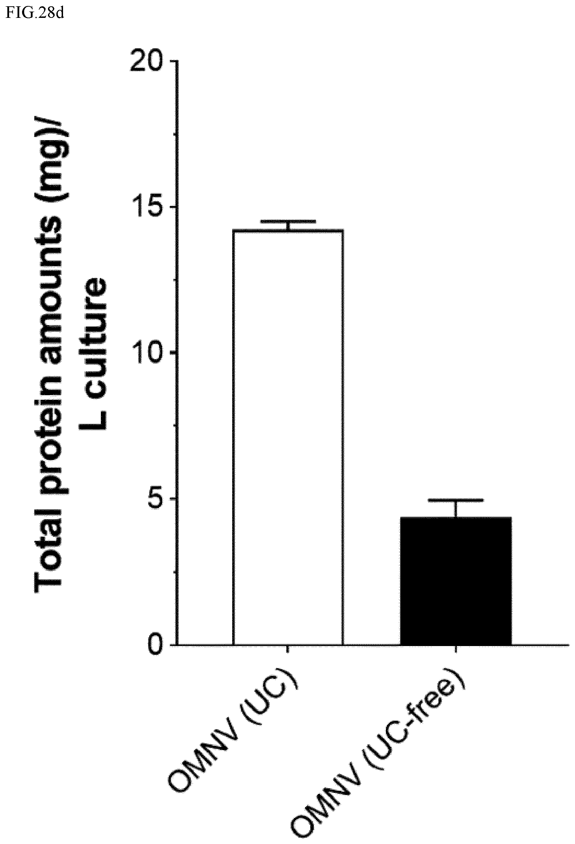

To quantifytotal particle numbers and total protein amounts of OMNV (UC) and OMNV (UC-free) prepared by the method of Example 20, OMNV (UC) and OMNV (UC-free) were analyzed and quantified using a nanoparticle tracking analyzer and Bradford assay with reference to Example 3-2. As shown in Figure 28c, an average of (7.35 ± 0.35) × 10¹³ particles and (7.45 ± 0.21) × 10¹³ particles could be obtained per liter of culture medium for OMNV (UC) and OMNV (UC-free), respectively. Additionally, as shown in Figure 28d, an average of 14.200 ± 0.283 mg and 4.400 ± 0.566 mg could be obtained per liter of culture medium for OMNV (UC) and OMNV (UC-free), respectively.

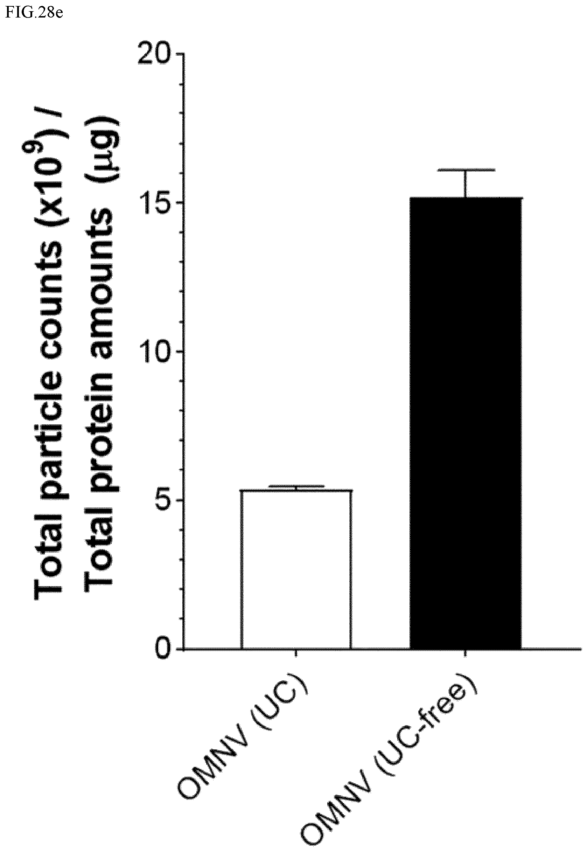

Furthermore, to determine the purity of OMNV (UC) and OMNV (UC-free), total particle numbers per total protein amounts were calculated. As shown in Figure 28e, OMNV (UC) and OMNV (UC-free) showed purities of (5.353 ± 0.107) × 10⁹ particles/µg and (15.183 ± 0.921) × 10⁹ particles/µg, respectively.

### Example 22. Analysis of Innate Immunity-inducing Activity According to the Method of Preparing OMNVs

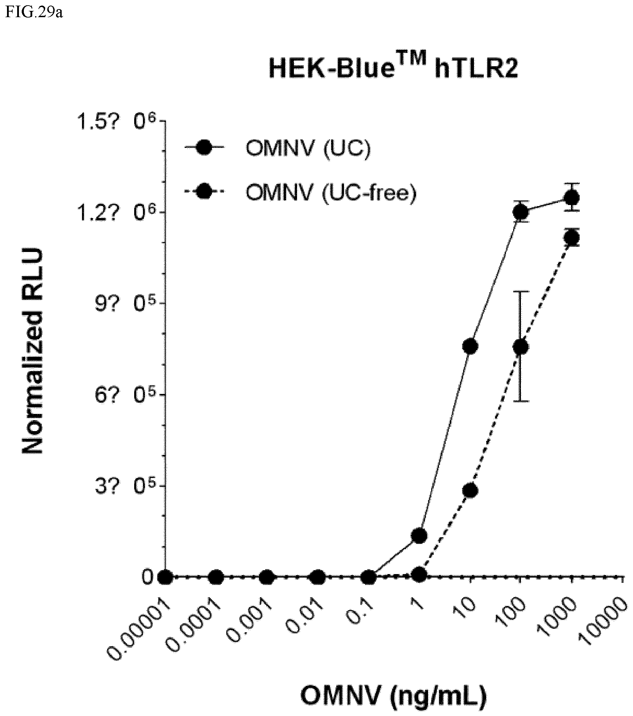

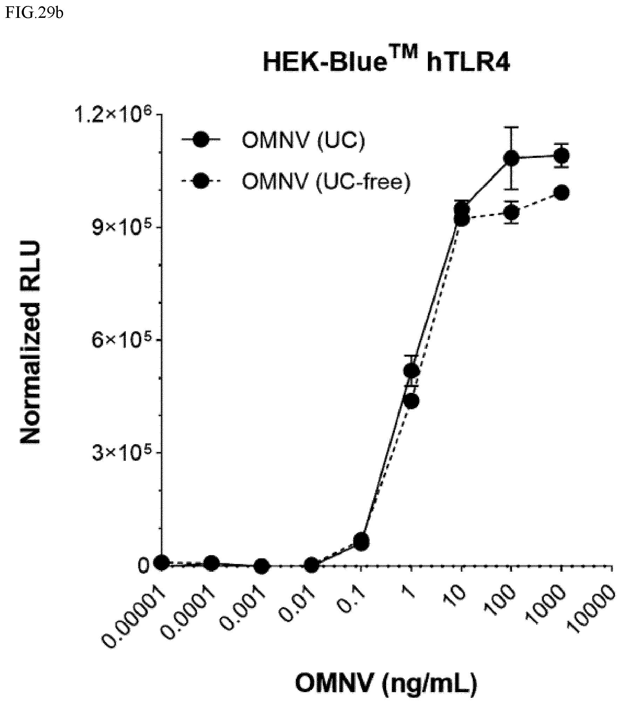

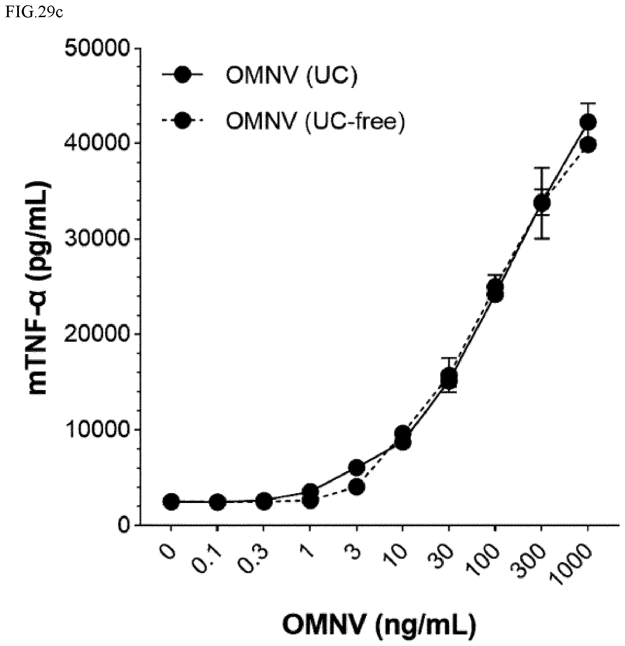

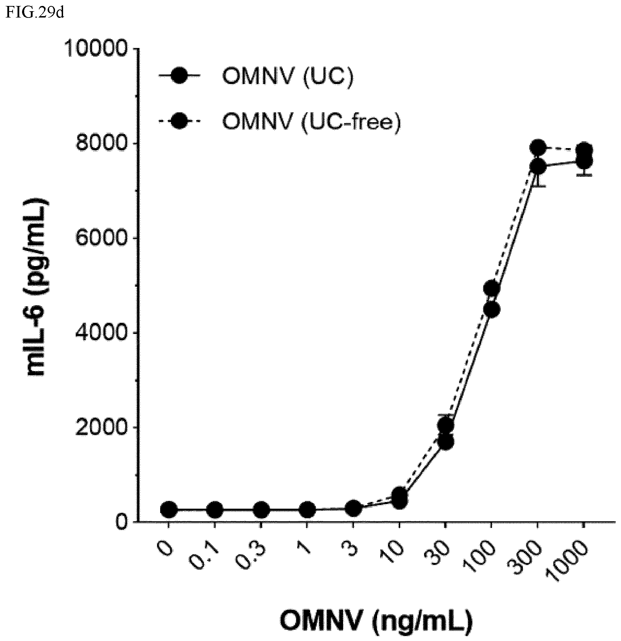

The innate immunity-inducing activity of OMNV (UC) and OMNV (UC-free) prepared by the method of Example 20 was determined by the method of Example 4. As shown in Figures 29a to 29d, OMNV (UC) and OMNV (UC-free) could induce innate immunity in dose-dependent manners to the similar extent.

### Example 23. Analysis of Anti-tumor Activity According to the Method of Preparing OMNVs

To determine anti-tumor activity of OMNV (UC) and OMNV (UC-free) prepared by the method of Example 20, MB49 (mouse bladder cancer cells, 5×10⁵ cells/head) were subcutaneously introduced into C57BL/6 (male, 7 weeks old) mice, and the size of the tumor tissue was measured over time using a vernier caliper (size = 0.5 × width × length × length). When the size of the tumor tissue reached approximately 100 mm³, OMNV (UC) and OMNV (UC-free) were intratumorally administered three times at 3-day intervals (10 µg/head per injection), and the size of the tumor tissue was measured over time.

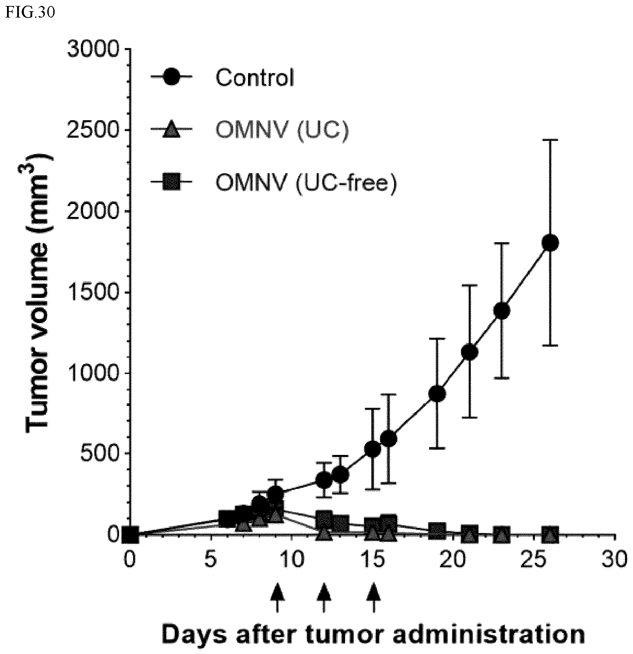

As a result, as shown in Figure 30, OMNV (UC) and OMNV (UC-free) exhibited anti-tumor activity.

In summary, to increase stability inside and outside the body, including the blood, to reduce adverse effects, and to enhance therapeutic efficacy, drug delivery efficacy, as well as vaccine delivery efficacy, various substances or their complexes, such as anticancer compounds, anti-inflammatory compounds, various immune enhancers including STING agonists, peptides, proteins, nucleic acids (mRNA, siRNA, DNA, plasmid, etc.), aptamers, toxins, and various forms of antigens, alone or in combination, can be loaded inside or displayed on the surface of OMNVs. Then, it is possible to effectively enhance the stability and efficacy of OMNVs as therapeutics, drug delivery carriers, and/or vaccine delivery carriers, while reducing adverse effects.

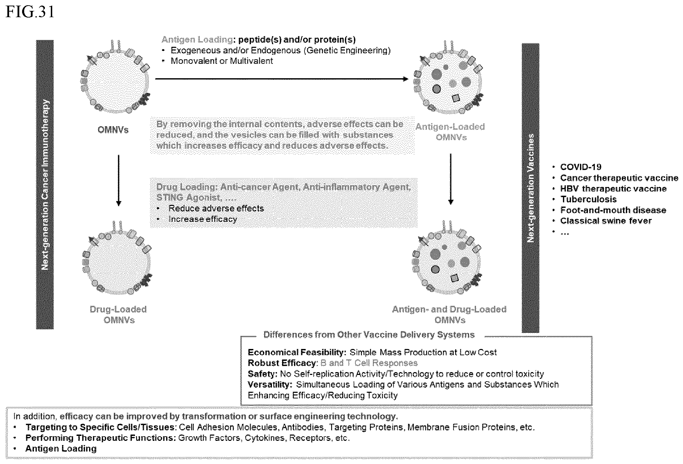

OMNVs according to the present invention can be manufactured by various methods and utilized in various fields (Figure 31). It is possible to effectively enhance the stability and efficacy of OMNVs as therapeutics, drug delivery carriers, and/or vaccine delivery carriers, while reducing adverse effects by additionally using various methods: 1) Modulating and homogenizing the size, or 2) Increasing the stability inside and outside the body, including the blood, reducing adverse effects, and enhancing therapeutic efficacy, drug delivery efficacy, as well as vaccine delivery efficacy by loading and/or displaying various substances or their complexes, such as anticancer compounds, anti-inflammatory compounds, various immune enhancers including STING agonists, peptides, proteins, nucleic acids (mRNA, siRNA, DNA, plasmid, etc.), aptamers, toxins, and various forms of antigens, alone or in combination, inside or on the surface of OMNVs, or 3) Combining the above methods

In addition, the method of manufacturing OMNVs of the present invention, in which substances for enhancing therapeutic efficacy, drug delivery carrier efficacy, and vaccine delivery carrier efficacy are loaded and/or displayed alone or in combination, can be used for therapeutic, drug delivery, or vaccine purposes, or for experimental purposes *in vitro* or *in vivo.*

Furthermore, in summary, OMNVs or OMNVs loaded with therapeutic, diagnostic, and vaccine substances can be administered in a dosage known to those skilled in the art of medicine or veterinary medicine, considering factors such as the age, sex, species, breed, and condition of the recipient animal, and the route of administration. The route of administration can be transdermal, mucosal (e.g., oral, nasal, rectal, vaginal), or parenteral (intramuscular, subcutaneous, intravenous, intraperitoneal, or intra-articular). The therapeutic, diagnostic, and vaccine compositions can be administered alone or co-administered or sequentially administered with other treatments or therapies. The dosage forms can include suspensions, syrups, or elixirs, and preparations for oral, parenteral, subcutaneous, intradermal, intramuscular, or intravenous administration, such as sterile suspensions or emulsions. The therapeutic, diagnostic, and vaccine compositions can be administered as a spray or mixed with food and/or water or delivered with suitable carriers, diluents, or excipients such as sterile water, saline, glucose, etc. The compositions may contain auxiliary substances such as wetting or emulsifying agents, pH buffers, adjuvants, gelling or thickening agents, preservatives, flavoring agents, colorants, etc., depending on the desired formulation and route of administration. Standard pharmaceutical textbooks such as "Remington's Pharmaceutical Sciences, 1990" can be referred to for preparing suitable formulations without undue experimentation.

## Claims

1. A method for preparing Gram-negative bacterial outer membrane-derived nanovesicles (OMNVs), comprising steps of:
(a) obtaining an outer membrane by removing an inner membrane from Gram-negative bacteria; and
(b) preparing nanovesicles from a suspension comprising the outer membrane.

2. The method of claim 1, wherein the Gram-negative bacteria are selected from the group consisting of *Escherichia* genus, *Helicobacter* genus, *Hemophilus* genus, *Neisseria* genus, *Cyanobacterium* genus, *Klebsiella* genus, *Acetobacter* genus, *Acinetobacter* genus, *Enterobacter* genus, *Chlamydia* genus, *Vibrio* genus, *Pseudomonas* genus, *Salmonella* genus, *Thiobacter* genus, *Borrelia* genus, *Burkholderia* genus, *Serratia* genus, and *Treponema* genus.

3. The method of claim 1, wherein the Gram-negative bacteria are transformed.

4. The method of claim 3, wherein the Gram-negative bacteria are transformed Gram-negative bacteria whose toxicity of nanovesicles derived from the outer membrane is attenuated.

5. The method of claim 3, wherein the Gram-negative bacteria are genetically modified Gram-negative bacteria having one or more genotypes selected from the group consisting of *ΔmsbB, ΔkdsA, ΔkdsB, ΔlpxB, ΔkdtA, ΔlpxC, ΔlpxD, Δssc, ΔlpxA,* and *ΔhtrB.*

6. The method of claim 3, wherein the Gram-negative bacteria are transformed Gram-negative bacteria expressing cell membrane fusion substances.

7. The method of claim 3, wherein the Gram-negative bacteria are transformed Gram-negative bacteria targeting specific cells or tissues.

8. The method of claim 3, wherein the Gram-negative bacteria are transformed Gram-negative bacteria expressing one or more selected from the group consisting of antigenic proteins, antigenic peptides, immunostimulatory proteins, immunosuppressive proteins, cell adhesion molecules, antibodies, targeting proteins, cell membrane fusion proteins, cytokines, enzymes, growth factors, extracellular domains of membrane receptors, marker proteins, fragments thereof, and fusion proteins thereof.

9. The method of any one of claims 3 to 8, wherein the Gram-negative bacteria are Gram-negative bacteria transformed two or more times.

10. The method of claim 1, wherein the membrane of the OMNVs further comprises components other than the outer membrane of the Gram-negative bacteria.

11. The method of claim 10, wherein the components other than the cell membrane of the Gram-negative bacteria are selected from the group consisting of targeting substances, cell membrane fusion substances, cyclodextrins, polyethylene glycol, and hyaluronic acid.

12. The method of claim 1, wherein the membrane components of the OMNVs are chemically modified.

13. The method of claim 12, wherein the membrane components of the OMNVs are chemically modified using thiol groups or amine groups, or proteins, polyethylene glycol, or hyaluronic acid are chemically bound to the nanovesicles derived from gram-negative bacterial outer membrane.

14. The method of claim 1, wherein the inner membrane of the Gram-negative bacteria is removed by treating the Gram-negative bacteria with Sarkosyl.

15. The method of claim 1, further comprising, after step (b), isolating OMNVs by a method selected from the group consisting of ultracentrifugation, density gradient ultracentrifugation, ultrafiltration, size-exclusion chromatography, ion exchange chromatography, Capto Core chromatography, immunoaffinity separation, microfluidic separation, aqueous two-phase system, polymer-based precipitation, sonication, and extrusion.

16. OMNVs prepared by the method of claim 1.

17. A pharmaceutical composition for treating a disease comprising the OMNVs according to claim 16.

18. The pharmaceutical composition of claim 17, wherein the disease is selected from the group consisting of thyroid cancer, liver cancer, osteosarcoma, oral cancer, brain tumor, gallbladder cancer, colon cancer, lymphoma, bladder cancer, leukemia, small intestine cancer, tongue cancer, esophageal cancer, renal cancer, gastric cancer, breast cancer, pancreatic cancer, lung cancer, skin cancer, testicular cancer, penile cancer, prostate cancer, ovarian cancer, and cervical cancer.

19. The pharmaceutical composition of claim 17, wherein the disease is selected from the group consisting of hypertension, osteoporosis, irritable bowel syndrome, acute coronary syndrome, stroke, diabetes, atherosclerosis, obesity, peptic ulcer, Alzheimer's disease, emphysema, chronic obstructive pulmonary disease, asthma, skin diseases, and autoimmune diseases.

20. The pharmaceutical composition of claim 17, wherein the composition further comprises a substance that enhances therapeutic effects or reduces adverse effects.

21. The pharmaceutical composition of claim 20, wherein the substance that enhances therapeutic effects or reduces adverse effects is loaded into the OMNVs.

22. The pharmaceutical composition of claim 20, wherein the substance that enhances therapeutic effects or reduces adverse effects is one or more selected from the group consisting of anticancer agents, immunostimulators, STING agonists, anti-inflammatory agents, endotoxin inhibitors, peptides, proteins, toxins, nucleic acids, beads, microparticles, and nanoparticles.

23. The pharmaceutical composition of claim 22, wherein the nucleic acids are selected from the group consisting of DNA, RNA, aptamers, locked nucleic acids (LNA), peptide nucleic acids (PNA), and morpholinos.

24. The pharmaceutical composition of claim 22, wherein the nanoparticles are selected from the group consisting of iron oxide, gold, carbon nanotubes, and magnetic beads.

25. A vaccine composition for preventing or treating a disease comprising the OMNVs of claim 16.

26. The vaccine composition of claim 25, wherein the vaccine composition further comprises an antigen.

27. The vaccine composition of claim 26, wherein the antigen is loaded into the OMNVs.

28. The vaccine composition of claim 26, wherein the antigen is a bacterial-derived antigen, a viral-derived antigen, a fungal-derived antigen, a cancer-derived antigen; or a mutant of Ras protein, Raf protein, Src protein, Myc protein, EGFR, PDGFR, VEGFR, p53, PTEN, or HER2/neu.

29. The vaccine composition of claim 25, wherein the disease is an infection caused by bacteria, viruses, or fungi.

30. The vaccine composition of claim 29, wherein the infection is selected from the group consisting of skin infections, respiratory infections, genitourinary infections, bone and joint infections, central nervous system infections, and sepsis.

31. The vaccine composition of claim 25, wherein the disease is selected from the group consisting of thyroid cancer, liver cancer, osteosarcoma, oral cancer, brain tumor, gallbladder cancer, colon cancer, lymphoma, bladder cancer, leukemia, small intestine cancer, tongue cancer, esophageal cancer, renal cancer, gastric cancer, breast cancer, pancreatic cancer, lung cancer, skin cancer, testicular cancer, penile cancer, prostate cancer, ovarian cancer, and cervical cancer.

32. The vaccine composition of claim 25, wherein the disease is selected from the group consisting of hypertension, osteoporosis, irritable bowel syndrome, acute coronary syndrome, stroke, diabetes, atherosclerosis, obesity, peptic ulcer, Alzheimer's disease, chronic obstructive pulmonary disease, asthma, skin diseases, and autoimmune diseases.

33. The vaccine composition of claim 25, wherein the vaccine is used in combination with drugs or adjuvants to increase efficacy or reduce adverse effects.

34. Use of the OMNVs of claim 16 for preparing a pharmaceutical composition for treating a disease.

35. A method for treating a disease comprising administering an effective amount of a pharmaceutical composition comprising the OMNVs of claim 16 to a subject in need thereof.
